(19) **Europäisches Patentamt / European Patent Office / Office européen des brevets**

(11) **EP 4 588 510 A1**

(12) **EUROPEAN PATENT APPLICATION**
published in accordance with Art. 153(4) EPC

(43) Date of publication:
**23.07.2025 Bulletin 2025/30**

(21) Application number: **23865933.8**

(22) Date of filing: **18.09.2023**

(51) International Patent Classification (IPC):
*A61N 7/00* (2006.01)

(52) Cooperative Patent Classification (CPC):
**A61N 7/00**

(86) International application number:
**PCT/KR2023/014074**

(87) International publication number:
**WO 2024/058640 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.09.2022 KR 20220117422**

(71) Applicants:
• **UIF (University Industry Foundation), Yonsei
University
Seoul 03722 (KR)**
• **Institute for Basic Science
Yuseong-gu
Daejeon 34126 (KR)**

(72) Inventors:
• **CHEON, Jinwoo
Seoul 08004 (KR)**
• **LEE, Jae-Hyun
Seoul 06640 (KR)**
• **KWAK, Minsuk
Gwangmyeong-si, Gyeonggi-do 14349 (KR)**
• **LEE, Jung-uk
Seoul 07312 (KR)**
• **KANG, Sunghwi
Yongin-si, Gyeonggi-do 16824 (KR)**
• **CHOI, Seo-Hyun
Seoul 04423 (KR)**

(74) Representative: **Kellas, Fiona et al
Maucher Jenkins
Seventh Floor Offices
Artillery House
11-19 Artillery Row
London SW1P 1RT (GB)**

Remarks:
The complete document including Reference
Table(s) and the Sequence Listing(s) can be
downloaded from the EPO website

(54) **OPENING/CLOSING CONTROL DEVICE FOR TARGET FORCE-SENSITIVE ION CHANNEL
THAT GENERATES SOUND WAVE PATTERN BY USING RESONANCE FREQUENCY(IES),
AND BRAIN FUNCTION CONTROL DEVICE**

(57) The present invention relates to: (i) a mechano-sensitive ion channel gating control device for a target mechano-sensitive ion channel in a biological lipid membrane; (ii) a brain function control device for non-invasively activating or inhibiting a target neural circuit or neural network; (iii) a computer-readable recording medium or a medium for transmitting a program to a computer for use in the aforementioned devices; and (iv) an acoustic wave oscillator operably controlled by said program. The present invention is characterized by generating an acoustic wave pattern that utilizes the resonance frequency(ies) of a target mechano-sensitive ion channel within a specific cell in a target body region to modulate the opening and closing of the target mechano-sensitive ion channel.

EP 4 588 510 A1

[Fig. 2]

**Description**

**TECHNICAL FIELD**

The present invention relates to: (i) a mechano-sensitive ion channel gating control device for a target mechano-sensitive ion channel in a biological lipid membrane; (ii) a brain function control device for non-invasively activating or inhibiting a target neural circuit or neural network; (iii) a computer-readable recording medium or a medium for transmitting a program to a computer for use in the aforementioned devices; and (iv) an acoustic wave oscillator operably controlled by said program.

**BACKGROUND ART**

Most metabolic processes in the body occur through complex chemical reactions. Traditional regulation of bodily metabolic functions using conventional chemical drug therapies has played a crucial role in the treatment of numerous diseases. However, orally administered drugs or injectable medications are systemically distributed through the bloodstream, often leading to off-target side effects in addition to their intended therapeutic effects.

Metabolism in the human body is an essential function for survival, requiring the coordinated activity of multiple physiological systems across various organs to maintain homeostasis. The regulation of homeostasis in response to external stimuli-such as food intake, temperature fluctuations, energy expenditure, and infections-is primarily governed by the endocrine system and the nervous system. For example, cytokines are signaling proteins secreted primarily by immune cells that modulate immune function, particularly in response to inflammation. The production and secretion of cytokines are among the metabolic processes regulated by neural circuits. When partial organ damage, functional loss, or dysfunction of neural circuits disrupts these metabolic processes, including immune regulation, it can lead to disease or pathological conditions. In recent years, an alternative therapeutic approach has emerged that differs from traditional chemical drugs used to modulate neural signaling. Instead of pharmacological agents, bioelectronic medicine (also known as electroceuticals) employs electrical stimulation or other physical stimuli to modulate neural activity for the treatment or alleviation of immune and metabolic disorders. Electroceuticals can be defined as therapeutic methods that regulate metabolic functions and restore or maintain physiological homeostasis by stimulating neural circuits using electric fields, light, magnetic fields, or ultrasound, rather than conventional drugs.

The nervous system consists of the central nervous system (CNS)-which includes the brain and spinal cord-and the peripheral nervous system (PNS)-which extends throughout the body and transmits neural signals from the CNS to various organs. The CNS plays a crucial role in sensing systemic metabolic states and regulating metabolism by releasing endocrine neural signals in response to these sensory inputs. The PNS, which branches throughout the body, serves to relay these neural signals to target organs. Damage to the brain's neural circuits can result in a range of neurological disorders, including stroke, depression, epilepsy, and Parkinson's disease. In such cases, artificial neural stimulation can be used to restore or compensate for lost function. This therapeutic approach is referred to as neuromodulation, and some of its applications have been validated-leading to FDA approval of technologies such as Deep Brain Stimulation (DBS). However, except in cases of severe neurological diseases or paralysis, the implantation of neural electrodes into brain regions involved in metabolism, sensation, or motor function has been met with significant psychological resistance, particularly in individuals with intact cognitive function. This reluctance remains a major barrier to the widespread adoption of invasive brain stimulation technologies. One of the key nerves in the PNS, the vagus nerve, is directly connected to vital metabolic organs, including brown adipose tissue, the liver, and the pancreas. Unlike direct brain stimulation, peripheral nerve stimulation for neuromodulation presents a relatively lower psychological barrier. Due to this advantage, bioelectronic medicine (electroceuticals) targeting the peripheral nervous system holds greater promise for neuromodulation-based therapies.

Electroceuticals, which rely on electrical stimulation, require implantation within the body, necessitating a sustained power source to ensure functionality over time. Additionally, these implanted devices must overcome significant challenges, such as electrode corrosion and immune responses, which can impair long-term efficacy in the dynamic in vivo environment.

Recent discoveries regarding the biological effects of light have spurred active clinical research into light-based interventions targeting specific biological sites. In parallel, optogenetics and photodynamic therapy have emerged as promising light-based therapeutic modalities in the field of bioelectronic medicine, incorporating advancements in materials and device structures. Unlike conventional neural stimulation techniques, optogenetics enables highly localized stimulation of specific neural populations, thereby modulating cellular function with exceptional precision. Compared to traditional electrical stimulation methods, optogenetics offers significantly higher spatiotemporal resolution, making it a rapidly growing field of interest for neurological disease treatment.

In addition to electrical stimulation and light-based therapies, focused ultrasound (FUS) stimulation has emerged as another method for physical neuromodulation in electroceuticals. Ultrasound refers to sound waves with frequencies exceeding the audible range, typically between 20 kHz and 2 MHz. By concentrating ultrasound energy at a specific localized target, FUS induces biological responses through high-energy focal stimulation. Several well-established FUS applications include thermal ablation of brain tumors using focused ultrasound, focused ultrasound thalamotomy for treating movement disorders such as Parkinson's disease and essential tremor, focused ultrasound ablation for psychiatric disorders. Recently, low-intensity transcranial focused ultrasound (LIFU) has been explored as a non-invasive therapeutic approach for Alzheimer's disease (AD). In clinical studies, LIFU combined with intravenously injected microbubble contrast agents has been used to transiently open the blood-brain barrier (BBB), thereby promoting neuroplasticity, cerebral glucose metabolism, and cognitive function.

Although electroceuticals employing electrical or optical stimulation have fewer systemic side effects than chemical drugs, surgical implantation into the nervous system still carries inherent risks, such as infection and device malfunction, which could potentially lead to neurological complications. For example, during clinical trials, EnteroMedics, a company developing electroceuticals for obesity treatment, warned that their device could cause infections, diarrhea, depression, or even organ damage and that device repositioning might be required due to abrupt bodily movements. Furthermore, the long-term presence of implanted devices poses risks of secondary injury, prompting the development of biodegradable wireless electroceuticals that can dissolve within the body over time.

Peripheral nerve stimulation has been explored as a therapeutic approach for a wide range of diseases, including diabetes (a representative metabolic disorder), rheumatoid arthritis (an autoimmune disease), cardiovascular disease (one of the leading global causes of death), obesity, urinary disorders such as incontinence and bladder dysfunction, asthma, depression, epilepsy, and pain management.

Among these applications, vagus nerve stimulators (VNS) have received FDA approval for various medical conditions. In 1997, VNS was approved for epilepsy treatment. In 2005, VNS was approved for the treatment of depression. In 2015, a VNS device designed to stimulate the dorsal gastric branch of the vagus nerve received FDA approval for obesity treatment.

Additionally, Medtronic's INTERSTIM, a neuromodulation device for urinary incontinence, was FDA-approved in 2011 and is implanted near the spinal cord root. To date, FDA-approved electroceuticals primarily target peripheral nerve stimulation for brain neuromodulation and direct stimulation of nerves within organs such as the heart, stomach, and bladder. However, recent research efforts are shifting toward stimulating peripheral nerves such as the vagus nerve rather than directly targeting organ-associated nerves, with ongoing investigations into electroceutical applications for metabolic and endocrine disorders such as diabetes and rheumatoid arthritis.

Neuroplasticity refers to the brain's ability to reorganize its neural circuits through growth and restructuring. Broadly, it describes the phenomenon in which the expression of a given genotype is influenced by environmental factors, resulting in adaptive changes in a specific direction. More specifically, neuroplasticity encompasses synaptic plasticity and represents the brain's capacity to modify neural connections in response to learning and memory formation, thereby enabling neurons to adapt to environmental stimuli more effectively. This adaptability signifies that the brain is plastic and malleable. Neural circuits undergo continuous modifications throughout life, with their activity reaching its peak during childhood when new neural circuits are extensively utilized for acquiring languages and motor skills. Although there is a slight decline in adulthood and old age, the brain retains a certain level of neuroplasticity throughout life, allowing for the acquisition of new languages and motor skills to a certain extent.

The brain has evolved the ability to redesign itself in response to experiences, allowing for functional adaptation under similar conditions. Thanks to the structural plasticity of the brain, it is possible to customize and optimize brain function for individual activities. Learning occurs through modifications in neural connections, including changes in the length of neuronal connections, the addition or elimination of synapses, and the formation of new neurons, and neuroplasticity is fundamentally associated with these learning processes.

Even into the 20th century, the prevailing belief was that the structure of the lower subcortical regions of the brain remained unchanged after childhood. However, it became widely accepted that meaningful learning occurs through changes in the length of neural connections and that regions involved in memory formation, such as the hippocampus and dentate gyrus, exhibit significant plasticity, as they continue to generate new neurons into adulthood. Subsequent studies have demonstrated that environmental changes can modify existing neuronal connections and alter behavior and cognition through neurogenesis in various brain regions, including the hippocampus and cerebellum.

Research conducted over centuries has confirmed that substantial changes occur within neocortical processing areas, and these changes appear to modify neural activation in response to experiences. According to theories of neuroplasticity, experiences can fundamentally alter both the physical structure and functional organization of the brain.

One of the most remarkable outcomes of neuroplasticity is that brain activity associated with a given function can relocate to different regions. This phenomenon commonly occurs as a result of experience or during the recovery process following brain injury. In conjunction with goal-directed experiential rehabilitation programs, neuroplasticity serves as a fundamental scientific foundation supporting rehabilitative approaches to mitigate the functional consequences of brain injuries.

The adult brain is not a rigid system with fixed or immutable neural circuits. Numerous studies have demonstrated cortical and subcortical rewiring of neural circuits in response to training. Additionally, there is substantial evidence supporting adult neurogenesis in mammalian brains, and such changes have been observed even at advanced ages. While evidence for neurogenesis has been primarily confined to the hippocampus and the olfactory bulb, recent studies suggest that other brain regions, including the cerebellum, may also be involved.

A wide range of technologies-including electrophysiology, optical imaging, magnetic resonance imaging (MRI), optogenetics, and chemogenetics-have provided new methodologies for studying the structure and function of neural circuits. However, the existing neuroscience toolkit still fails to meet a critical experimental need: the ability to observe and perturb whole-brain-scale neural circuit dynamics in behaving mammals. Furthermore, most neuroscience techniques are not applicable to humans without an unacceptable level of invasiveness. These limitations primarily arise from the underlying physics that governs the interaction between various energy modalities and biological subtances.

Spiking neuron recordings require probes to be positioned within approximately 200 $\mu$m of the cell, which-along with the limited size of probes-restricts the density and scope of in vivo electrophysiology. Visible light is typically scattered within 1 mm of tissue, making it challenging to precisely resolve or target optical signals beyond this depth. The weak polarization of nuclear spins constrains signal strength, thereby limiting the spatial resolution of functional MRI (fMRI). Additionally, the difficulty in focusing and localizing electromagnetic fields deep within the brain reduces the precision of electrical or magnetic stimulation applied externally and hinders the accurate identification of stimulation sources.

Radiotracer probes are limited by pharmacokinetics and the spatial dispersion of emissions. Similarly, chemogenetic tools generally require invasive delivery methods to function in anatomically defined regions.

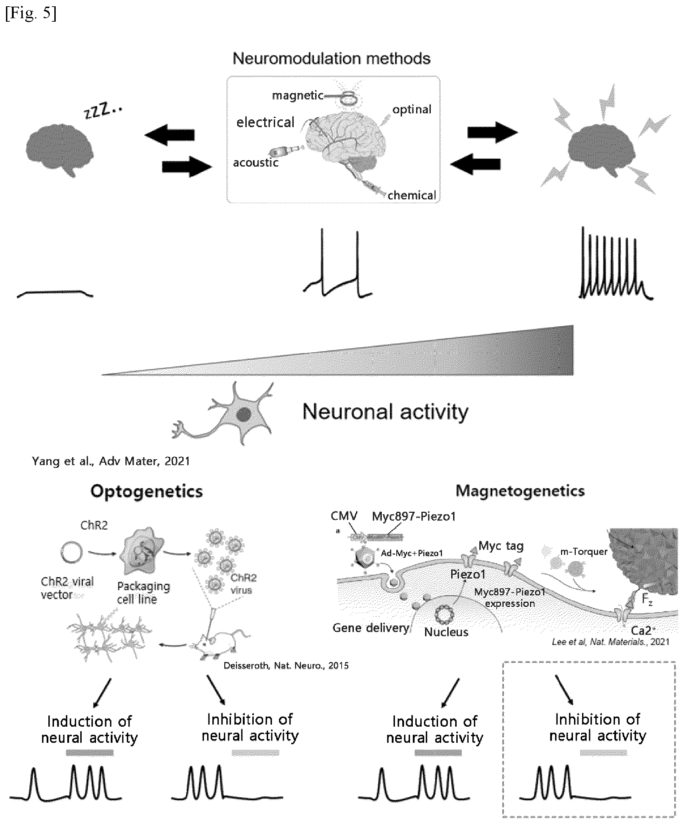

Optogenetics and chemogenetics have recently been developed as biological approaches that enable cell-specific neuromodulation through the expression of ion transport membrane proteins in specific neuronal populations (Fig. 5). Optogenetic methods, which activate ion transport membrane proteins using light, offer advantages in speed and precise targeting. However, the delivery of light into deep tissues remains a challenge. Most mammalian tissues exhibit low optical transparency, necessitating the use of relatively invasive techniques to effectively deliver light to target cells.

In contrast, magnetic fields can penetrate bones and tissues without significant attenuation, enabling magnetic stimulation to reach ion transport membrane proteins and magnetic nanoparticles located within the body, even when a magnetic field generator is positioned at a distance (~70 cm) from the target. This characteristic suggests that magnetogenetics offers a less invasive alternative to optogenetics for modulating in vivo proteins, as it does not require surgical intervention or tissue damage, particularly in central nervous system (CNS) structures such as the brain.

Meanwhile, chemogenetics involves injecting chemical agents into the bloodstream to activate membrane proteins expressed in target cells, thereby modulating neural activity. While chemogenetic approaches are considered relatively non-invasive, they have a key limitation: the onset and duration of neuromodulation are prolonged (typically lasting 30 minutes to 2 hours) following chemical administration, preventing precise temporal control over neural activity.

In 1875, Richard Caton first detected electrical currents in the exposed brains of animals using a galvanometer, marking the beginning of electrical stimulation-based neuromodulation. Initially, researchers randomly inserted electrodes into the intact brain to stimulate different regions. Over time, this technique evolved to target specific neural circuits associated with particular behaviors or cognitive functions.

Electrical stimulation methods initially developed for research purposes were later adapted for therapeutic applications. A notable example is deep brain stimulation (DBS), a technique that received U.S. FDA approval in 1997 and has since been widely used to treat various neurological disorders, including Parkinson's disease, dystonia, and obsessive-compulsive disorder (OCD). DBS is believed to modulate neural activity by increasing the output of deep brain nuclei, exerting a combined excitatory and inhibitory effect that influences surrounding neural fibers. Through this mechanism, DBS is understood to regulate the basal thalamocortical tract.

The relatively precise and non-invasive transcranial magnetic stimulation (TMS) has been applied not only to non-human primates but also to humans. Recently, TMS has been utilized to stimulate neural activity in patients with treatment-resistant depression, improving depressive symptoms when conventional antidepressant medications fail. Therapeutic TMS often involves delivering repetitive magnetic pulses, a technique referred to as repetitive TMS (rTMS).

For more precise magnetic stimulation techniques, it is essential to establish a functionally based anatomical brain map. Direct magnetic stimulation of the brain has been shown to induce measurable effects on perception and behavior. However, electrical stimulation-while effective in enhancing neuronal activity-has limitations in inhibiting neural function, leading to the development of alternative neuromodulation techniques such as optogenetics and chemogenetics.

Ultrasound (US) is a form of energy with a wavelength of approximately 100 $\mu$m, allowing it to easily penetrate soft tissues. Accordingly, US-based methods can generate images with spatial precision corresponding to this wavelength or deliver focused energy up to several centimeters deep into tissues. Additionally, due to its high acoustic wave propagation speed (~1.5 km/s in soft tissue), US operates with temporal precision of less than 1 ms. These advantages have made ultrasound imaging one of the most widely used technologies in clinical medicine, owing to its relatively low cost, high portability, and safety. At the same time, focused ultrasound (FUS)-the only form of non-ionizing energy capable of penetrating deep tissues-has rapidly emerged as a non-invasive therapeutic modality for ablative treatments of cancer and neurological disorders, driven by advancements in U.S. hardware and image-guided technologies.

These fundamental capabilities have motivated significant efforts to leverage ultrasound as a modality for large-scale brain imaging and neuromodulation. As a result, high-resolution hemodynamic functional imaging, focused ultrasound neuromodulation, and localized drug delivery have already been developed as useful neuroscience tools.

The brain is a biologically complex and compartmentalized organ, with its complexity arising from the intricate interactions of neuronal networks. Technologies for studying, controlling, and stimulating neurons play a crucial role in neuroscience and medicine, and advancements in this field contribute to the treatment of neuron-based diseases and a deeper understanding of brain function.

To stimulate specific neurons at desired times, researchers have explored various physical signals that can interact with biological tissues, including electric fields, light, magnetic fields, and ultrasound (US).

As previously described, optogenetics requires invasive surgical procedures to deliver light of a specific wavelength to target neurons for activation or inhibition.

Following optogenetics, which involves activating specific neurons using light stimulation to study their functions, a new technique known as sonogenetics (or acoustic genetics) has emerged, enabling the activation and inhibition of neuronal activity using sound waves.

Researchers at the Salk Institute have experimentally demonstrated that ultrasound stimulation can function as a switch to activate or inhibit specific neurons. They reported the feasibility of sonogenetics by conducting experiments in *Caenorhabditis elegans* (*C. elegans*), where they engineered specific neurons to express an ultrasound-sensitive gene. Upon applying amplified ultrasound stimulation, they observed neuronal activation. The researchers utilized TRP-4, a channel protein that, upon ultrasound stimulation, opens membrane ion channels, allowing the influx of ions that serve as signaling molecules for neuronal activation. To test this mechanism, they genetically introduced TRP-4 expression into specific neurons of *C. elegans.* When amplified ultrasound stimulation was applied, neuronal activation and inhibition were observed, resulting in alterations in the worm's movement direction (https://www.youtube.com/watch?v=rUhgUq5VIJo).

In optogenetics, delivering light stimulation requires implanting an optical fiber into the brain-an invasive method. In contrast, sonogenetics offers the advantage of utilizing ultrasound, which can penetrate deep into the body without the need for surgical intervention. Similar to how optogenetics requires light-sensitive ion channel proteins (genes), sonogenetics also necessitates the introduction of ultrasound-sensitive ion channel proteins (genes) into the target organism.

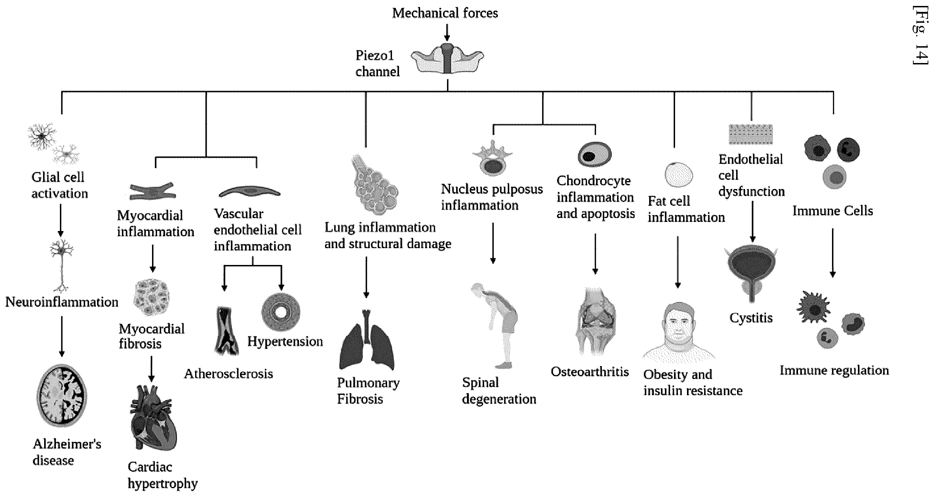

Ultrasound has higher tissue penetration compared to electric fields or light, making it non-invasive, generally safe, and compatible with imaging technologies, which presents significant advantages. However, ultrasound-based brain stimulation typically employs focused ultrasound (FUS) to selectively target specific neurons. This approach can lead to thermal side effects due to heat generation and lacks cell-type specificity, resulting in low spatial resolution. As a result, safety concerns and spatial resolution limitations pose challenges for ultrasound-based neuromodulation (see Fig. 14).

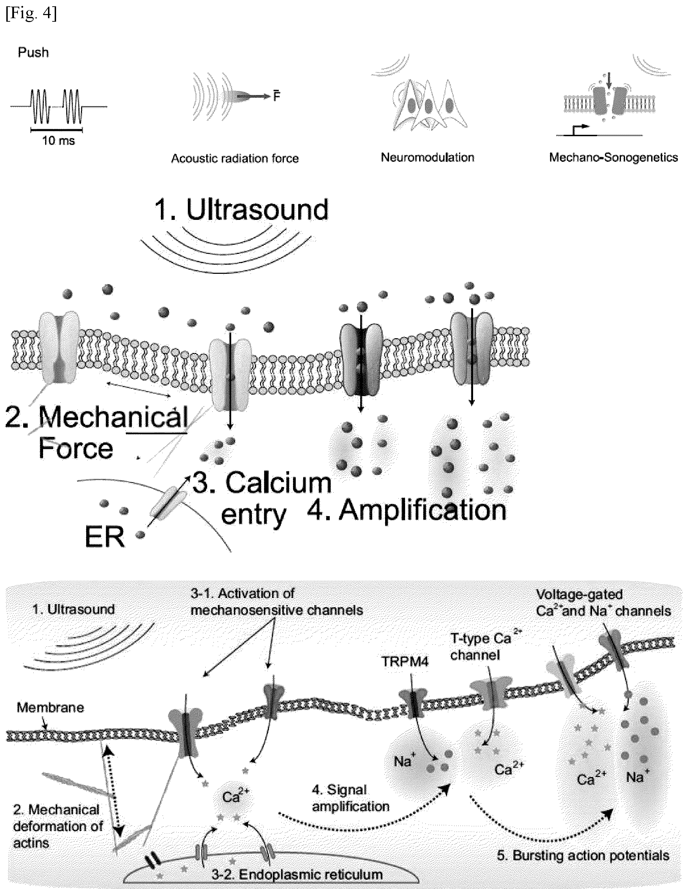

Applying focused ultrasound (FUS) continuously at the millisecond scale can generate mechanical forces, leading to cellular or molecular activation (Fig. 4). Prolonged focused ultrasound exposure accumulates thermal energy in tissues, which can activate temperature-dependent molecular functions.

Studies have demonstrated that FUS parameters (>100 ms and >3 W/cm$^2$) can induce direct and reversible neural responses (Fig. 4), without involving significant cavitation, heating, large-scale mechanical deformation, or synaptic transmission.

Instead, FUS has been shown to induce calcium accumulation through specific mechanosensitive ion channels, leading to the activation of calcium-dependent sodium channels and low-threshold calcium channels, ultimately triggering significant neuronal excitation (Fig. 4). Pharmacological and genetic perturbation studies have identified TRPP1/2, TRPC1, and TRPM4 as ion channels involved in FUS responsiveness. Overexpression of these channels has been shown to increase neuronal sensitivity to ultrasound stimulation. While these findings provide evidence that neurons themselves can respond to FUS, other studies suggest that astrocytes may act as additional or alternative cell types involved in ultrasound neuromodulation (UNM).

Regarding UNM, early studies in small animals have shown that motor responses do not always align with the expected spatial targeting of FUS. For instance, unilateral stimulation of the motor cortex has been observed to induce bilateral limb movements, and targeting non-motor cortical areas has resulted in maximal motor responses. Given the small size of a rodent's head relative to the FUS beam, combined with millisecond-scale pulse durations used in UNM, it is possible that standing waves are formed due to multiple reflections of ultrasound within the skull, leading to reduced spatial specificity.

Furthermore, FUS may not only exert a direct neuromodulatory effect on the targeted brain region but may also vibrate the inner ear, indirectly activating auditory pathways, suggesting an alternative mode of action for FUS-based neuromodulation.

**DETAILED DESCRIPTION OF THE INVENTION**

**TECHNICAL PROBLEM**

To address the aforementioned challenges and enable precise spatiotemporal control over the opening and closing of target mechanosensitive ion channels, the present invention proposes screening the resonance frequency (or frequencies) of specific mechanosensitive ion channels in target cells within a designated body region. These resonance frequencies are distinct from the frequency range of focused ultrasound and are selected within the ultrasound frequency range of 5 to 100 MHz, preferably 0.5 to 10 MHz. The identified resonance frequencies of target mechanosensitive ion channels are then used to generate an acoustic stimulation pattern, as a form of bioelectronic medicine, delivering mechanical stimulation patterns to modulate the function of the target ion channel.

Furthermore, the present invention aims to provide a non-invasive brain function control device that utilizes highly tissue-penetrant acoustic waves to activate or inhibit target neural circuits or neural networks.

**TECHNICAL SOLUTION**

A first aspect of the present invention provides a brain function control device for non-invasively activating or inhibiting a target neural circuit or neural network, wherein the device is configured to generate an acoustic wave pattern that utilizes the resonance frequency(ies) of a target mechano-sensitive ion channel within a specific neuron in a target brain region to modulate the opening and closing of the target mechano-sensitive ion channel.

A second aspect of the present invention provides a mechano-sensitive ion channel gating control device for a target mechano-sensitive ion channel in a biological lipid membrane, wherein the device is configured to generate an acoustic wave pattern utilizing the resonance frequency(ies) of a specific mechano-sensitive ion channel within a specific cell in a target body region to modulate the opening and closing of the target mechano-sensitive ion channel.

A third aspect of the present invention provides computer-readable recording medium or a medium for transmitting a program to a computer for use in a brain function control device of the first aspect or the mechano-sensitive ion channel gating control device of the second aspect, wherein the medium stores or transmits a program executable by a processor to generate an acoustic wave pattern optimized for the resonance frequency(ies) of a target mechano-sensitive ion channel in order to activate or reversibly inhibit the target mechano-sensitive ion channel within a specific body region.

A forth aspect of the present invention provides an acoustic wave oscillator, operably controlled by the program of the third aspect, in the brain function control device of the first aspect or the mechano-sensitive ion channel gating control device of the second aspect, wherein the medium stores or transmits a program executable by a processor to generate an acoustic wave pattern optimized for the resonance frequency(ies) of a target mechano-sensitive ion channel in order to activate or reversibly inhibit the target mechano-sensitive ion channel within a specific body region.

The invention is further described below.

As used herein, the term "target mechano-sensitive ion channel in a biological lipid membrane" refers to a specific mechanosensitive ion channel present in specific cells within a target body region. For example, it may refer to a specific mechanosensitive ion channel within specific neurons in a target brain region. By controlling the opening and closing of mechanosensitive ion channels in neurons within a target brain region, it is possible to activate or inhibit the corresponding neural circuits or neural networks, thereby enabling the modulation of various brain functions.

As used herein, the term "neuromodulation" refers to a process in which a mechanosensitive ion channel undergoes conformational changes in response to an acoustic stimulation pattern that utilizes the resonance frequency (or frequencies) of a specific mechano-sensitive ion channel within a specific cell in a target body region. This conformational change results in the opening or closing of the mechanosensitive ion channel, leading to the release of neural signals from the neuron.

As used herein, the phrase "activating or inhibiting a target neural circuit or neural network" encompasses the modulation of the intricate interactions of neuronal networks within the highly complex and compartmentalized brain.

As used herein, the specific mechanosensitive ion channel targeted for opening or closing modulation may be naturally expressed in specific cells within the target body region, or it may be artificially expressed and/or genetically engineered using sonogenetic technology to possess a predetermined resonance frequency, in a specific cell within the target body region.

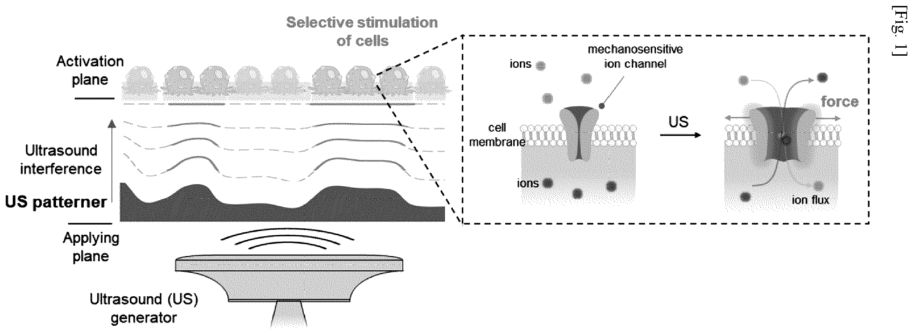

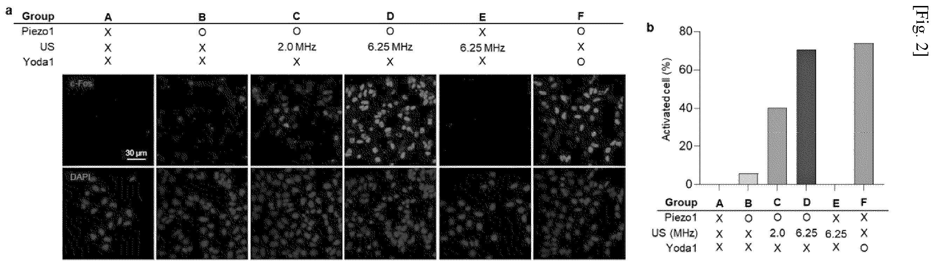

The inventors screened the resonance frequency(ies) of target mechano-sensitive ion channels located in the biological lipid membrane within an ultrasound frequency range distinct from the focused ultrasound (FUS) frequency range, specifically between 0.5 MHz and 100 MHz, preferably between 0.5 MHz and 20 MHz, and more preferably between 0.5 MHz and 10 MHz. They then utilized an acoustic wave pattern based on the identified resonance frequency(ies) of the target mechano-sensitive ion channels as an electroceutical providing mechanical stimulation patterns. Furthermore, the inventors developed a sonogenetics technique capable of stimulating the brain with high resolution and cellular specificity by employing neurons modified via genetic engineering techniques (Example 1, Figs. 1 and 2).

First, genetic engineering techniques are utilized to express a mechano-sensitive ion channel (Piezo1 ion channel) in neurons, thereby rendering target neurons in a specific region highly responsive to mechanical forces. Subsequently, an acoustic wave pattern utilizing the resonance frequency(ies) of the target mechano-sensitive ion channel located in the biological lipid membrane is applied. When the acoustic wave pattern based on the resonance frequency(ies) of the target mechano-sensitive ion channel is delivered, ultrasound propagates through biological tissues, generating mechanical forces. The mechanical force corresponding to the resonance frequency(ies) of the target mechano-sensitive ion channel (Piezo1) specifically stimulates the cells expressing the target mechano-sensitive ion channel (Piezo1). At this time, the magnitude of the mechanical stimulation pattern applied to neurons expressing the target mechano-sensitive ion channel (Piezo1) is carefully controlled to be weak enough to avoid altering the activity of non-target cells. As a result, precise spatiotemporal regulation of target neuron activity can be achieved without requiring any intracranial implants, while minimizing unintended effects on surrounding cells, thereby preventing side effects exemplified in Fig. 14.

The present invention is based on the discovery that when used as an electroceutical providing mechanical stimulation patterns, an acoustic wave pattern utilizing the resonance frequency(ies) of a target mechano-sensitive ion channel within a frequency range of 0.5 MHz to 100 MHz, preferably 0.5 MHz to 20 MHz, and more preferably 0.5 MHz to 10 MHz, which induces the resonance phenomenon of the target mechanosensitive ion channel located in the biological lipid membrane, enables high spatiotemporal resolution control over the opening and closing of a specific mechano-sensitive ion channel within a specific cell in a target body region (Example 1).

Depending on the type of mechano-sensitive ion channel, the type of cell in which the opening and closing of the mechano-sensitive ion channel is to be modulated, the body region or tissue in which the target mechano-sensitive ion channel is located (e.g., a specific brain region), or the physiological environment of the target body region (e.g., the tumor microenvironment), the resonance frequency(ies) of the acoustic wave inducing the resonance phenomenon of the target mechano-sensitive ion channel may vary. By precisely controlling the resonance frequency(ies) within the acoustic wave pattern, the opening and closing of a specific mechano-sensitive ion channel within a specific cell in a target body region can be spatiotemporally controlled with a defined sequence and high resolution.

For example, the resonance frequency(ies) of acoustic waves that induce the resonance phenomenon of a target mechano-sensitive ion channel located in the biological lipid membrane can be identified based on cell-specific reference values obtained from cells expressing the target mechano-sensitive ion channel or through in vitro experiments; or/and can be determined based on reference values acquired from various animal models or clinical studies or through in vivo experiments. Furthermore, patient-specific resonance frequency(ies) can be directly identified in real time at the point of care, allowing for real-time modulation of the acoustic wave pattern to match the identified frequency(ies).

Furthermore, the inventors first demonstrated that a Magnetic Torquer (m-Torquer) conjugate-based drug, in which (a) a magnetic nanoparticle capable of generating rotational force upon application of a controlled rotating magnetic field and (b) a binding moiety that specifically binds to a drug target protein present in the biological lipid membrane are linked either directly or via a linker, can magneto-mechanically stimulate specific populations of lateral hypothalamic (LH) neurons in deep brain regions, thereby modulating the behavior of freely moving animals (Example 3). In this regard, the disclosure of PCT/KR2023/012209 is incorporated herein by reference in its entirety.

Here, the magnetic nanoparticle that generates rotational force upon application of a rotating magnetic field (also referred to as m-Torquer) is a technology utilizing a rotating magnetic field and a magnetic nanoparticle (m-Torquer), specifically designed to induce rotational force from the magnetic nanoparticle via interaction with the rotating magnetic field. The magnetic stimulation associated with the m-Torquer conjugate functions as a magneto-mechanical stimulation, wherein, when the m-Torquer conjugate binds to a drug target protein, the rotational force generated by the magnetic nanoparticle upon application of a controlled rotating magnetic field is transmitted to the drug target protein, undergoing conformational changes in response to the magneto-mechanical force.

Specifically, Example 3, which utilizes the m-Torquer conjugate-based drug, establishes a novel mode of action (MoA) in which that magneto-mechanical stimulation of specific LH neuronal populations in the deep brain region controls the behavior of freely moving animals. In Example 3, the inventors utilized the Cre-loxp system to selectively express magnetogenetic ion channels in LH neurons, which play a key role in feeding behavior regulation. The study confirmed that cell-type specific expression of the target drug protein in Vgat (Slc32a1)-positive and Vglut2 (Slc17a6)-positive LH neurons, along with m-Torquer conjugate-based magneto-mechanical stimulation, could bidirectionally regulate feeding behavior. Moreover, the study demonstrated that transient modulation of feeding behavior confirmed reversibility. Furthermore, the results showed that sustained and repeated activation of specific LH neurons led to long-term anti-feeding effects, resulting in attenuation of obesity in obese mice. Lastly, the findings indicate that magnetogenetics can be adapted for various neuroscience studies, particularly for selective interrogation of the LH-VTA circuit in the context of social interactions under less restrictive conditions.

Example 3 establishes m-Torquer conjugate-based drugs as a significant neuromodulatory drug modality by employing a minimally invasive remote animal behavior control technology. This study utilizes a controlled rotating magnetic field generated by a rotating magnetic field generator, which induces rotational force in magnetic nanoparticles conjugated with a binding moiety targeting a drug target protein. Furthermore, Example 3 not only demonstrates a promising therapeutic approach for obesity by regulating feeding behavior and body weight, but also provides a versatile platform for controlling various animal behaviors, including social behavior.

Additionally, the m-Torquer conjugate-based drug was demonstrated in animal models to regulate neuronal activity in the lateral hypothalamus (LH), a central brain region for appetite control, thereby modulating feeding behavior and food intake. This study further established that the wireless, remote modulation of deep brain-specific cells was feasible and could deliver long-term stimulation (>10 days), outperforming existing deep brain modulation technologies such as optogenetics and chemogenetics.

The present invention leverages acoustic waves and mechanosensitive ion channels as a core technology. Instead of utilizing magnetic nanoparticles (m-Torquer) that generate rotational force upon application of a controlled rotating magnetic field-as demonstrated in Example 3-the present invention designs a bioelectronic medicine capable of delivering mechanical stimulation patterns through acoustic waves. Specifically, the invention employs acoustic wave patterns that utilize the resonance frequency(ies) of target mechanosensitive ion channels on the biological lipid membrane, ensuring that only the target mechanosensitive ion channels undergo conformational changes.

Accordingly, similar to the mechanism of action (MoA) demonstrated by the m-Torquer conjugate-based drug in Example 3, the acoustic wave pattern utilizing the resonance frequency(ies) of a target mechano-sensitive ion channel located in the biological lipid membrane-within a frequency range of 0.5 to 100 MHz, preferably 0.5 to 10 MHz-serves as an electroceutical providing mechanical stimulation patterns. This approach enables the precise spatiotemporal activation of individual neurons, particularly those deep within the brain, with a high degree of reliability, as well as achieving non-invasive activation or inhibition of target neural circuits or neural networks (Examples 1-3).

In summary, the target mechano-sensitive ion channel gating control device and brain function control device of the present invention are characterized by generating acoustic wave patterns that utilize the resonance frequency(ies) of a specific mechano-sensitive ion channel to modulate the opening and closing of the specific mechanosensitive ion channel in a particular cell of a target body region. Non-limiting examples of such specific cells include neurons, endocrine cells, epidermal cells, epithelial cells, muscle cells, immune cells, and cancer cells. **In** particular, the present invention is characterized by generating acoustic wave patterns that utilize the resonance frequency(ies) of a specific mechano-sensitive ion channel to modulate its opening and closing within a specific neuron in a target brain region.

The resonance frequency(ies) of mechanosensitive ion channels used in the acoustic wave patterns of the present invention may be in the ultrasound frequency range of 0.5-100 MHz, preferably 0.5-20 MHz, and more preferably 0.5-10 MHz.

According to the present invention, an acoustic wave pattern designed to modulate the opening and closing of target mechanosensitive ion channels by utilizing the resonance frequency(ies) of the target mechano-sensitive ion channel in a specific cell within a target body region provides a new drug modality by delivering mechanical stimulation to the target mechano-sensitive ion channel, which undergoes conformational changes due to resonance phenomena.

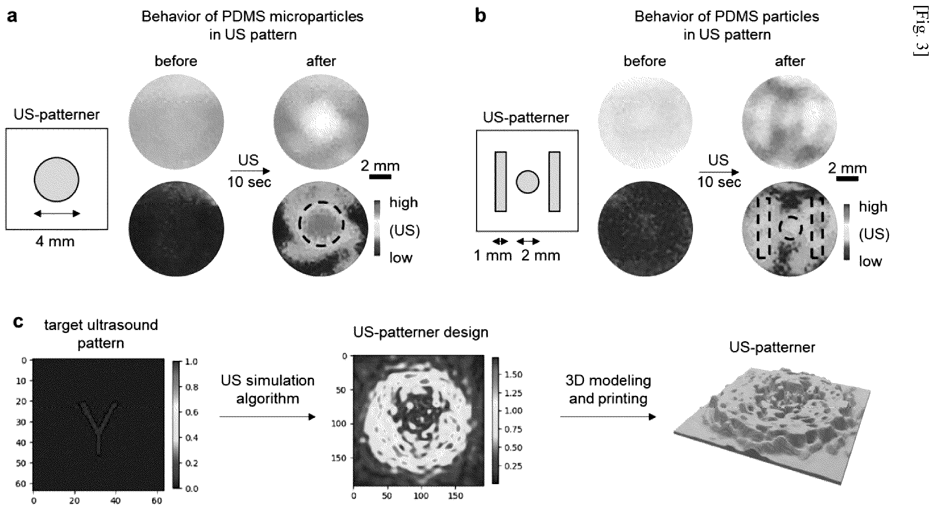

The present invention leverages the interference effects of ultrasound while inducing resonance phenomenon of a specific mechano-sensitive ion channel in a specific cell within a target body region, and generating one-dimensional (1D), two-dimensional (2D), or three-dimensional (3D) ultrasound patterns in a spatiotemporally controlled sequence at an intensity weak enough to avoid altering the activity of non-target cells (Example 2, Fig. 3).

As used herein, the phrase "weak enough to avoid altering the activity of non-target cells" refers, for example, to an intensity that does not induce an inflammatory response in non-target cells, as exemplified in Fig. 14.

The acoustic wave pattern of the present invention, as a type of electroceutical, not only provides the resonance frequency(ies) of a target mechano-sensitive ion channel but, in certain cases, also allows for the modulation of the resonance frequency(ies) of the target mechano-sensitive ion channel by controlling various stimuli or environmental factors in a specific cell within a target body region. Additionally, depending on various stimuli or microenvironmental conditions applied to a specific cell within the target body region, the acoustic wave pattern containing the resonance frequency(ies) of the target mechano-sensitive ion channel, can be dynamically modulated.

Furthermore, according to the present invention, an acoustic wave pattern that applies mechanical force to a target mechano-sensitive ion channel at its resonance frequency(ies) is transmitted to induce conformational changes in the target mechano-sensitive ion channel. The conformational changes or alterations in the biological activity of the target mechano-sensitive ion channel resulting from resonance phenomena are not only reversible but also allow for the precise temporal control and regulation of the degree of opening and closing of the target ion channel with high time resolution and accuracy.

Additionally, the mechano-sensitive ion channel gating control device of the present invention can be mounted onto a target body region of a freely moving animal and remotely controlled to generate an acoustic wave pattern utilizing the resonance frequency(ies) of a specific mechano-sensitive ion channel within a specific cell of the target body region to modulate the opening and closing of the ion channel. For example, the brain function control device of the present invention can be mounted onto the head of a freely moving animal and remotely controlled to generate an acoustic wave pattern that modulates the opening and closing of a specific mechano-sensitive ion channel in a specific neuron within a target brain region. Thus, an acoustic wave pattern utilizing the resonance frequency(ies) of a specific mechano-sensitive ion channel within a specific cell of a target body region, which is designed to control the gating of the mechano-sensitive ion channel, can be used to regulate neural circuits or behaviors in a freely moving animal.

Furthermore, according to the present invention, an acoustic wave pattern utilizing the resonance frequency(ies) of a specific mechano-sensitive ion channel within a specific cell of a target body region, which is designed to control the gating of the mechano-sensitive ion channel, can stimulate a specific neuronal cell in a target body region and induce the activation and/or inhibition of specific neurons. This mechanism enables the regulation of neurotransmitter secretion (e.g., glutamate, gamma-aminobutyric acid (GABA), dopamine, etc.), including whether neurotransmitters are released and their secretion levels, thereby modulating neuronal signaling to other excitatory cells.

Furthermore, according to the present invention, an acoustic wave pattern utilizing the resonance frequency(ies) of a specific mechano-sensitive ion channel within a specific cell of a target body region, which is designed to control the gating of the mechano-sensitive ion channel, can be used to modulate animal behavior or to prevent or treat physiological disorders or diseases through long-term, neuron-specific neuromodulation.

Accordingly, the present invention aims to employ an acoustic wave pattern utilizing the resonance frequency(ies) of the target mechanosensitive ion channel in order to regulate the opening and closing of a specific mechanosensitive ion channel in specific neurons within a target brain region. Specifically, the present invention reliably activates individual neurons, particularly those located deep within the brain, using sonogenetics, which switches neural activity on and off using sound waves, and amplified ultrasound stimulation as a neuronal activation switch.

Specifically, the present invention designs an optimized acoustic wave pattern (conditions) using the resonance frequency(ies) of a target mechanosensitive ion channel to activate or reversibly inhibit the ion channel in a desired region. The frequency and intensity of the acoustic waves are optimized in one-dimensional, two-dimensional, or three-dimensional configurations, and the interference effects of acoustic waves are utilized to modulate the acoustic wave pattern, thereby enabling the activation or reversible inhibition of a target mechanosensitive ion channel expressed in biological targets under various conditions. An ion channel that has been activated or reversibly inhibited by an acoustic wave pattern applied at a specific location and time alters the intracellular and extracellular ion concentrations in the targeted region, thereby modulating cellular signaling. This modulation can achieve pharmacological effects or mitigate off-target side effects.

Accordingly, the optimized acoustic wave pattern (conditions) using the resonance frequency(ies) of a target mechanosensitive ion channel to activate or reversibly inhibit the ion channel at a desired location can serve as a type of electroceutical-a therapeutic approach that stimulates neural circuits using ultrasound instead of drugs to regulate metabolic functions and restore or maintain physiological homeostasis.

Furthermore, the present invention mechanically stimulates target tissue-forming cells, thereby regenerating tissues, selectively modulating stem cells, or defining methods for cellular activation or inactivation at the cellular level, by utilizing acoustic wave patterns that employing the resonance frequency(ies) of a target mechano-sensitive ion channel to control the opening and closing of the target mechano-sensitive ion channel in a specific cell within a target body region and, if necessary, incorporating sonogenetic technology (sonogenetics).

Ultrasound-based techniques have previously failed to selectively activate neural stimulation in vertebrate models. While electrical and optical stimulation of photoresponsive ion channels has been reported as a heterologous approach for neural activation in specific brain regions, such techniques targeted BNST (bed nucleus of the stria terminalis) neurons rather than lateral hypothalamic (LH) neurons, which are central to appetite regulation. Additionally, the therapeutic indication of such approaches has been anxiety disorders rather than metabolic regulation.

Meanwhile, conventional gene delivery techniques result in non-selective gene expression in neuronal cells of the target brain region, thereby making it impossible to modulate the function of specific neural circuits. Similarly, conventional magnetogenetics techniques introduce genes into brain cells in a non-specific manner, making it difficult to precisely control specific brain cells and neural circuits.

In contrast, according to the present invention, an acoustic wave pattern utilizing the resonance frequency(ies) of a specific mechano-sensitive ion channel within a specific cell of a target body region, which is designed to control the gating of the target mechano-sensitive ion channel, allows for selective targeting of specific types of deep-brain neurons located in deep brain tissues and regulate their activation in a wireless, remote-controlled manner.

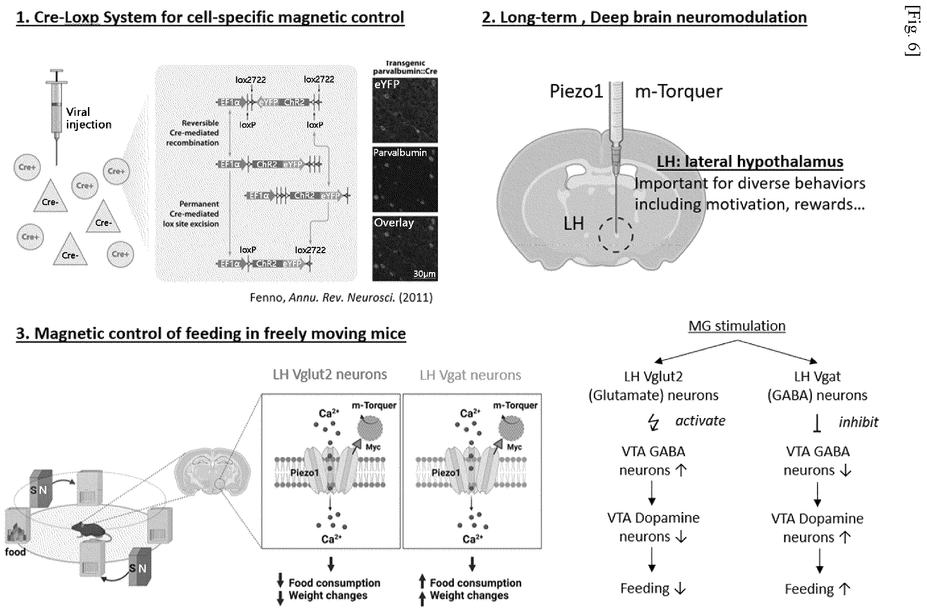

In Example 3, which serves as a reference for the present invention, the inventors demonstrated that cell-type-specific gene delivery technology enables selective gene expression and functional modulation of specific types of neurons. Furthermore, this study established that targeting deep brain regions where optogenetic applications are challenging and delivering long-term stimulation is feasible. Remarkably, the study validated that the key characteristics of magnetogenetics-remote, spatiotemporally precise, and neuron-specific neuromodulation-can be leveraged to treat metabolic disorders such as obesity, which are typically difficult to resolve in a short period (Figs. 6 to 11).

Accordingly, the pharmacological effects of the acoustic wave pattern of the present invention allow for targeted modulation of cellular signaling and/or neuronal activation, thereby enabling behavioral regulation, genetic therapy, alleviation of neurological disorders, obesity treatment, or anti-aging interventions.

As used herein, an acoustic wave pattern (condition) optimized for the resonance frequency(ies) of a target mechano-sensitive ion channel to activate or reversibly inhibit the channel refers to an acoustic wave pattern (condition) that achieves activation or reversible inhibition at a reliable level where pharmacological effects can be validated. Therefore, the drug targets of the acoustic wave pattern in the present invention are not limited to mechano-sensitive ion channels but also extend to drug target proteins present in the biological lipid membrane that undergo conformational changes in response to an acoustic wave pattern utilizing resonance frequency(ies) inducing resonance phenomena (e.g., receptors undergoing conformational changes upon ligand binding). These applications are also encompassed within the scope of the present invention.

For example, in order to activate or reversibly inhibit a target mechanosensitive ion channel at a desired site, the acoustic wave pattern (condition) optimized for the resonance frequency(ies) of the target mechanosensitive ion channel-according to the present invention-acts as a form of electroceutical that provides a mechanical stimulation pattern, thereby modulating the opening and closing of an ion channel, which is a drug target protein present in the biological lipid membrane.

Accordingly, in order to activate or reversibly inhibit a target mechanosensitive ion channel at a desired site, the acoustic wave pattern (condition) optimized for the resonance frequency(ies) of the target mechanosensitive ion channel-according to the present invention-may be used as a drug for physiological function regulation or behavioral modulation, wherein it mechanically and physically stimulates targeted neural cells or muscle cells (e.g., motor/glandular secretion) in a non-invasive manner.

[Resonance Frequency and Acoustic Wave Pattern]

Ultrasound (US) interacts with biological tissue through mechanical forces.

Ultrasound can easily penetrate the brain and, in some cases, the skull, interacting with tissues at a fundamental resolution of approximately 100 millimeters and 1 millisecond.

The brain functions as a dynamic cell culture system, in which neurons and glial cells are suspended in cerebrospinal fluid (CSF). Accordingly, Example 1, which was designed as an in vitro model to mimic this biological environment, demonstrated that the ultrasound pattern-which utilizes the resonance frequency(ies) inducing resonance phenomena of a target mechano-sensitive ion channel within a specific cell at a targeted anatomical site-can be tuned to a sufficiently low intensity so as not to alter the activity of non-targeted cells, while still effectively controlling the opening and closing of the target mechanosensitive ion channel.

As used herein, the term "acoustic wave pattern utilizing the resonance frequency(ies) of a specific mechano-sensitive ion channel within a specific cell in a target body region" refers to an optimized ultrasound frequency and intensity that is further modulated via acoustic interference patterns, thereby enabling selective gating control of mechanosensitive ion channels expressed in biological targets, for example, in specific target neurons within a target brain region.

Resonance refers to a phenomenon in which an object oscillates with a large amplitude at a specific frequency. This specific oscillation frequency is known as the resonance frequency, at which even a small external force can induce a significantly amplified oscillation and energy transfer.

All physical entities, including mechanosensitive ion channels and other drug target proteins, exhibit intrinsic oscillation frequencies at which they naturally vibrate. These intrinsic oscillation frequencies are referred to as natural frequencies. An object may have multiple natural frequencies, and when an external force is periodically applied at one of these natural frequencies, the object experiences a significant increase in oscillation amplitude-a phenomenon known as resonance.

When oscillating bodies are interconnected under resonant conditions, energy exchange between them occurs more efficiently.

Cells expressing mechanosensitive ion channels on the biological lipid membrane constitute the fundamental structural units of living organisms. These cells are organized into tissues, organs, and biological systems, collectively performing a wide range of biological processes essential for life. Cells play a critical role in survival and reproduction, serving as the fundamental building blocks that contribute to biological diversity and functional specialization. The size of cells varies widely, typically ranging from a few micrometers (μm) to several hundred micrometers. In humans, the average cell size is approximately 100 μm.

In multicellular organisms, cells undergo morphological and functional differentiation, typically assembling into groups of similar cell types arranged to perform specific functions. Such organized cell groups are referred to as tissues. In animals, tissues are classified into epithelial tissue, connective tissue, cartilage tissue, bone tissue, blood and lymph, muscle tissue, and nervous tissue based on their morphology and function.

Epithelial tissue covers the surfaces of the body, the lumens of the digestive and respiratory tracts, and the peritoneal and pericardial cavities, forming a single-layered or multilayered sheet of tightly packed cells with minimal intercellular space. In some cases, epithelial tissue folds inward to form glandular tissues composed of secretory cells, while certain specialized epithelial structures, such as sensory epithelium in the visual, auditory, and vestibular systems, as well as structures like hair and nails, exhibit unique functional properties.

Connective tissue, cartilage tissue, bone tissue, blood, and lymph are collectively referred to as supporting tissues, which are characterized by an abundance of extracellular matrix. These tissues function to maintain the structural integrity of the body and organs. The extracellular matrix consists of fibers and a ground substance, with cells embedded within it. Blood and lymph are classified as supporting tissues because plasma and lymph fluid serve as the ground substance, while fibrin is considered the fibrous component of the extracellular matrix.

Muscle tissue consists of muscle cells specialized for contraction. These muscle cells, also known as muscle fibers, exhibit a long and slender fiber-like morphology due to their structural organization.

Nervous tissue comprises neurons (nerve cells) and glial cells (neuroglia) and functions as a wired communication system for transmitting biological information. In higher-order animals, the brain and spinal cord constitute the central nervous system (CNS), while the nerves branching from it form the peripheral nervous system (PNS). The CNS consists of nervous tissue combined with blood vessels and connective tissue, whereas the PNS is primarily composed of neurons, nerve fibers, and Schwann cells (a type of glial cell) that envelop the fibers.

The biological lipid membrane environment varies significantly depending on the type of organ, tissue type, body region, cell type, and pathological state. Notably, lipid rafts, unique microdomains within the cell membrane, have been observed. These lipid rafts serve as critical hubs for signal transduction, where a variety of drug target proteins involved in cellular signaling are concentrated. Consequently, lipid rafts play a crucial role in signal transduction, protein sorting, and membrane transport.

Accordingly, the resonance frequency(ies) of ultrasonic waves that induce the resonance phenomenon of target mechanosensitive ion channels-which are dynamically embedded within various biological lipid membranes-varies depending on multiple factors, including the type of mechanosensitive ion channel, the cell type in which the gating of the mechanosensitive ion channel is to be regulated; the targeted anatomical region or tissue, such as a specific brain area, the physiological or pathological environment of the targeted body region, including tumor microenvironments or inflammatory conditions.

Additionally, the present invention utilizes the interference effect of ultrasound to induce the resonance phenomenon of target mechanosensitive ion channels while ensuring that the stimulation remains weak enough to avoid altering the activity of non-target cells. Specifically, one-dimensional (1D), two-dimensional (2D), or three-dimensional (3D) ultrasonic wave patterns-optimized for the target mechanosensitive ion channels-are designed to minimize heat generation in surrounding tissues while precisely controlling the gating of the target mechanosensitive ion channels. Accordingly, the acoustic wave pattern of the present invention, functioning as a type of electroceutical that delivers mechanical stimulation patterns, selectively modulates and stimulates specific cells within the targeted anatomical region. For example, within a target brain region, the acoustic wave pattern can specifically control and activate designated neurons

The ultrasound pattern provided by the present invention functions as a type of electroceutical, allowing for the design of an appropriate dose-dosage regimen for its application.

Dose refers to the amount of a drug administered at one time. The appropriate dose is typically prescribed by a healthcare provider based on factors such as the patient's age, weight, and overall health condition. Dosage, on the other hand, refers to the frequency and duration of drug administration. It measures the total amount of the drug delivered over a given period and is typically expressed in daily or weekly amounts. Both dose and dosage are critical considerations for ensuring the safe and effective use of a therapeutic intervention.

By adjusting the dose-dosage parameters of the mechanical stimulation induced by the acoustic wave pattern (i.e., electroceutical therapy) provided in the present invention, it is possible to not only modulate synaptic potential changes but also activate or inhibit target neural circuits or neuronal networks.

The one-dimensional (1D), two-dimensional (2D), or three-dimensional (3D) acoustic wave patterns of the present invention, which enable spatiotemporally selective control and stimulation of specific cells within a target body region, including specific neurons within a target brain region, can be precisely engineered using an ultrasound patterner. For example, the acoustic wave pattern may incorporate structured ultrasound patterning, which can include predefined ultrasound sequences optionally interspersed with arbitrarily defined rest periods.

Fig. 3 illustrates a set of ultrasound interference images of specific shapes, multiple ultrasound pulses, and/or an ultrasound sequence with intensity gradients, which are implemented to provide a diverse range of acoustic wave patterns that utilize the resonance frequency(ies) of a specific mechano-sensitive ion channel within a specific cell in a target body region to control the opening and closing of the ion channel in specific cells that express it (Example 2).

One of the simplest ways to conceptualize various ultrasound sequences available for use in an ultrasound patterner is to categorize them based on their dominant effects on the shape of the target body region, such as a target brain region, while considering factors such as inflammation, fat, or fluid attenuation in different physiological environments.

[Hypothalamus as a Target Brain Region and the Role of Neurohormones]

The hypothalamus is located beneath the two thalami, positioned just above the brainstem. Anatomically, it is part of the diencephalon and is situated at the ventral center beneath both thalami. The hypothalamus is a structure found in the brains of all vertebrates, and in humans, it is approximately the size of an almond.

One of the primary functions of the hypothalamus is to bridge the nervous system and the endocrine system via the pituitary gland.

The hypothalamus regulates metabolic processes and controls the autonomic nervous system (ANS). Additionally, it synthesizes and secretes neurohormones, which, in turn, stimulate or inhibit the release of pituitary hormones. As a result, the hypothalamus plays a crucial role in regulating body temperature, hunger, thirst, fatigue, sleep, and circadian rhythms.

Neurohormones are a specialized class of hormones produced by neurons (nerve cells) and released into the bloodstream or extracellular fluid. Unlike conventional hormones, which are typically produced by endocrine glands, neurohormones are synthesized within specialized neurons and are released in response to specific neural signals.

The term "neurohormone" encompasses a broad range of signaling molecules that exhibit hormone-like functions and are involved in the regulation of various physiological processes. Similar to classical endocrine hormones, neurohormones can act on distant target cells or organs, eliciting specific physiological responses. Neurohormones are generally classified into two major types: releasing hormones and inhibiting hormones.

Releasing hormones are neurohormones that stimulate the secretion of other hormones from endocrine glands. For instance, the hypothalamus produces and releases various releasing hormones that regulate hormone secretion from the pituitary gland. These releasing hormones travel through the bloodstream to the pituitary gland, where they stimulate the release of specific hormones such as growth hormone (GH), thyroid-stimulating hormone (TSH), or adrenocorticotropic hormone (ACTH).

Inhibiting hormones, on the other hand, function to suppress the secretion of specific hormones. The hypothalamus also produces inhibiting hormones that regulate hormone secretion from the pituitary gland. These hormones act on the pituitary to reduce the secretion of specific hormones, thereby providing negative feedback regulation.

Neurohormones can also play a crucial role in modulating behavior, emotions, and other aspects of neural function. For example, the neuropeptide oxytocin functions as a neurohormone, released from specific neurons in the hypothalamus. Oxytocin is involved in social bonding, maternal behavior, and the regulation of reproductive functions.

The release and regulation of neurohormones are tightly controlled and can be influenced by neural signals, feedback mechanisms, and external stimuli. Neurohormones often exhibit pulsatile or rhythmic patterns of secretion, allowing for precise and coordinated responses in the body.

As essential signaling molecules, neurohormones bridge the gap between the nervous and endocrine systems. They play a critical role in regulating physiological processes, maintaining homeostasis, and coordinating various bodily functions.

The lateral hypothalamus (LH) is a region of the hypothalamus, which is centrally located in the brain and plays a critical role in regulating various physiological functions and maintaining homeostasis.

The lateral hypothalamus is primarily involved in the regulation of appetite and energy balance. Activation of this region induces hunger and the drive to eat, promoting food intake and meal consumption. Conversely, damage to this area can lead to reduced appetite, weight loss, and anorexia-related symptoms

The lateral hypothalamus (LH), also referred to as the lateral hypothalamic area (LHA), contains primary orexinergic nuclei, which are widely projected throughout the nervous system. This neural network mediates various cognitive and physiological processes, including feeding behavior, arousal promotion, pain perception reduction, thermoregulation, digestive function, and blood pressure control. Clinically significant disorders associated with dysfunctions of the orexinergic projection system include narcolepsy, movement disorders, functional gastrointestinal disorders related to visceral hypersensitivity (e.g., irritable bowel syndrome), and feeding disorders. The key neurotransmitters and neuromodulators involved in the activity of orexin neurons include glutamate, endocannabinoids (e.g., anandamide), and the orexin neuropeptides-orexin A and orexin B. Pathway-specific neurochemical mediators include GABA, melanin-concentrating hormone (MCH), nociceptin, glucose, dynorphin peptides, and the appetite-regulating peptide hormones leptin and ghrelin. Notably, cannabinoid receptor 1 (CB1) is co-localized with orexinergic projection neurons in the lateral hypothalamus and many output structures, indicating that CB1 and orexin receptor 1 (OX1) are co-expressed. The body forms CB1-OX1 receptor heterodimers.

**[Nervous system, neurons, and neural circuits]**

According to the present invention, a brain function control device that non-invasively activates or inhibits a target neural circuit or neural network is characterized by generating an acoustic wave pattern utilizing the the resonance frequency(ies) of a target mechanosensitive ion channel to modulate the opening and closing of a specific mechanosensitive ion channel in a specific neuron within a target brain region.

Furthermore, in accordance with the present invention, a mechano-sensitive ion channel gating control device for a target mechano-sensitive ion channel in a biological lipid membrane is characterized by generating an acoustic wave pattern that utilizes the resonance frequency(ies) of a mechanosensitive ion channel to regulate the opening and closing of a specific mechanosensitive ion channel in a specific neuron or glial cell within a target body region.

The brain is the organ responsible for generating behavior. Behavior emerges at the level of the nervous system, where neurons aggregate to form neural circuits, and the interconnections of these circuits constitute the nervous system.

A neural circuit is a group of interconnected neurons that, when activated, perform a specific function via synaptic connections. Neural circuits are interconnected to form large-scale brain networks.

The nervous system is primarily composed of neurons, which serve as the fundamental units of biological information processing, and glial cells, which provide structural and functional support. Among these, neurons are uniquely responsible for information processing, a function exclusively performed by the brain.

The brain generates voltage pulses.

Neurons of brain are classified based on function. Afferent or sensory neurons provide input to the nervous system, such as the optic nerve. Motor neurons relay control signals to muscles and glands. Interneurons process local information and propagate signals between different regions, making up the largest population of neurons within the nervous system.

A neuron consists of dendrites, a soma (cell body), an axon, and terminal buttons. Dendrites are the sites where chemical signals (neurotransmitters) released by other neurons are converted into electrical signals. The soma (cell body) performs vital functions necessary for the neuron's survival and acts as a central processing unit, integrating electrical signals received through the dendrites and determining whether to propagate the signal to the next neuron. The axon propagates the electrical signal to the axon terminals, where neurotransmitters are released at the synapse in response to the electrical signal.

The soma consists of the cell nucleus and perikaryon, with an average diameter of approximately 50 $\mu$m.

The axon is a tubular structure with a diameter ranging from 0.2 to 20 $\mu$m, and some axons can extend up to 1 meter in length. The axon originates from the axon hillock, where the action potential is generated, serving as the primary conduction mechanism of the neuron.

Dendrites are tree-like branched structures, with most neurons possessing multiple dendrites. The dendrites of a neuron form synaptic connections with the axons of other neurons, thereby creating a biological network. Dendrites function as the antennae of the neuron, and their spine structures undergo modifications based on the type and intensity of synaptic activity. There are two types of dendrites: apical dendrites; and basal dendrites, which facilitate both excitatory and inhibitory functions in axonal signal generation.

A cell that generates a signal is referred to as a presynaptic cell, while a cell that receives a signal is called a postsynaptic cell. The end of an axon is divided into presynaptic terminals, which serve as the primary transmission mechanism of a neuron. A synapse is the junction where the axonic terminal of a presynaptic neuron connects to the axon of a postsynaptic neuron. A single neuron can form between 1,000 to 10,000 synapses.

The morphology of neurons is classified based on the number of processes extending from the cell body (soma) as follows. Unipolar cells lack dendrites extending from the soma. They possess only a single primary process and are typically found in invertebrates. Bipolar cells have two distinct processes-one dendrite and one axon. Multipolar cells constitute the majority of the vertebrate nervous system, consisting of a single axon and one or more clusters of dendrites.

The generation of synaptic potentials involves complex chemical and electrical processes. A sensory or chemical stimulus induces changes in synaptic potential, allowing one neuron to influence the state of another neuron. Within the soma (cell body), these signals are integrated to determine whether an axon potential will be generated, enabling both excitation and inhibition. One of the most fascinating aspects of neuronal function is the conversion of graded input action potentials into an all-or-nothing output at the soma. This property has been modeled in various artificial neural networks and computational models. If the total accumulated stimulus is below the threshold, no action potential is generated. If the total accumulated stimulus exceeds the threshold, an action potential is triggered, and the magnitude of the output remains constant regardless of how much the threshold is exceeded. This all-or-nothing principle is a fundamental characteristic of neuronal function and is independent of the specific type of neuron.

The action potential of an activated neuron is a spiked signal, where its frequency is proportional to the soma's potential. If the soma potential of a neuron exceeds a certain threshold value, the neuron begins firing. Accordingly, an action potential can induce changes in the potential of associated neurons. The average frequency of action potentials is referred to as the neuron's mean firing rate. The ratio of the mean soma potential to the mean resting soma potential is defined as the neuron's activation level.

Neurons communicate with many other neurons, and the integration of multiple stimuli received by a post-synaptic cell determines its firing pattern of action potentials.

According to the present invention, the acoustic wave pattern that utilizes the resonance frequency(ies) of a target mechanosensitive ion channel in a specific cell within a target body region to modulate the opening and closing of the target mechanosensitive ion channel, can stimulate the target mechanosensitive ion channel naturally or artificially expressed in the postsynaptic cell. As a result, this enables the generation of a desired action potential firing pattern in the postsynaptic cell.

Table 1 below presents specific parameters of the electro-neural process, including receptors, synaptic activity, and action potentials.

[Table 1]

| Feature | Receptor potential | Synaptic potential | Action potential |
| --- | --- | --- | --- |
| Amplitude | 0.1 - 10 mV | 0.1 - 10 mV | 70 - 110 mV |
| Duration | 5 - 100 ms | 5 ms to 20 min | 1 - 10 ms |
| Resolution | Graded (continuous) | Graded | Bilevel (all or none) |

When a neurotransmitter is said to be excitatory, it means that the soma potential of the receiving neuron is increased. Conversely, when a neurotransmitter is inhibitory, it means that it either lowers the soma potential of the receiving neuron or prevents it from increasing. A special case of inhibition occurs through presynaptic inhibition, which is mediated by synapses located on presynaptic nerve fibers or synaptic knobs. This type of inhibition results in a gradual reduction in the magnitude of action potentials at the synapse. The overall effect is an attenuation of signal transmission, which helps regulate neuronal activation. In contrast, postsynaptic inhibition functions as a negative feedback mechanism that prevents excessive propagation of activation within neural circuits.

In summary, a biological neuron consists of three main components, soma (cell body), axon and dendrites. The axon serves as the output channel of the neuron, transmitting action potentials along the nerve fibers via synaptic connections with other neurons. The dendrites function as input receptors, receiving signals from other neurons and relaying the input potential to the soma through postsynaptic mechanisms. The soma acts as an accumulator or amplifier.

The intelligent behavior observed in biological systems results from the fusion of thousands to billions of microscopic biological processing units, such as neurons.

Glial cells include oligodendrocytes, Schwann cells, astrocytes, radial glial cells, and microglia. Oligodendrocytes and Schwann cells are responsible for forming myelin sheaths. Astrocytes support neuronal maintenance, information transmission, and blood-brain barrier (BBB) formation. Radial glial cells guide neuronal migration during neurodevelopment. Microglia function as immune cells.

The myelin sheath surrounding an axon is formed by glial cells. While some neurons lack myelination, axons with myelin sheaths conduct signals approximately 100 times faster than unmyelinated axons. Consequently, myelination is highly efficient for transmitting signals over long distances. This mode of electrical signal propagation is referred to as saltatory conduction.

Neurons function as voltage converters, transforming analog voltage waves received through multiple neural processes into digital voltage pulses. Each neuron forms thousands to tens of thousands of synaptic connections with other neurons. The region where a neuron's processes meet another neuron's dendrite or axon is called a synapse, where neurotransmitter diffusion is converted into voltage wave transmission.

Accordingly, the brain function control device of the present invention regulates the activation of neurons involved in information transmission and facilitates the understanding of brain function by modulating neuronal activity. This is achieved through an acoustic wave pattern that utilizes the resonance frequency(ies) of target mechano-sensitive ion channels to control the opening and closing of specific mechano-sensitive ion channels in specific neurons within a target brain region.

In biology, structure determines function. Human behavior emerges from the harmonization of the functions of different body parts. Brain function can be inferred from brain structure and neural connectivity. For example, human hands, eyes, and lips contribute to language-related behaviors. Memory formation relies on recognizing and recalling recurrent, unchanging patterns within objects and events. Perceptual categorization arises from the identification of common repeating patterns in sensory inputs.

When examining the biological mechanisms of animals in a stepwise manner, the process progresses from the neural circuit to neurons, then to ion channels, and ultimately reaches the genetic level.

At the synapse, ion channels exist that bind to neurotransmitters. These ion channels are composed of proteins, which are synthesized by translating genes encoded in the DNA within the nucleus into amino acid sequences.

Brain function arises from neural circuits, which consist of interconnected neurons within the brain. For example, axons of thalamic neurons project to the cerebral cortex, while reciprocally, axons of cortical neurons form synapses with neurons in the thalamic nuclei.

For neuromodulation, the present invention provides an ultrasound pattern that utilizes the resonance frequency(ies) of a target mechanosensitive ion channel to control its opening and closing in a specific neuron within a target brain region. This approach enables selective targeting of specific neuronal cells in the central nervous system (CNS), including the brain and/or spinal cord, and allows for patterned stimulation to induce the formation of a desired neural circuit (loop) associated with a specific brain function.

A neural circuit loop refers to recurring patterns of connectivity and activity within neural circuits. These loops are essential for information processing and transmission, playing critical roles in learning, memory, motor control, and various other brain functions. Neural circuit loops arise from interactions between different brain regions, and they function by transmitting information via synaptic connections and processing electrochemical signals.

According to the present invention, an acoustic wave pattern that utilizes the resonance frequency(ies) of a target mechanosensitive ion channel to control its opening and closing in a specific neuron within a target brain region can induce a precisely controlled spatiotemporal physical stimulation pattern. This stimulation enables the post-synaptic cell to fire action potentials in a desired pattern.

Furthermore, in accordance with the present invention, an acoustic wave pattern utilizing the resonance frequency(ies) of a target mechano-sensitive ion channel to regulate the opening and closing of a specific mechano-sensitive ion channel within a specific neuron in a target brain region can mechanically stimulate specific neurons in a particular brain region. This mechanical stimulation enables the modulation of neural circuits that transmit chemical signals to other regions of the brain. Additionally, the modulation of these neural circuits can optionally trigger or regulate behaviors associated with the targeted neural circuit.

The nervous system is an organ system responsible for transmitting stimuli and coordinating responses to various environmental changes.

The nervous system consists of the central nervous system (CNS), which includes the brain and spinal cord and plays a critical role in sensing overall metabolic status and regulating metabolism by releasing endocrine neuro-signals in response to sensory inputs, and the peripheral nervous system (PNS), which branches out from the CNS and extends throughout the body, transmits these released neural signals to target organs.

The peripheral nervous system (PNS) is further divided into the autonomic nervous system (ANS), which controls involuntary functions of smooth muscle and glands, and the somatic nervous system (SNS), which governs voluntary muscular movements within the body.

The autonomic nervous system (ANS) innervates the heart, blood vessels, glands, and digestive organs, autonomously regulating internal bodily functions independently of conscious control. It plays a critical role in maintaining homeostasis in response to environmental changes. The ANS is further subdivided into the sympathetic nervous system (SNS) and the parasympathetic nervous system (PNS).

The central nervous system (CNS) consists of neuronal clusters in the brain and spinal cord, which are protected by the skull and vertebral column. The peripheral nervous system (PNS) comprises neuronal clusters distributed outside the brain and spinal cord, including those in the skin, muscles, and visceral organs.

In the central nervous system (CNS), which consists of the brain and spinal cord, a neural nucleus refers to a cluster of thousands to tens of millions of neuronal cell bodies densely packed within a structure ranging in size from a few millimeters to several centimeters.

In the peripheral nervous system, which consists of the 12 cranial nerves and spinal cord nerves, a collection of neurons is called a ganglia. As many as 100,000 neurons can be packed into a volume of 1 mm$^3$, and a single neuronal axon can extend tens of centimeters. The neuronal cell body (soma) is roughly 20 micrometers in size, but axons that are 1,000 times longer than the soma branch off from the cell body

Accordingly, for neuromodulation, an acoustic wave pattern utilizing the resonance frequency(ies) of a target mechano-sensitive ion channel to regulate the opening and closing of a specific mechano-sensitive ion channel within a specific neuron in a target body region can be designed to provide stimulation in a precisely controlled spatiotemporal resolution with a predefined sequence.

Furthermore, according to the present invention, an acoustic wave pattern utilizing the resonance frequency(ies) of a target mechano-sensitive ion channel to regulate the opening and closing of a specific mechano-sensitive ion channel within a specific neuron in a target body region can induce the formation of neural circuits through repeated physical stimulation over a prolonged period of at least two days and can be used to train animal behavior.

Non-limiting examples of generated or modulated neural circuits include memory circuits, emotional circuits, and reward circuits associated with dopamine secretion.

The behaviors regulated through the generated or controlled neural circuits include feeding behavior, social interactions, addiction, habitual behavior, and cognitive behavior.

Accordingly, in the present invention, an acoustic wave pattern utilizing the resonance frequency(ies) of a target mechano-sensitive ion channel to regulate the opening and closing of a specific mechano-sensitive ion channel within a specific cell in a target body region can induce or modulate neural circuit formation to initiate or regulate movement or behavior (including sequences of movements or organized motor actions). Furthermore, it can be applied to prevent or treat various disorders, including cognitive impairments and metabolic diseases.

The human body contains numerous peripheral nerves, among which the vagus nerve plays a predominant role in autonomic nervous system regulation and metabolic control. The vagus nerve exhibits distinct connectivity with various organs, and its regulation of organ function is mediated through spike-patterned action potentials. Accordingly, in the present invention, an acoustic wave pattern utilizing the resonance frequency(ies) of a target mechano-sensitive ion channel to regulate the opening and closing of a specific mechano-sensitive ion channel within a specific neuron in a target body region can modulate the action potential patterns of peripheral nerves and thereby regulate the release of neurotransmitters. This process, in turn, activates intracellular signaling molecules, influencing the activity of interconnected or neighboring cells.

**[Brain Function, Target Brain Regions, Cerebrospinal Fluid, and Acoustic wave pattern for Controlling the Gating of Target Mechanosensitive Ion Channels]**

The present invention aims to provide a brain function control device that enables the non-invasive activation or inhibition of target neural circuits or neural networks using acoustic wave with high tissue permeability.

The brain function control device of the present invention is characterized by generating an acoustic wave pattern that utilizes the resonance frequency(ies) of a target mechano-sensitive ion channel to regulate the opening and closing of a specific mechano-sensitive ion channel within a specific neuron in a target brain region.

The properties of acoustic wave such as ultrasound (US), including its ability to penetrate deep into tissues while achieving high spatiotemporal precision, make it an attractive modality for neuromodulation. Furthermore, the fact that US propagates in a unidirectional manner for neuromodulation and is typically used at lower frequencies suggests that it can be applied transcranially, including in humans.

The functioning of the brain arises from the interconnections among the spinal cord, brainstem, cerebellum, hypothalamus, thalamus, hippocampus, amygdala, cingulate cortex, and cerebral cortex. The hippocampus is involved in temporary memory storage, while the cerebral cortex is responsible for long-term memory storage. The thalamus functions as a sensory relay center, plays a role in neural oscillation synchronization, and generates sleep spindles. The brainstem acts as a switch regulating sleep-wake states. The cerebral cortex is highly interconnected via long-range neural fibers, and its primary function is sensory input processing, which is fundamental to perception formation.

At the center of the cerebrum lies the ventricular system, which is formed during development as cells in the ventricular zone proliferate to shape the central nervous system. The brain is enveloped by the pia mater, and the central nervous system is further protected by the pia mater, arachnoid mater, and dura mater, with cerebrospinal fluid (CSF) circulating in the subarachnoid space.

Although the human brain weighs approximately 1,400 g, it is suspended in cerebrospinal fluid, which provides buoyancy, thereby reducing its effective weight and serving as a shock absorber.

The choroid plexus, a network of capillaries intertwined within the ventricles, produces approximately 500 mL of cerebrospinal fluid per day. CSF flows sequentially through the lateral ventricles, third ventricle, cerebral aqueduct, and fourth ventricle.

The brain is immersed in cerebrospinal fluid (CSF), effectively functioning as a dynamic cellular incubator composed of neurons and glial cells. Most of the metabolic processes occurring in the brain are governed by complex biochemical reactions. Additionally, the brain undergoes neuronal division, proliferation, migration, and axonal growth cone formation, while also exhibiting cortical map reorganization and neurogenesis, both of which are associated with neuroplasticity. Furthermore, the brain releases neurotrophic factors that enhance neuronal and synaptic development, and enriched environments promote axonal and dendritic sprouting.

Cerebrospinal fluid (CSF) plays a crucial role in nutrient delivery and waste removal within the brain. The choroid plexus, a structure composed of capillaries and connective tissue, is responsible for secreting CSF and recycling metabolic waste. The composition of CSF includes proteins, glucose, chloride ions, and lymphocytes, with a total volume of approximately 130 milliliters, which is completely replenished every five hours. The central canal of the spinal cord extends upward into the brain, connecting sequentially to the cerebral aqueduct, third ventricle, fourth ventricle, and lateral ventricles. From an energy consumption perspective, the brain receives approximately 20% of the body's total blood supply. Oxygen and glucose are delivered to neurons via the carotid artery, vertebral arteries, and an intricate capillary network. Additionally, the brain is encased and protected by the skull and the meninges, which consist of the dura mater (outermost layer), arachnoid membrane (middle layer), and pia mater (innermost layer). The CSF flows between the arachnoid and pia mater, providing essential nutrients and energy, while also transporting metabolic waste into the bloodstream. The CSF also acts as a protective cushion, ensuring that the brain remains safeguarded against mechanical shocks. Although the adult brain weighs approximately 1,400 grams, the buoyancy provided by the CSF reduces its effective weight to about 50 grams.

The brain can be regarded as a cellular incubator because the central nervous system (CNS) is immersed in cerebrospinal fluid (CSF) and requires a continuous supply of oxygen and glucose for survival. The brain derives its energy from CSF, which contains proteins and glucose. CSF is constantly produced in the choroid plexus and circulates within the ventricular system, driven by arterial pulsations.

Many of the cells that constitute the brain maintain a certain degree of spatial separation, and there are substantial white spaces between them. The regions between cell bodies are densely filled with neural processes.

Accordingly, the present invention allows for the compartmentalization of the target brain region to which the acoustic wave pattern (conditions) is applied, taking into account the circulation process of cerebrospinal fluid (CSF), brain function, and/or brain structure.

Furthermore, because ultrasound (US) can readily penetrate the brain-and, in some cases, the skull-while interacting with tissues at a fundamental resolution of approximately 100 millimeters spatially and 1 millisecond temporally, the acoustic wave patterns of the present invention can reliably activate individual neurons, particularly those located in deep brain regions.

Accordingly, the acoustic wave patterns of the present invention can utilize the interference effects of ultrasound to induce the resonance phenomenon of a target mechano-sensitive ion channel, while generating spatiotemporally controlled sequences of weak-intensity one-dimensional (1D), two-dimensional (2D), or three-dimensional (3D) ultrasound patterns in a manner that does not alter the activity of non-target cells, depending on the environmental conditions of the target brain region.

Furthermore, to precisely modulate the activity of neurons in a desired area or distribution within a 2D or 3D space using the interference effects of ultrasound, the present invention employs an "ultrasound patterner" affixed to the surface of the ultrasound generator. This primary patterning of ultrasound waves can be further refined using multiple ultrasound generators to spatially pattern more complex and highly specific forms. Such "patteners" can be optimized using deep learning algorithms. By leveraging these interference effects, the present invention enhances the spatial resolution of neuronal activity modulation, thereby enabling the precise targeting of interactions within complex, compartmentalized neural circuits and networks in the brain.

In accordance with the present invention, acoustic wave patterns utilizing the resonance frequency(ies) of target mechano-sensitive ion channels should preferably be designed to exert a physical force that does not alter the activity of non-target proteins or non-target cells. This allows for the spatiotemporal modulation of specific neurons without requiring invasive implants within the brain, while minimizing unintended effects on surrounding cells, thereby preventing adverse side effects.

The acoustic wave pattern utilizing the resonance frequency(ies) of target mechano-sensitive ion channels to regulate the opening and closing of a specific mechano-sensitive ion channel in a specific neuron within a target brain region is designed to generate a precisely controlled spatiotemporal force that is sufficient to modulate the activity of the target mechano-sensitive ion channel. However, to minimize unintended effects on surrounding cells and prevent adverse side effects, the acoustic wave pattern should be calibrated to deliver a force that does not significantly alter the activity of non-target cells. This modulation can also be optimized using an ultrasound patterner and deep learning algorithms. Specifically, the interference effects of ultrasound can be leveraged to control the activation of neurons in desired regions and distributions within two-dimensional (2D) or three-dimensional (3D) spaces.

The acoustic wave pattern utilizing the resonance frequency(ies) of target mechano-sensitive ion channels to regulate the opening and closing of a specific mechano-sensitive ion channel in a specific neuron within a target brain region can operate without the use of high-intensity focused ultrasound (FUS), which is associated with thermal side effects.

Furthermore, non-targeted adverse effects, such as lack of neuronal specificity, unintended thermal effects, potential auditory side effects, and/or pain-related side effects, can be prevented or mitigated by leveraging the high spatiotemporal resolution of the acoustic wave pattern based on the resonance frequency(ies) of the target mechano-sensitive ion channels in accordance with the present invention.

Biological tissues, including the brain, exhibit mechanical properties similar to those of water. According to the present invention, the acoustic wave pattern utilizing the resonance frequency(ies) of target mechano-sensitive ion channels to regulate the opening and closing of a specific mechano-sensitive ion channel in a specific cell within a target body region can be applied as a complex one-dimensional (1D), two-dimensional (2D), or three-dimensional physical pattern corresponding to the specific cells and intracellular structural arrangements within the desired region. By exerting precisely controlled physical forces, the acoustic wave pattern can modulate the opening and closing of target mechano-sensitive ion channels expressed on the biological lipid membrane within the target region, enabling highly refined control over their activation or inhibition.

According to the present invention, an acoustic wave pattern optimized for the resonance frequency(ies) of a specific mechano-sensitive ion channel within a specific cell in a target body region can be used to achieve precise spatiotemporal control over the opening and closing of target mechano-sensitive ion channels. In particular, the acoustic wave pattern can be designed to target specific neural circuits or neural networks by utilizing the resonance frequency(ies) of a target mechano-sensitive ion channel, thereby applying physical forces that effectively regulate the opening and closing of the target ion channel in specific neurons (Example 1).

Furthermore, when low-frequency ultrasound is used, it can penetrate the skull. Accordingly, for example, an acoustic wave pattern utilizing the resonance frequency(ies) of a target mechano-sensitive ion channel to control the opening and closing of specific mechano-sensitive ion channels within specific cells in a target body region can be designed to selectively target specific types of brain cells located in deep brain regions.

The key distinction between the CNS and PNS lies in the type of glial cells responsible for electrical insulation of axons. In the CNS, oligodendrocytes provide myelination, with a single oligodendrocyte capable of wrapping around multiple neurons. In contrast, in the PNS, Schwann cells myelinate axons, but each Schwann cell only insulates a single neuron. Consequently, oligodendrocyte dysfunction in the CNS can impair multiple neurons simultaneously, whereas damage to a single Schwann cell in the PNS only affects one neuron, allowing axonal regeneration and remyelination by newly formed Schwann cells.

A group of nerve cells in the central nervous system is called a nucleus, and a nerve cell cluster or a group of nerve cell bodies in the peripheral nervous system is called a ganglion. A ganglion is the site of synapse formation.

In vertebrates, nerves, blood vessels, and muscles are segmentally organized along spinal cord segments, forming metameric structures. Spinal nerves extend into muscle tissues, while skeletal muscles attach to structural components such as the limbs, vertebrae, and spinal column. The nerves that control the forelimbs and hindlimbs extend from the spinal cord, and at the caudal end of the spinal cord, nerve fibers densely converge in a bundle resembling a "horse's tail"-known as the cauda equina.

With recent advancements in nuclear medicine, imaging technologies such as positron emission tomography (PET), functional magnetic resonance imaging (fMRI), and transcranial magnetic stimulation (TMS) have been developed to visualize and measure human and neural metabolism. These tools enable precise and convenient assessments of neural activity. For example, by identifying damaged neural circuits or specific brain regions, targeting them, and applying the present invention, it is possible to design an acoustic wave pattern (condition) that controls the opening and closing of specific mechano-sensitive ion channels in specific neurons.

According to the present invention, the target neural circuit or neural network that is activated or inhibited through an acoustic wave pattern (condition) that controls the opening and closing of specific mechano-sensitive ion channels in specific neurons within a target brain region may involve connections between neurons, or connections between neurons and sensory cells or muscle cells. For example, the final effect may manifest in excitable tissues such as the central nervous system (CNS), autonomic ganglia, or neuromuscular junctions. Specifically, the motor signal output may be directed toward skeletal muscles, or through the autonomic nervous system, it may regulate visceral muscles, cardiac muscles, or secretory glands.

The present invention enables modulation of neural circuit changes in response to external stimuli, internal stimuli, or administered drugs through an acoustic wave pattern (condition) that spatiotemporally controls the opening and closing of specific mechano-sensitive ion channels in specific neurons within a target brain region. Accordingly, the brain function control device of the present invention can induce neuroplasticity or synaptic plasticity by applying an acoustic wave pattern (condition) that spatiotemporally controls the opening and closing of specific mechano-sensitive ion channels in specific neurons within a target brain region. This capability may provide a rehabilitation approach for addressing the functional outcomes of brain injuries.

For example, the brain function control device of the present invention, through the spatiotemporal control of an acoustic wave pattern (condition) that modulates the opening and closing of specific mechano-sensitive ion channels in specific neurons within a target brain region, can facilitate the reconnection of cortical and subcortical neural circuits that have been trained or reorganized.

In a normal brain, changes in the nervous system related to learning are generally associated with modifications in neural network connectivity within specific regions involved in performing a given task. Moreover, neural networks and regions associated with specific functions are consistently found in the same anatomical locations across most human brains. For example, the brain regions responsible for language function are typically located in the temporal lobe of the left hemisphere, while motor areas, sensory areas, and primary visual areas are found in nearly identical locations across individuals. However, each brain region exhibits plasticity, wherein its size, boundaries, or functional role can be reassigned to adjacent areas or reorganized through functional neural network connections with other regions in response to environmental interactions and external stimuli.

For example, the brain function control device of the present invention, through an acoustic wave pattern (condition) that spatiotemporally controls the opening and closing of a specific mechano-sensitive ion channel in a specific neuron within a target brain region, can activate or inhibit neural circuits, thereby enabling experience-dependent reorganization of synaptic networks, including those in the cerebral cortex.

Non-limiting examples of target brain regions for the acoustic wave pattern (condition) of the present invention, which controls the opening and closing of a specific mechano-sensitive ion channel in a specific neuron, include the hippocampus, dentate gyrus, neocortex, and cerebellum.

The hippocampus and dentate gyrus are key brain regions associated with memory formation.

The hippocampus, located at the center of the limbic system, plays a crucial role in long-term memory, spatial cognition, and emotionally driven behaviors. Information entering the hippocampus invariably passes through the dentate gyrus, which consists of granule cells and interneurons.

Among brain structures, the dentate gyrus contains a high density of granule cells, which serve as output neurons of the dentate gyrus by forming synaptic connections with the pyramidal neurons in the hippocampus.

Interneurons play an essential role in mediating and regulating excitatory transmission between neurons in the central nervous system (CNS). These interneurons, organized in neural fiber bundles, can be classified into excitatory or inhibitory types, depending on whether they enhance or suppress postsynaptic potentials in their respective target neurons.

The granule cells and interneurons in the dentate gyrus process incoming information by grouping highly correlated memories together.

The mechanism underlying this process operates as follows:

The dendrites of neurons function similarly to antennas, receiving incoming signals. However, interneurons are subject to strong inhibitory regulation due to high concentrations of chloride transporters and $GABA_A$ (gamma-aminobutyric acid type A) receptors, which amplify inhibitory signals.

While interneurons do not directly process information, they determine which granule cells are recruited for encoding specific inputs. In contrast, granule cells contain lower concentrations of $GABA_A$ receptors, making them less susceptible to inhibitory regulation.

Through this coordinated interaction, the interneurons and granule cells of the dentate gyrus encode environmental signals, projecting them onto the dentate gyrus in a map-like manner.

If the inhibitory action mediated by GABA receptors or other related mechanisms is altered, the dentate gyrus may fail to properly function in accessing and encoding input information. This dysfunction can lead to memory impairment or other neurological disorders.

For example, an acoustic wave pattern (condition) that modulates the opening and closing of a specific mechano-sensitive ion channel in a specific neuron can activate cortical regions such as the visual cortex and auditory cortex.

By leveraging the interference effect of ultrasound, the present invention induces the resonance phenomenon of a target mechano-sensitive ion channel while ensuring that the intensity remains weak enough not to alter the activity of non-target cells. By applying one-dimensional (1D), two-dimensional (2D), or three-dimensional (3D) ultrasound patterns in a spatiotemporally controlled sequence, the invention addresses the issue of strong activation of specific brain functional regions (e.g., the auditory cortex) by focused ultrasound (FUS), which may otherwise lead to wider, unintended cortical activation.

Additionally, focused ultrasound (FUS) can induce auditory side effects, cutaneous perception issues, and other potential sensory disturbances, such as tactile or pain sensations. In contrast, by leveraging the interference effect of ultrasound, the present invention induces the resonance phenomenon of a target mechano-sensitive ion channel while ensuring that the intensity remains weak enough not to alter the activity of non-target cells. By designing and applying one-dimensional (1D), two-dimensional (2D), or three-dimensional (3D) ultrasound patterns in a spatiotemporally controlled sequence, the invention can prevent, mitigate, or treat sensory disturbances, including pain-related side effects.

**[Acoustic Wave Generator Providing the Resonance Frequency(ies) of a Mechano-Sensitive Ion Channel]**

A wave transmits energy not through the movement of objects but through the oscillation of a medium. Wave energy is proportional to the square of both amplitude and frequency.

$$\text{Intensity of wave energy} \propto (\text{Amplitude})^2 \times (\text{Frequency})^2$$

A wave in which the oscillation direction of the medium is parallel to the propagation direction of the wave is called a longitudinal wave. Since longitudinal waves create regions of compression (dense areas) and rarefaction (less dense areas) within the medium, they are also referred to as compressional waves.

Ultrasound refers to sound waves with an audible frequency of approximately 20 kHz or higher. Due to its high frequency, greater intensity compared to typical sound waves, and shorter wavelength, ultrasound enables the generation of highly directional sound propagation.

Biological tissues, including the brain suspended in cerebrospinal fluid (CSF), exhibit mechanical properties similar to those of water.

In biological tissues, ultrasound propagates at a velocity of approximately 1,500 m/s, with wavelengths ranging from 15 $\mu$m to 1.5 mm. Ultrasound can penetrate soft tissues to depths of 1-10 cm without significant distortion, and when using low-frequency ultrasound, it can also penetrate the skull. Due to these deep tissue penetration properties, ultrasound is widely utilized for medical imaging diagnostics and is also employed to measure the mechanical properties (acoustic impedance) of biological targets. Moreover, ultrasound enables precise internal structural analysis of objects.

An ultrasound pattern is a form of energy pattern, and in accordance with the present invention, an acoustic wave pattern utilizing the resonance frequency(ies) of a target mechano-sensitive ion channel is designed to control the opening and closing of a specific mechano-sensitive ion channel within a target brain region. This acoustic wave pattern can spatiotemporally and selectively stimulate specific neurons within the target brain region and can be designed to provide conditions that either activate or inhibit specific neuronal populations.

The present invention enables the activation or inhibition of a target neural circuit or neural network in a desired sequence by utilizing an acoustic wave pattern that stimulates specific neurons with precise temporal resolution at a specific time, thereby activating or inactivating the target neurons in a controlled manner.

The acoustic wave pattern optimized for the resonance frequency(ies) of the target mechano-sensitive ion channel is designed to prevent heat generation in the biological target due to acoustic waves. To achieve this, the wave pattern is divided into an operational period and a resting period, where the operational and resting periods may range from 10 milliseconds to 10 seconds.

The ultrasound frequency employed in the present invention may range from 0.5 to 100 MHz, preferably from 0.5 to 20 MHz, and more preferably from 0.5 to 10 MHz.

The acoustic wave pattern optimized for the resonance frequency(ies) of the target mechano-sensitive ion channel may be a one-dimensional (1D), two-dimensional (2D), or three-dimensional (3D) physical wave pattern generated through the interference effect of acoustic waves.

Furthermore, the acoustic wave pattern optimized for the resonance frequency(ies) of the target mechano-sensitive ion channel may be an ultrasound pattern selected from the group consisting of targeting based on beat frequency interference (interference effect) and ultrasound holography.

The acoustic wave pattern optimized for the resonance frequency(ies) of the target mechano-sensitive ion channel may be a pattern with a resolution of less than 100 micrometers, including dot patterns, linear stripe patterns, circular patterns, donut-shaped patterns, or complex patterns corresponding to the arrangement of cells and intracellular structures in the target region.

The beating phenomenon occurs when two waves with similar or slightly different frequencies interfere with each other, generating a new wave. In the case of acoustic waves, the intensity of sound periodically varies, a phenomenon known as beating. The beating phenomenon caused by a frequency difference (fus) results in periodic intensity variations.

As a type of interference effect between two waves, the wave generated through beating progresses at a frequency equal to the average of the two original wave frequencies, and its amplitude varies periodically with a relatively slower cycle.

By leveraging the interference effects of ultrasound, it is possible to modulate the activity of specific cells within a desired region and distribution in two-dimensional (2D) or three-dimensional (3D) space by utilizing the resonance frequency(ies) of target mechano-sensitive ion channels within the acoustic wave pattern (Example 2 and Fig. 3). An "ultrasound patterner" can be attached to the surface of an ultrasound generator to initially pattern the emitted ultrasound. Multiple ultrasound generators can then be utilized to spatially pattern more complex and specific waveforms. These ultrasound patterners can be optimized using deep learning algorithms. By employing this interference effect, the spatial resolution of neuronal activity can be enhanced, allowing for a more precise targeting of compartmentalized, complex neural circuits and neuronal network interactions within the brain. This invention presents a brain stimulation technology that integrates ultrasound with genetic engineering, offering new possibilities in neuron activation studies, brain mapping, neurodegenerative disease treatment, and the broader understanding of brain function.

The brain function control device of the present invention can target a desired brain region with ultrasound (US) using a directionally adjustable transducer array and a 3D-printed acoustic lens.

Ultrasound (US) represents a novel mode of non-invasive brain stimulation, offering highly precise spatial resolution and depth control.

In the present invention, while leveraging the penetration depth advantage of ultrasound, the resolution limitation imposed by the diffraction limit of ultrasound can be addressed through the resonance frequency(ies) of the target mechano-sensitive ion channel and/or sonogenetics.

Sonogenetics technology enables high temporal precision and resolution for cell-type-specific, ultrasound-based modulation of cellular activity across various cells, circuits, and brain structures. Therefore, sonogenetics serves as a neuroscientific research tool for the experimental interrogation of neural circuits, providing insights into their functional connectivity and role in brain function. The brain function control device or the mechano-sensitive ion channel gating control device of the present invention can integrate sonogenetics technology to generate ultrasound wave patterns with high spatiotemporal resolution.

Depending on the type of mechano-sensitive ion channel, the present invention can activate or inhibit a target neural circuit or neural network. Additionally, by enabling targeted delivery of stimulation and on/off control of stimulation, it allows for alternating neuromodulation of neural activity. Thus, by applying a one-dimensional (1D), two-dimensional (2D), or three-dimensional (3D) ultrasound pattern in a spatiotemporally controlled sequence, the present invention can modulate the gating of target mechano-sensitive ion channels in specific neurons within a target brain region, enabling various forms of neuromodulation. As a result, the invention provides a clinical strategy for restoring and enhancing brain function. For example, this technology can serve as a therapeutic intervention for treating neurological and psychiatric disorders, including epilepsy, depression, traumatic brain injury, Parkinson's disease, and obesity.

The mechano-sensitive ion channel gating control device of the present invention can be mounted on a freely moving animal's target body region and is capable of remotely controlling the generation of an acoustic wave pattern that utilizes the resonance frequency(ies) of a target mechano-sensitive ion channel to regulate its opening and closing within a specific cell of the target body region. For example, the brain function control device of the present invention can be mounted on the head of a freely moving animal and is designed to remotely control the generation of an acoustic wave pattern that modulates the gating of a specific mechano-sensitive ion channel in a specific neuron within a target brain region.

The aforementioned acoustic wave pattern generator can be integrated into the brain function control device or the mechano-sensitive ion channel gating control device of the present invention.

**[Neuroplasticity and Synaptic Plasticity]**

Non-invasive neuromodulation techniques serve as valuable tools in both basic neuroscience and clinical applications. Ultrasound (US) can deliver acoustic energy to neurons located deep within the brain, inducing mechanical effects without thermal accumulation, depending on pulsing parameters such as frequency, pressure, pulse duration, and duty cycle.

According to the present invention, the spatiotemporal mechanical force generated by the acoustic wave pattern that utilizes the resonance frequency(ies) of a target mechano-sensitive ion channel to control the opening and closing of a specific mechano-sensitive ion channel in a target neuron within a target brain region can provide multiple modes of physical interaction depending on the composition of biological tissue and ultrasound (US) pulse parameters. By offering various mechanisms for neuromodulation, such as the direct activation or inhibition of neurons without inducing significant deformation, the invention enables the non-invasive activation or inhibition of target neural circuits or neural networks in freely moving animals.

According to the present invention, the acoustic wave pattern that utilizes the resonance frequency(ies) of a target mechano-sensitive ion channel to control the opening and closing of a specific mechano-sensitive ion channel in a target neuron within a target brain region can be precisely targeted with high spatial accuracy or focused at specific depths. Moreover, by non-invasively activating or inhibiting target neural circuits or neural networks, the invention enables the modulation of brain function.

It has been revealed that the human brain consists of hundreds of billions of neurons. Neurons establish electrical circuits by receiving and transmitting information in the form of electrical pulses, but communication between neurons relies on chemical neurotransmitters. The mechanism of neuronal response indicates that most neurons exhibit electrical excitability, and this property is known to be regulated by the activity of various types of ion channels present in their cell membrane or biological membrane.

An electrical signal transmitted through the dendrites of a neuron is integrated at the axon hillock after passing through the cell soma, where the decision to generate an action potential in the axon is made. When the electrical signal propagates to the nerve terminal, it induces the release of neurotransmitters, a process in which voltage-gated ion channels-which are regulated by changes in membrane potential-play a crucial role. However, the activity of these ion channels is not solely regulated by membrane depolarization-induced voltage differences, but can also be simultaneously modulated by neurotransmitters or hormone receptors present in the cell membrane.

The cell membrane is a fluid, dynamic structure, allowing continuous morphological changes. Notably, the plasma membrane of neurons extends into numerous projections and branches.

Each neuron forms approximately 10,000 synapses. The number of synapses can increase in response to sensory stimulation and decrease during sleep. The neuronal protrusions that form synapses are referred to as spines. A single neuron can extend 10,000 spines, which interact with adjacent neurons through voltage pulses and neurotransmitter signaling, thereby facilitating neural communication. Neurons are specialized cells capable of converting electrochemical signals into neurotransmitter-mediated action potentials. Neurons generate voltage pulses and transmit them to other neurons, forming neural circuits.

When neurons receive stimulation and establish functional connections with other cells, they receive an adequate supply of oxygen and glucose, which is essential for their survival. If neurons fail to form such connections, they cannot sustain life. Neural circuits emerge from neuronal interconnections, and fundamental brain functions such as memory and language arise from the operation of these neural networks.

According to the present invention, a specific neuron within a target brain region that is stimulated by an acoustic wave pattern utilizing the resonance frequency(ies) of a target mechano-sensitive ion channel can generate action potentials by controlling the opening and closing of the target mechano-sensitive ion channel. Consequently, this stimulation can induce changes in neural networks and synapses within the brain's nervous system.

Therefore, the present invention enables the construction, strengthening, expansion, or weakening of neural networks, neural circuits, or their interconnections by utilizing an acoustic wave pattern that spatiotemporally regulates the opening and closing of a specific mechano-sensitive ion channel in a specific neuron within a target brain region.

The brain integrates sensory information. In this regard, there are primary sensory areas, association cortices, and multimodal association cortices.

The brain creates sensations, perceptions, and thoughts.

The brain forms memories. This process involves the hippocampus, limbic system, and multimodal sensory association areas.

The brain generates intentional movements. This process is associated with prediction, selection, and the prefrontal cortex.

The brain facilitates language. This involves tools, symbols, and the virtual world.

The brain function control device of the present invention can be designed to modulate various brain functions by selectively activating or inhibiting a target neural circuit or neural network. This is achieved by controlling the opening and closing of specific mechano-sensitive ion channels in specific neurons within a target brain region using an acoustic wave pattern that is tailored for different brain functions and/or customized for individual users.

For example, the brain function control device of the present invention can be designed to stimulate only specific neural circuits during particular behavioral or cognitive states by optimizing the acoustic wave pattern accordingly.

An example of brain function involves language and intentional behavior, which are mediated by the association cortex. The adaptation process in vertebrates corresponds to the evolutionary development of the central nervous system. The expansion of the neocortex has enabled the transition from passive movements to active behaviors. **In** humans, the evolution of the association cortex has facilitated the emergence of language and intentional behavior.

The neural axis (neural network infrastructure) is embedded in nearly all somatic cell groups that constitute the human body. As a result, sensory signals are continuously received from sensory cells distributed throughout the body and are processed to generate motor output signals directed to skeletal muscles and secretory glands. Additionally, the autonomic nervous system (ANS) controls visceral muscles, cardiac muscles, and glands.

The human nervous system has evolved to feel emotions, store memories, learn, plan, and develop self-awareness. Furthermore, humans have developed the ability to store and retrieve abstract symbols, such as language, enabling rapid cultural evolution. These sensory, perceptual, and consciousness-related neural processing functions are primarily performed within the cerebral cortex.

Accordingly, the target neural network of the present invention may process sensory perception and/or consciousness-related neural processing via the cerebral cortex.

Additionally, the target neural network of the present invention may facilitate active behavior output through the cerebral cortex, transmit motor signals to skeletal muscles, or regulate motor signals directed to visceral muscles, cardiac muscles, or secretory glands via autonomic nervous system activity.

The brain function control device of the present invention is capable of modulating neuroplasticity and/or synaptic plasticity by generating an acoustic wave pattern that utilizes the resonance frequency(ies) of a target mechano-sensitive ion channel to control the opening and closing of a specific mechano-sensitive ion channel within a specific neuron in a target brain region.

Additionally, the present invention facilitates the regulation of neuroplasticity and/or synaptic plasticity by artificially expressing or genetically engineering specific mechano-sensitive ion channels within the target neuron of the target brain region to modulate their opening and closing.

The brain neural network is known to be the most complex network discovered to date. In addition to efforts to apply such highly sophisticated networks to computing systems, the medical field has been striving to understand the principles of neural network formation and apply them to therapeutic interventions.

Recently, researchers have successfully mapped inhibitory neuronal circuitry and identified distinct circuit formation principles. This breakthrough has enabled the monitoring of structural changes in neural networks over time and the ability to capture moments when an organism grows and adapts to its environment.

The activation or inhibition of target neural circuits or neural networks can occur through changes in neuronal connectivity length, the addition or removal of connections, and the formation of new neurons. Therefore, the brain function control device of the present invention can facilitate the realization of simulated experiences or learning processes intended to be imprinted into brain function.

The concept of plasticity can be applied not only at the molecular level but also to environmental events. This phenomenon is complex and involves multiple levels of organization. Virtually all changes in brain activation can be categorized under plasticity. For example, short-term visual adaptation to motion or contours, the maturation and pruning of cortical maps, recovery after a stroke, and the changes that typically occur when an adult learns something new are widely explained using the concept of axonal guidance during development. More recently, plasticity has frequently been described as an inherent property of the central nervous system, particularly in relation to specific forms of reorganization that involve axonal growth, long-term potentiation, or genetically mediated responses.

One of the fundamental mechanisms of neuroplasticity is related to synaptic pruning. Individual connections within the brain are continuously eliminated and reformed depending on how they are used. Neurons that fire together tend to wire together, while neurons that fire separately tend to remain unconnected. If two neurons frequently generate stimuli simultaneously and are located near each other, their cortical maps may merge into a single representation. Conversely, if neurons generate asynchronous and irregular stimuli, they will form distinct and separate cortical maps.

**[Pharmacological Mechanism of Acoustic Wave Patterns Utilizing the Resonance Frequency(ies) of Target Mechano-Sensitive Ion Channels]**

The present invention enables the modulation of neural circuit changes in response to external stimuli, internal stimuli, or administered drugs by spatiotemporally controlling the opening and closing of specific mechano-sensitive ion channels in specific neurons within a target brain region through acoustic wave patterns.

Non-limiting examples of external stimuli include reward, environmental changes, experiences, individual activities, practice, learning, and training.

Internal stimuli encompass inflammation, infection, tumors, aging, and other pathological conditions, as well as positive stimuli such as food intake, energy expenditure, and pain relief.

Administered drugs may include those acting on the nervous system but are not limited thereto. Preferably, the drugs may be those intended for the prevention or treatment of neurological disorders.

Neurotransmitters are critical factors that determine the type of neurons that secrete them and their physiological functions within the nervous system.

Drugs that mimic or enhance the effects of neurotransmitters at synapses are referred to as agonists. Conversely, drugs that interfere with neurotransmitter action or inhibit signal transmission are called antagonists. Most drugs that act on the nervous system exert their effects by functioning as either agonists or antagonists, thereby influencing mental states or neurological functions.

According to the present invention, an acoustic wave pattern utilizing the resonance frequency(ies) of a target mechano-sensitive ion channel to regulate the opening and closing of a specific mechano-sensitive ion channel in a specific neuron within a target brain region can function analogously to a neurotransmitter agonist by facilitating or enhancing the role of neurotransmitters at synapses. Conversely, it can also act similarly to a neurotransmitter antagonist by disrupting neurotransmitter activity or inhibiting signal transmission.

The spatiotemporally controlled acoustic wave pattern of the present invention, designed to regulate the opening and closing of a specific mechano-sensitive ion channel in a specific neuron within a target brain region, serves as an electroceutical providing mechanical stimulation patterns. By adjusting dose and/or administration protocols, the acoustic wave pattern can be engineered to mimic the effects of chemical agonists or chemical antagonists of target mechano-sensitive ion channels.

According to the present invention, an acoustic wave pattern utilizing the resonance frequency(ies) of a target mechano-sensitive ion channel to regulate its opening and closing in a specific cell within a target body region can, when the target mechano-sensitive ion channel is expressed in the cell membrane of a specific neuron in a particular brain region, stimulate the specific neuron to induce its activation and/or inhibition. This modulation enables control over the secretion and secretion levels of neurotransmitters (e.g., glutamate, gamma-aminobutyric acid (GABA), dopamine, etc.), which transmit neuronal signals from the stimulated brain region to other regions of the brain.

Brain regions, neural circuits, or their neural networks associated with neurological disorders are as follows.

A drug that mimics or enhances the effect of a neurotransmitter at the synapse is called an agonist. In contrast, a drug that interferes with the action of a neurotransmitter or inhibits signal transmission is called an antagonist. Most drugs that act on the nervous system function as either agonists or antagonists, thereby influencing mental states or neurological functions.

Addiction-related behaviors can be explained by the repeated action of dopamine at synapses along the reward pathway, which extends from the midbrain, passing through the nucleus accumbens, and reaching the prefrontal cortex.

Schizophrenia is associated with dopaminergic hyperactivity within the reward pathway. In particular, this hyperactivation leads to prefrontal cortex dysfunction, which can impair problem-solving, planning, decision-making, and judgment, all of which are essential for situational awareness and cognitive processing.

Decisions regarding movement, made in the prefrontal cortex, are transmitted to the premotor cortex, which then sends specific motor plans to the primary motor cortex. The primary motor cortex issues motor commands to the brainstem and spinal cord, which then execute movements by activating the corresponding muscles of the targeted body parts. Throughout this process, the basal ganglia play a crucial role in modulating movement force by interacting with the cerebral cortex.

Excessive and uncontrolled movements, as seen in Tourette syndrome, are believed to result from abnormalities in the basal ganglia, particularly in the right hemisphere basal ganglia. This condition can be managed with haloperidol, which acts by blocking dopaminergic synapses within the basal ganglia.

The basal ganglia, which are involved in coordinating slow movements by regulating movement force, facilitate the selection of a particular movement by releasing inhibition on that movement. Movement disorders associated with the basal ganglia can be classified into hypokinetic disorders (e.g., Parkinson's disease) and hyperkinetic disorders (e.g., Tourette syndrome). These movement disorders are closely linked to abnormal dopaminergic activity within the basal ganglia.

The basal ganglia, which are involved in coordinating slow movements by regulating movement force, facilitate the selection of a particular movement by releasing inhibition on that movement. Movement disorders associated with the basal ganglia can be classified into hypokinetic disorders (e.g., Parkinson's disease) and hyperkinetic disorders (e.g., Tourette syndrome). These movement disorders are closely linked to abnormal dopaminergic activity within the basal ganglia.

Post-traumatic stress disorder (PTSD) is primarily caused by hyperactivation of the amygdala and structural and functional impairments in the prefrontal cortex and hippocampus, which are involved in situational awareness.

The present invention can induce neuroplasticity through an ultrasound pattern (condition) that spatiotemporally controls the opening and closing of specific mechano-sensitive ion channels in specific neurons within a target brain region. By leveraging this mechanism, the invention can be applied to the treatment of pain, addiction, neurological disorders, brain injuries, or learning disabilities.

The concept of neuroplasticity is central to memory and learning theories, as it relates to experience-driven changes in synaptic structure and function.

The expression of neuroplasticity can be categorized into positive behavioral outcomes and adaptation to negative behavioral outcomes. For instance, if an organism is able to recover to a normal level after a stroke, this adaptability would be considered "positive plasticity." In contrast, excessive neuronal growth leading to seizures or paralysis, or excessive neurotransmitter release in response to an injury resulting in neuronal damage, can be regarded as "negative plasticity." Additionally, drug addiction and obsessive-compulsive disorders are considered forms of negative plasticity due to maladaptive synaptic remodeling.

The sensory system of cortical tissue is often described in terms of maps. For example, sensory information from the foot is transmitted to a specific cortical location, while information from the arm is directed to a different target area. As a result of this somatosensory organization, sensory inputs to the cortex are arranged in a manner analogous to a map.

When a stimulus is cognitively associated with reinforcement, its cortical representation is strengthened and expanded. Studies have shown that when a new sensory-motor behavior is acquired, the cortical representation can increase two- to threefold within one to two days, with the transformation completing within a few weeks. These controlled studies indicate that this change is not merely driven by sensory experience alone, but rather requires learning based on sensory experience. Moreover, the most robust changes occur when the stimulus is linked to reinforcement, such as reward or effective pain relief, within a typical regulatory framework.

According to the present invention, the acoustic wave pattern utilizing the resonance frequency(ies) of target mechano-sensitive ion channels to control the opening and closing of a specific mechano-sensitive ion channel within a target brain region can spatiotemporally regulate dopamine secretion in specific neurons, target neural circuits, or neural networks.

Practicing a particular skill reorganizes the brain within certain limits to maximize the performance of that skill. When an individual focuses attention on something perceived as important, dopamine is released, which acts on cortical regions to enhance the representation of stimuli that were present at the time of dopamine secretion.

In general, such expanded cortical representations are beneficial in that they allow the cortex to allocate more attention to a stimulus or process it more deeply. However, in extreme cases, such reorganization may lead to complications. Considering this, the acoustic wave pattern utilizing the resonance frequency(ies) of target mechano-sensitive ion channels to control the opening and closing of specific mechano-sensitive ion channels in specific neurons within a target brain region-as provided by the present invention-can finely regulate dopamine secretion with spatiotemporal precision.

It has been discovered that severe reorganization of the sensory thalamus and cortex causes tactile responses to one finger to overlap with those of another finger. There exists a strong correlation between the degree of somatosensory remapping and the intensity of phantom limb pain. This serves as a compelling example of new neural connections forming in the adult human brain.

When a specific neural network in the brain sustains partial damage, the motor functions previously controlled by that network are lost, leading to paralysis of the contralateral limbs, partial motor impairment, and sensory abnormalities. In particular, stroke occurs when blood vessels supplying oxygen and glucose to various brain regions become blocked or rupture, resulting in the interruption of blood flow to neurons. This leads to necrosis of neurons in specific brain regions, causing neurological damage within the central nervous system.

Brain damage caused by stroke is one of the leading causes of severe disability, rendering voluntary movement necessary for daily physical activities impossible. Along with the intrinsic neural reorganization that occurs following brain injury, the functional capacity of specific motor cortical areas can be modulated by individualized motor experiences. This phenomenon of neuroplasticity provides critical insights for designing various forms of motor rehabilitation training for stroke patients.

Despite the fact that damaged brain cells do not regenerate, the motor function of paralyzed or weakened limbs can recover partially or even significantly after the recovery phase. This phenomenon is referred to as motor function recovery through neuroplasticity. In the cortical neurons of mature primates, extensive functional and structural plasticity enables adaptive changes within the nervous system.

Regarding the treatment of learning disabilities, a computer program called "Fast ForWord" has been developed based on neuroplasticity. This program provides seven brain exercises designed to help individuals with dyslexia, language deficits, and learning difficulties. Recent studies have conducted empirical training on adults to determine whether negative plasticity, such as age-related cognitive decline (ARCD), can be mitigated. The training included exercises specifically designed to counteract cognitive, memory, and motor control impairments caused by ARCD. After 8 to 10 weeks of using the program, participants exhibited significant improvements in task processing. These findings suggest that neuroplasticity-based programs can enhance cognitive function and memory in adults experiencing ARCD.

In the brain, neurons can die but do not regenerate. However, there is evidence supporting experience-dependent reorganization of synaptic networks, including those in the cerebral cortex.

Accordingly, the brain function control device of the present invention can be mounted on the head of a freely moving animal and allows for remote control of the acoustic wave pattern generation that modulates the opening and closing of target mechano-sensitive ion channels in specific neurons within a target brain region. This capability enables the device to be applied to various forms of motor rehabilitation training or learning disability treatment programs. Additionally, in certain cases, it may facilitate neural reorganization by controlling neuroplasticity and/or synaptic plasticity modulation, potentially in conjunction with pain relief.

**[Target Mechano-Sensitive Ion Channels on Biological Lipid Membranes as Drug Targets for Electroceuticals Providing Mechanical Stimulation Patterns]**

According to the present invention, the acoustic wave pattern that utilizes the resonance frequency(ies) of specific mechano-sensitive ion channels in specific cells within a target body region to control the opening and closing of target mechano-sensitive ion channels functions as a type of electroceutical that provides a mechanical stimulation pattern. The drug target protein of specific cells in the desired region that can be remotely and wirelessly controlled is a mechanosensitive ion channel. For example, in accordance with the present invention, the acoustic wave pattern that utilizes the resonance frequency(ies) of specific mechano-sensitive ion channels in specific cells within a target body region to control the opening and closing of the target mechano-sensitive ion channel can alter the electrical polarization of the cell membrane (Fig. 5).

An ion channel is a transmembrane transport protein that mediates the passage of specific ions across the biological membrane. These channels are regulated by various intracellular and extracellular stimuli, and when open, they facilitate the passive movement of specific ions according to the electrochemical gradient or actively transport specific ions using energy.

According to the present invention, an acoustic wave pattern utilizing the resonance frequency(ies) of a specific mechano-sensitive ion channel within a target body region induces spatiotemporally diverse mechanical stimulation patterns and resonance phenomena. As a result, the target mechano-sensitive ion channel undergoes structural changes, allowing for precise spatiotemporal control of its opening and closing. Therefore, drug target proteins that undergo structural changes in response to an acoustic wave pattern utilizing resonance frequency(ies) to induce resonance phenomena also fall within the scope of the present invention.

The cell membrane is a fluid, dynamic structure, allowing continuous morphological changes.

A biological lipid membrane is typically a phospholipid bilayer, forming the membrane of a living cell. The hydrophilic head groups of the phospholipids are oriented toward the outer and inner surfaces of the membrane, while the hydrophobic tails are directed inward. This bilayer structure contains various functional proteins, including multiple types of enzymes, as well as carbohydrates and lipids, which are arranged within the membrane's inner, outer, or middle regions according to their physicochemical properties, making it a highly complex composite structure.

The plasma membrane of eukaryotic cells is a lipid bilayer into which proteins or glycoproteins are embedded, serving to separate the intracellular and extracellular environments. The movement of substances across the membrane occurs selectively through the lipid bilayer. Due to the hydrophobic nature of the cell membrane, hydrophilic substances can only enter the cell through specific mechanisms. In the case of small ions, their transport across the membrane is regulated by the opening and closing of ion channels. The ionic composition inside and outside the cell differs across the plasma membrane. This difference in ion distribution, combined with the selective permeability of the membrane, creates a membrane potential, which typically ranges between -50 to -100 mV, with the cell interior being negatively charged relative to the exterior. This electrical potential is referred to as the resting membrane potential or transmembrane potential, and maintaining this electrochemical balance is essential for cell survival.

Many cells regulate their physiological functions through various mechanisms, including modulating the permeability of ions across the cell membrane, releasing neurotransmitters at the presynaptic nerve fiber terminal,

utilizing intracellular Ca$^{2+}$ for muscle contraction, and secreting catecholamines from the adrenal medulla.

Excitable cells, such as neurons and muscles, exhibit the property of altering their membrane's electrical characteristics when appropriately stimulated. Specifically, cation influx into the negatively charged intracellular environment generates an excitation wave known as the action potential. The link between membrane voltage changes and physiological responses is mediated by polar molecules embedded in the membrane, which undergo dipole movement and structural rearrangement, thereby regulating the opening and closing of ion channels. This, in turn, modulates ion permeability across the membrane. The current generated during this gating process is referred to as gating current.

Ion channels are extremely narrow, allowing only small ions such as Na$^+$ and K$^+$ to pass through, while preventing the passage of larger particles. Ion channels selectively permit the passage of one or multiple types of ions, which are classified based on the ions they transport, such as Na$^+$, K$^+$, Ca$^{2+}$, or Cl$^-$ channels. These channels primarily regulate the resting membrane potential, the generation of electrical signals, the flow of Ca$^{2+}$ as a second messenger, cell volume regulation, and the overall ion flux in cells such as secretory cells.

A ligand-gated ion channel is activated upon binding to an agonist, leading to the opening of the ion channel and the subsequent transport of ions. This process results in the depolarization or hyperpolarization of the postsynaptic neuron or muscle cell membrane, thereby transmitting extracellular signals into the intracellular environment. According to the present invention, an ultrasound pattern (condition) designed to activate or reversibly inhibit a target mechano-sensitive ion channel at a specific location functions as a type of electroceutical, performing the role of an agonist or antagonist. By providing a mechanical stimulation pattern, it regulates the opening and closing of membrane ion channels, which serve as drug target proteins embedded in the biological lipid membrane.

In voltage-gated ion channels, changes in membrane potential trigger conformational changes in the ion channel protein, leading to the opening of the Na$^+$ ion channel gate and the subsequent influx of sodium ions. This influx causes the membrane potential to become more positive. Na$^+$ ions continue to enter the cell until the local membrane potential reaches a positive value. Once the membrane potential reverses from a negative to a positive value, the Na$^+$ ion channels close, and K$^+$ ion channels open, allowing K$^+$ ions to exit the cell. The outward flow of K$^+$ ions restores the membrane potential to its resting state. Additionally, channel gating can be influenced by intracellular second messengers, proteins, phosphorylation events, and mechanical stimuli.

According to the present invention, an acoustic wave pattern that utilizes the resonance frequency(ies) of a target mechano-sensitive ion channel within a specific cell of a target body region to control the opening and closing of the target mechano-sensitive ion channel functions as a type of electroceutical that provides a mechanical stimulation pattern. Since this acoustic wave pattern can alter the electrical polarization of the cell membrane, it can also regulate the opening and closing of voltage-gated ion channels.

Ion channels are pore-forming proteins that primarily facilitate the generation and transmission of electrical signals in the membranes of cells constituting organs such as the brain, nervous system, cardiovascular system, and contractile muscles.

Extensive research is actively being conducted on the development of drugs targeting various ion channels and the study of diseases caused by ion channel mutations using gene replication, recombination, and expression of ion channel proteins. Moreover, several anesthetics, including volatile anesthetics, are known to induce anesthesia by modulating the function of voltage-gated or ligand-gated ion channels.

Eukaryotic cells, including human cells, possess an intracellular membrane system that compartmentalizes various organelles. The biological membranes constituting this system are composed of lipid bilayers, with proteins either embedded within or attached to the membrane surface.

Eukaryotic cells and mitochondria share interconnected membranes, with mitochondria-ER contact sites (MERCS) serving as dynamic interfaces involved in mitochondrial quality control, lipid and calcium homeostasis, protein homeostasis, and various intracellular signaling pathways. These MERCS act as structural platforms that optimize energy production and utilization in response to cellular environmental changes, thereby regulating cell survival, proliferation, and apoptosis.

Therefore, as used herein, the biological lipid membrane includes not only the cell membrane but also the endoplasmic reticulum (ER) membrane and the mitochondrial membrane, both of which contain ion channels.

Conventional electrical stimulation technologies have limitations due to the crudeness of electrodes, which fail to distinguish between different types of neurons and indiscriminately stimulate all areas in contact with the electrode. Moreover, electrical stimulation alone is insufficient to suppress neuronal activity and block signal transmission.

The present invention demonstrates that mechanical stimulation of drug target proteins (e.g., mechano-sensitive ion channels)-which generate membrane potential differences in response to various mechanical stimulation patterns-can be precisely applied to specific neurons at specific times and locations through sonogenetics technology (Example 1) and magnetogenetics technology (Example 3), as described later.

In Example 1 and Example 3, the target mechano-sensitive ion channel used is PIEZO1, which is a mechanosensitive ion channel protein. PIEZO1 is expressed in the lungs, bladder, and skin, where mechanosensation plays a biologically significant role. PIEZO1 is not expressed in sensory neurons. When deformation of the cell membrane occurs,

extracellular calcium ions (Ca$^{2+}$) can enter the cell through the PIEZO1 channel.

The ligands of PIEZO1 include agonists such as Jedi1/2 and Yoda1 (a small molecule agonist). The antagonists of PIEZO1 include Gadolinium, Streptomycin, Ruthenium Red, GsMTx4, and Dooku1.

Example 3 combines Cre-dependent expression of Piezo1 with cell-type-specific control of neural activity to provide a method for magneto-mechanically stimulating specific populations of excitatory or inhibitory neurons in deep brain regions in vivo. By selectively activating glutamatergic and GABAergic neurons in the lateral hypothalamus (LH), the study successfully demonstrated the ability to either promote or suppress food intake in freely moving animals, thereby confirming the role of LH neural circuits in feeding behavior regulation. Furthermore, long-term and repeated activation of Vglut2 neurons in the LH induced a sustained anti-feeding effect, leading to significant weight loss and metabolic changes in obese mice. Therefore, Example 3 presents a novel non-invasive approach for long-term, feeding-specific neural circuit control for obesity treatment, introducing an electroceutical strategy that delivers spatiotemporally controlled mechanical stimulation patterns in a non-invasive manner.

Therefore, by utilizing (i) an electroceutical device that provides mechanical stimulation patterns through an acoustic wave pattern using the resonance frequency(ies) of target mechano-sensitive ion channels to control the opening and closing of specific mechano-sensitive ion channels in specific cells within a target body region in accordance with the present invention, and (ii) a sonogenetics technique that delivers spatiotemporally controlled mechanical stimulation patterns similar in mechanism of action to the magnetogenetics technique of Example 3, it is possible not only to regulate the gating of target mechano-sensitive ion channels located in the biological lipid membrane of specific cells within a target body region but also to advance various neuroscientific studies. These include selective interrogation of neural cell populations and circuits, tethered optogenetic control of neural activity, investigation of complex behavioral paradigms that are otherwise disrupted by unnatural environments, and studies on neurological disorders requiring long-term stimulation and response monitoring.

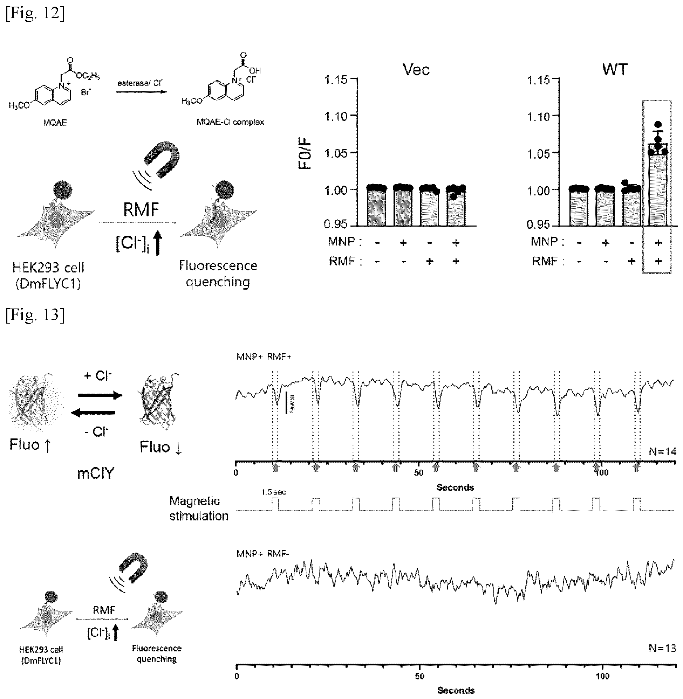

Meanwhile, in Example 4, it was demonstrated that targeting mechanical stimulation to mechano-sensitive anion channels using a mechanism of action similar to magnetogenetics can facilitate the influx of chloride ions (Cl$^-$) into the cell or the efflux of hydrogen ions (H$^+$) out of the cell, thereby inhibiting neuronal activity (Figs. 12 and 13). Accordingly, using a similar mechanism of action as in Example 4, an acoustic wave pattern utilizing the resonance frequency(ies) of target mechano-sensitive ion channels to control their opening and closing in specific cells within a target body region in accordance with the present invention can be applied not only to cation channels such as Piezo1 but also to anion channels as an electroceutical that provides mechanical stimulation patterns. For example, it may be used to activate or inhibit target neural circuits or neural networks. Additionally, by combining the resonance frequency(ies) of two or more target mechano-sensitive ion channels, the acoustic wave pattern can be used either simultaneously or in a specific sequence, enabling the precise spatiotemporal control of one or more target neural circuits or neural networks within one or more target brain regions to either activate or inhibit them.

The neuromodulation induced by utilizing an acoustic wave pattern-which employs the resonance frequency(ies) of target mechano-sensitive ion channels in specific cells within a target body region-as an electroceutical providing mechanical stimulation patterns, according to the present invention, is reversible. Over time or under certain conditions, neuronal activity returns to its baseline state, ensuring that key electrophysiological properties of neurons, such as electrophysiological sensitivity, membrane potential, and intracellular resistance of the cell membrane, remain largely unchanged. Consequently, the neuronal activity can be modulated in a reversible manner.

Ion channel proteins are found in the biological membranes of all living organisms, with approximately 300 distinct ion channel proteins identified in humans.

Although ion pores exist in virtually all cellular membranes, they are predominantly concentrated in excitable tissues, including the central nervous system (CNS), autonomic ganglia, and neuromuscular junctions. Ionotropic receptor proteins typically consist of four to five subunits.

Ion channels constitute a highly attractive drug target protein family, accounting for 15 out of the top 100 best-selling drugs, making them a key focus in drug discovery and development. As a membrane transport protein, ion channels represent an important category of drug targets. Accordingly, in the present invention, the drug target proteins of the electroceutical providing mechanical stimulation patterns may include ion channel proteins and active transporter proteins, which belong to the membrane transport protein family, but are not limited thereto.

In the present invention, the drug target proteins of the electroceutical providing mechanical stimulation patterns include membrane transport proteins that transport cations and membrane transport proteins that transport anions.

Cations include Na$^+$, Ca$^{2+}$, and K$^+$, but are not limited thereto and may also include combinations of various cations.

Anions include Cl$^-$, but are not limited thereto and may also include combinations of various anions.

Non-limiting examples of the membrane transport proteins that serve as drug targets of the electroceutical providing mechanical stimulation patterns in the present invention are as follows:

H$^+$ Transport Membrane Proteins: Hv channel, Proton pumps (H-type, V-type, F-type, and P-type), etc.

Na$^+$ Transport Membrane Proteins: Nav channel, ASIC channel, ENaC channel, etc.

$Ca^{2+}$ Transport Membrane Proteins: Cav channel, IP3 receptor, TPCN channel, etc.

$K^+$ Transport Membrane Proteins: Kv channel, Kca channel, Kna channel, K2p channel, KscA channel, etc.

Nonspecific Cation Transport Membrane Proteins: TRP channel, Piezo channel, MscS, MscM, MscL channel, etc.

$Cl^-$ Transport Membrane Proteins: CLC channel, E-CLC channel, CLIC channel, GABA receptor, etc.

Nonspecific Anion Transport Membrane Proteins: MscS channel, MscL channel, SWELL channel, ANO channel, Maxi anion channel, FLYC channel, etc.


Other Ion Pumps and Related Transporters.

Furthermore, in the present invention, the drug targets of the electroceutical providing mechanical stimulation patterns include naturally occurring or genetically recombinant membrane transport proteins, as well as their mutants and split variants. As an example, the membrane transport protein may be expressed as multiple separate fragments, which subsequently self-assemble intracellularly to form a functional protein. Alternatively, the target may be a split variant utilizing only a portion of the membrane transport protein, or it may be a type of chimeric protein in which split variants of different membrane transport proteins are fused together.

An example of such a mutant variant is a mutant form of DmFLYC1 protein, which may include single and/or multiple mutations at positions R599 (arginine 599), K606 (lysine 606), V656 (valine 656), and T659 (threonine 659). In one embodiment of the present invention, the mutant variants may include single mutants (e.g., R599K, K606R, V656L, and T659L), double mutants (e.g., V656L/T659L), triple mutants (e.g., R599K/V656L/T659L), quadruple mutants (e.g., R599K/K606R/V656L/T659L), or higher-order mutants. In Example 4, the DmFLYC1 mechanosensitive anion channel, which mediates chloride ion transport, is utilized. The ion transport membrane protein used in Example 4 is an anion channel that allows the influx of anions into the cell upon activation.

In accordance with the present invention, the acoustic wave pattern utilizing the resonance frequency(ies) of the target mechano-sensitive ion channel to control the opening and closing of a specific mechano-sensitive ion channel in a particular neuron within a target brain region functions as an electroceutical providing mechanical stimulation patterns. Furthermore, since this acoustic wave pattern (condition) can be extended to control the opening and closing of specific mechano-sensitive ion channels in particular cells within a target region, it is not limited to the brain and can be applied to regulate various physiological functions.

In higher-order animals, ion channels play essential roles in electrical signal generation and transmission, muscle contraction, hormone and neurotransmitter secretion, as well as intracellular and extracellular ion homeostasis. They also directly or indirectly regulate various cellular biological processes, including cell growth, differentiation, and intracellular signaling pathways. The physiological significance of these ion channels is exemplified by various ion channelopathies caused by genetic mutations. Representative examples include long QT syndrome, which results from mutations in cardiac $Na^+$ or $K^+$ channels, and cystic fibrosis, which is caused by mutations in the $Cl^-$ channels of airway epithelial cells.

Therefore, by regulating the activity of specific ion channels, it is possible to control the cellular and physiological phenomena in which these ion channels are involved. In particular, artificially increasing or decreasing their activity can mitigate specific disease conditions and provide medical and pharmaceutical benefits. Notably, ligand-gated ion channels and voltage-gated ion channels have long been recognized as major drug targets, leading to continuous research and development efforts in this area.

Currently marketed blockbuster drugs include the antihypertensive drug amlodipine, which acts as an antagonist of voltage-gated $Ca^{2+}$ channels; the insomnia treatment drug zolpidem, which functions as an agonist of the $GABA_A$ receptor, a central nervous system $Cl^-$ channel; and the antiepileptic drug lamotrigine, which serves as a blocker of voltage-gated $Na^+$ channels.

Despite ion channels being highly promising targets for new drug development, they present several additional challenges compared to intracellular enzymes or receptors.

First, since the function of ion channels involves the movement of ions across the cell membrane, the experimental methods available to measure their activity are highly limited. Traditionally, electrophysiological measurements have been the best and almost the only means of assessing ion channel activity. However, these electrophysiological techniques are labor- and skill-intensive, have an extremely slow turnover, and are not suitable for high-throughput screening through automation.

Second, most ion channels belong to families comprising multiple subtypes, with each channel protein consisting of highly homologous functional modules. For example, voltage-gated $K^+$ channels consist of 12 known members, with approximately 40 associated genes. These proteins share highly similar voltage sensors and $K^+$ permeation pathways. Consequently, identifying and developing drugs that selectively target a specific channel among highly homologous proteins, which are expressed across various tissues and play critical physiological roles, remains a significant challenge.

Third, in higher animals, including humans, various ion channels are specifically expressed in the heart, playing crucial roles in cardiac function. Therefore, if a discovered or developed drug affects cardiac ion channels, it may cause severe cardiotoxicity.

In contrast, according to the present invention, an acoustic wave pattern that utilizes the resonance frequency(ies) of a target mechano-sensitive ion channel to control its opening and closing in specific cells within a target body region can serve as an electroceutical providing mechanical stimulation patterns. By leveraging the interference effect of ultrasound, this acoustic wave pattern induces the resonance phenomenon of the target mechano-sensitive ion channel while generating a precisely controlled spatiotemporal sequence of one-dimensional (1D), two-dimensional (2D), or three-dimensional (3D) ultrasound patterns at an intensity weak enough to avoid altering the activity of non-target cells. Therefore, by identifying the resonance frequency(ies) of the target mechano-sensitive ion channel, the present invention can provide an electroceutical that offers a mechanical stimulation pattern capable of replacing conventional therapeutic agents or addressing the aforementioned challenges.

The physical stimulation sites for alleviating a patient's discomfort symptoms have expanded from spinal nerves to deep brain regions and the vagus nerve, encompassing a broader range of internal neural targets. According to the present invention, an optimized acoustic wave pattern (condition) utilizing the resonance frequency(ies) of a target mechano-sensitive ion channel to activate or reversibly inhibit the target mechano-sensitive ion channel at a desired site can be designed to identify disease-related neural circuits, clarify anatomical regions, and recognize conventional electrical signals or action potentials. Furthermore, the optimized acoustic wave pattern (condition) utilizing the resonance frequency(ies) of the target mechano-sensitive ion channel to activate or reversibly inhibit the target mechano-sensitive ion channel at a desired site can be wirelessly controlled, enabling localized and appropriately intensified physical stimulation to minimize side effects.

According to the present invention, an optimized acoustic wave pattern (condition) utilizing the resonance frequency(ies) of a target mechano-sensitive ion channel to activate or reversibly inhibit the target mechano-sensitive ion channel at a desired site can induce beneficial modifications in biological functions or pathological processes of the body by employing highly controlled and diverse patterns of mechanical stimulation, thereby enabling the treatment of various diseases, including intractable conditions. Furthermore, the optimized acoustic wave pattern (condition) utilizing the resonance frequency(ies) of the target mechano-sensitive ion channel to activate or reversibly inhibit the target mechano-sensitive ion channel at a desired site can selectively treat localized regions of the body by mechanically stimulating target neural or muscle cells in a non-invasive manner. This approach can achieve therapeutic effects comparable to existing pharmaceuticals or provide treatment efficacy for conditions that are difficult to address with conventional drugs, medical procedures, or surgeries.

Furthermore, according to the present invention, an optimized acoustic wave pattern (condition) utilizing the resonance frequency(ies) of a target mechano-sensitive ion channel to activate or reversibly inhibit the target mechano-sensitive ion channel at a desired site can non-invasively modulate neural signals artificially, thereby treating or alleviating metabolic disorders caused by dysfunction in neural circuits.

According to the present invention, an optimized acoustic wave pattern (condition) utilizing the resonance frequency(ies) of a target mechano-sensitive ion channel to activate or reversibly inhibit the target mechano-sensitive ion channel at a desired site can not only evolve into a personalized therapeutic approach by detecting real-time changes in a patient's symptoms and adjusting therapeutic stimulation accordingly, but also facilitate remote monitoring of symptom-related data. The scope of application can extend from neuropsychiatric disorders to obesity, autoimmune diseases such as rheumatoid arthritis, and the treatment of intractable diseases, including dementia, cancer, and Parkinson's disease.

**[Cell-Specific Gene Delivery Techniques and Targeting Strategies]**

In the present invention, the target mechano-sensitive ion channel, whose opening and closing are to be controlled, may be either naturally expressed in specific cells within the target body region or artificially expressed in specific cells within the target body region through sonogenetics technology and/or genetically engineered to possess predetermined resonance frequency(ies).

Non-limiting examples of cells in which the target mechano-sensitive ion channel of the present invention is naturally or artificially expressed include neurons, muscle cells, endocrine cells, cancer cells, epidermal cells, and epithelial cells.

In certain cases, the present invention enables selective signal modulation for specific cell types through cell-specific gene introduction technology. The specific cells may include not only general cells but also neurons. For example, it is possible to control specific brain cells and neural circuits. This provides a novel method for studying the structure and function of neural circuits. Additionally, it allows for the selective targeting of neural networks.

The targeting strategy of expressing the target mechano-sensitive ion channel exclusively in specific regions of the brain or specific cells is the greatest advantage of the sonogenetics technique. The targeting strategy can be established through various methods, including the selection of viral vectors, cell-specific promoters, genetically engineered animals with recombinases, and anatomical localization.

The targeting strategy for mechano-sensitive ion channels offers several advantages. First, inhibition of neuronal activity does not affect surrounding untargeted neurons or upstream neural circuits, thereby providing relatively precise information on the role of the targeted neural circuit. Second, since neuronal firing is not merely a summation of incoming neural information, the results obtained from either inhibition or excitation of neuronal activity can help expand our understanding of how neurons process information.

When combined with an electroceutical that provides non-invasive mechanical stimulation patterns to the brain, magnetogenetics or sonogenetics-based cell-specific gene delivery technology can enable the selective activation and inhibition of various cell types in different brain regions. This approach facilitates the understanding of how specific cell types within neural circuits contribute to brain circuit function.

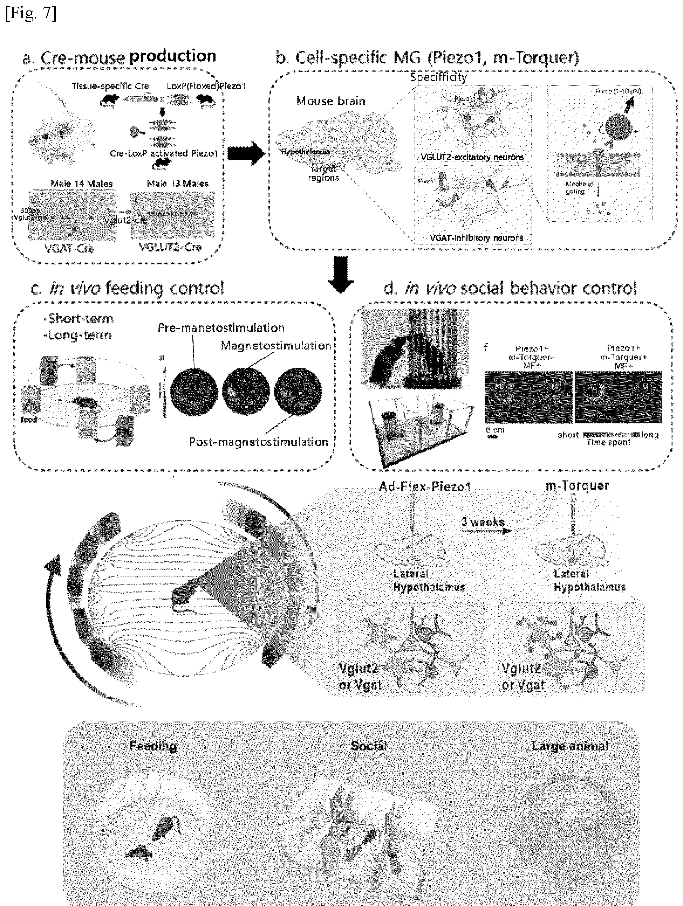

In light of this, Example 3 presents, for the first time, a method to magneto-mechanically stimulate distinct populations of excitatory or inhibitory neurons within deep brain regions in vivo using the m-Torquer conjugate-based drug, by integrating Cre-dependent expression of Piezo1 with cell-type-specific control of neuronal activity (Figs. 6 and 7). Using the m-Torquer conjugate-based drug, glutamatergic and GABAergic neurons in the lateral hypothalamus (LH) were selectively activated, successfully facilitating or suppressing feeding behavior in freely moving animals, thereby validating the role of LH neural circuits in feeding regulation. Furthermore, prolonged and repeated activation of Vglut2 neurons in the LH using the m-Torquer conjugate-based drug resulted in sustained anorexigenic effects, significant weight loss, and metabolic alterations in obese mice. Therefore, an m-Torquer conjugate-based drug that selectively binds to the target mechano-sensitive ion channel protein within specific neurons of the target brain region, when used in combination with an electroceutical providing mechanical stimulation patterns, presents a novel approach and drug modality for the long-term, non-invasive modulation of feeding-specific neural circuits for obesity treatment.

In Example 3, the m-Torquer conjugate-based drug was designed to provide mechanical stimulation patterns exclusively to specific cells within a target body region. Mimicking this drug modality, the present invention is characterized by identifying and utilizing the resonance frequency(ies) of target mechano-sensitive ion channels within specific cells of the target body region, instead of employing an m-Torquer conjugate-based drug that selectively binds to the target mechano-sensitive ion channel proteins in those specific cells.

According to the present invention, an acoustic wave pattern utilizing resonance frequencies within the range of 0.5 to 100 MHz, preferably 0.5 to 10 MHz, which induces the resonance phenomenon of target mechano-sensitive ion channels on a biological lipid membrane, can also be used as an electroceutical providing mechanical stimulation patterns. This was demonstrated in Example 1 (Fig. 2), where sonogenetics technology was employed using Piezo1 protein as the target mechano-sensitive ion channel. The resonance frequencies (2.0 MHz and 6.25 MHz) of the Piezo1 ion channel expressed in HEK293 cells were identified and applied in a manner that ensured the use of weak-intensity acoustic wave patterns, sufficient to induce the resonance of the target ion channel without affecting the activity of non-target cells.

As used herein, a cell-specific gene refers to a gene that includes a promoter enabling spatially regulated expression in a target cell.

Representative examples include the Cre-LoxP and Flp-FRT systems, but are not limited thereto.

Additionally, for temporal regulation, the Cre-LoxP system may be coupled with an inducible system that can be activated at a specific time. The induction mechanism may be physical, chemical, or biological. Representative examples include the tetracycline-inducible system, but are not limited thereto.

According to the present invention, a mechano-sensitive ion channel gating control device and a brain function control device, or the software (SW) program that operates them, utilize an acoustic wave pattern based on the resonance frequency(ies) of the target mechano-sensitive ion channel to regulate the opening and closing of the target ion channel in specific cells within a target body region. These devices and programs can be used in combination with a viral vector designed to introduce the genetic information of the target mechano-sensitive ion channel into the target cells, thereby enabling the expression of the target ion channel.

In this case, for example, the opening and closing of the mechano-sensitive ion channels expressed in the target cells at a desired time can be activated or reversibly inhibited using an acoustic wave pattern based on the resonance frequency(ies) of the specific mechano-sensitive ion channels in the target cells. This can lead to changes in the electrical polarization of the cell membrane and may also induce changes in protein expression levels due to alterations in ion concentrations.

According to the present invention, in order to activate or reversibly inhibit the target mechano-sensitive ion channel at a desired site, an acoustic wave pattern can be applied specifically to the target mechano-sensitive ion channel by expressing a genetically engineered target mechano-sensitive ion channel in the target cell, which has a predetermined resonance frequency.

Gene delivery can be achieved by simply introducing the gene encoding the desired protein for expression. However, to enhance its efficacy, delivery methods such as liposomes and viral vectors can be utilized.

Various vectors, including viral vectors, can be used for the intracellular introduction of recombinant genes. Since each vector has distinct advantages and limitations, the selection of an appropriate vector depends on the objectives of the genetic engineering experiment and the target cell type.

Non-limiting examples of virus types that can be used for targeting strategies include recombinant adeno-associated virus (rAAV), lentivirus (LV), canine adenovirus (CAV), retrograde rAAV (rAAV retro), pseudorabies virus (PRV), herpes simplex virus (HSV), and vesicular stomatitis virus variant (VSV). Among these, AAV and LV are the most commonly used because they are relatively safe and can infect non-dividing cells such as neurons. However, AAV and LV can accommodate only approximately 5 kb and 9 kb of genetic material, respectively, which makes HSV a preferred choice when longer genes need to be introduced. One method for selectively targeting neural circuits using viruses involves injecting CAV expressing a recombinase enzyme into the neuronal cell body, followed by introducing a target mechano-sensitive ion channel gene under recombinase-dependent expression via AAV/LV in the brain region where the neuron extends its axons. Alternatively, rAAV and PRV can be used to introduce genes into neurons retrogradely, allowing gene delivery into synaptically connected neurons under specific conditions. While using CAV recombinase in combination with another virus requires coadministration of two types of viruses, making it experimentally challenging, PRV application is relatively easier. However, PRV's high toxicity necessitates completion of experiments within a few days. A newly developed variant, CAV-N2c (ΔG) RV, has been designed to reduce toxicity while maintaining targeting advantages. Additionally, the HSV-1 H129 strain and VSV variants possess the unique ability to spread in the anterograde direction from the cell body to synaptic terminals, enabling gene expression across synapses.

One of the techniques utilizing various viral combinations is TRIO (Tracing the Relationship between Input and Output). TRIO employs CAV-Cre, an AAV expressing a gene of interest in a recombinase-dependent manner, an AAV expressing the G protein in a recombinase-dependent manner, and RV (ΔG) to map neural circuits. For example, to target only neurons located in region B that receive input from region A and send output to region C, CAV-Cre is injected into region C of a wild-type animal that does not express recombinase, while an AAV expressing the gene of interest in a recombinase-dependent manner, an AAV expressing the G protein in a recombinase-dependent manner, and RV (ΔG) are injected into region B. This enables selective targeting of neurons that are specifically connected to both the input and output regions. However, the TRIO technique alone cannot distinguish neurons that also project to other regions, such as region D, in addition to region C. To overcome this limitation, multiple recombinases can be used. In this modified approach, a transgenic animal expressing Cre recombinase is used, along with CAV-Flp injected into region C, an AAV carrying a gene of interest under Flp-dependent expression, an AAV carrying the G protein gene under Flp-dependent expression, and RV (ΔG). This allows selective targeting of neurons in region B that receive input from region A and project only to region C, but not to region D. This enhanced version of TRIO is referred to as cTRIO (cell type-specific TRIO).

The expression of the recombinant gene encoding the ion transport membrane protein in Example 4 is driven by constitutive promoters, such as the CMV promoter, Ef1-alpha promoter, or LTR. In another embodiment, the promoter may be an inducible or cell-type-specific promoter. A cell-type-specific promoter that enables the expression of the recombinant ion transport membrane protein in specific subpopulations of cells, particularly in neurons, may be preferred. These cells may include neurons within the nervous system, but are not limited thereto. Cell-type-specific promoters are well known in the art. Particularly preferred cell-type-specific promoters include hSyn, vGat, vGlut, and TH, but are not limited to these. Methods for the intracellular delivery of the recombinant genetic sequence may employ conventional techniques. However, it is preferable to introduce the gene into the cell using adeno-associated virus (AAV) or lentivirus.

Regarding cell-specific promoters applicable to targeting strategies, transcriptional targeting using the promoter of the target cell ensures that the introduced gene is expressed only in specific cell types. However, since the activity of each promoter varies, it is essential to verify promoter activity when the level of gene expression is a critical factor. In neuroscience research, the most commonly used promoters for neuronal activity regulation are listed in Table 2 (Promoters Used for Neuronal Activity Regulation and Their Cell Specificity).

[Table 2]

| Promoter | Abbreviation | Specificity |
|---|---|---|
| Human glial fibrillary acidic protein | hGFAP | astrocytes |
| Tyrosin hydroxyase | TH | catecholarminergic neurons |
| Choline acetyltransferase | CHAT | cholinergic neurons |
| Dopamine beta hydroxylase | DBH | dopaminergic neurons |
| Ca2+/calmodulin dependent protein kinase 2 | CaMK2 α | excitatory neurons in the cortex and hippocamus, amygdala |
| Vesicular GABA transporter | VGAT | GABAergic neurons and glycinergic neurons |
| Fugu somatostatin | fSST | inhibitory neurons |
| Elongation factor 1 α | EF1α | neurons |

(continued)

| Promoter | Abbreviation | Specificity |
|---|---|---|
| Neuron-specific enolase | NSE | neurons |
| Tubulin $\alpha$ 1 | T$\alpha$1 | neurons |
| Superior cervial ganglion 10 | SCG10 | neurons |
| Neurofilament heavy chain | NFH | neurons |
| Myelin basic protein | MBP | oligodendrocytes |
| Human synapsin1 | hSyn1 | pan-neuronal |
| Human thymocyte 1 | hThy1 | pan-neuronal |
| Parvalbumin | Pvalb | Pvalb-positive interneurons |
| Tryptophan hytdroxylase2 | TPH2 | serotonergic neurons |

Regarding recombinases applicable to targeting strategies, the use of promoters for cell-type-specific targeting has limitations. These include low promoter activity, which may result in poor gene expression, or excessively long promoter sequences, which can pose challenges for viral packaging. Recombinase-dependent expression of target mechanosensitive ion channels significantly helps to overcome these limitations. For example, a widely used general promoter such as Efla can be engineered by inserting a recombinase-responsive genetic sequence and then packaged into a viral vector. Subsequently, this vector can be co-administered with a virus expressing the recombinase, or it can be introduced into genetically modified animals that already express the recombinase. This approach ensures strong and selective gene expression exclusively in cells that express the recombinase within the targeted region where the virus is administered. Hundreds of Cre-, Flp-, or Dre-expressing mouse lines are available for such applications. Using this method, optogenetic techniques have been employed to demonstrate the involvement of parvalbumin-expressing interneurons in the cerebral cortex in regulating social behavior in animals. Additionally, cholinergic neurons located in the septum have been shown to regulate hippocampal circuit activity. Another strategy involves using recombinase-dependent suppression of gene expression, which has been applied to study the electrophysiological characteristics of non-cholinergic neurons in the globus pallidus externa that extend into the frontal lobe.

If two or more types of recombinases are used, the INTRSECT (INTronic recombinase sites enabling combinatorial targeting) strategy can be applied. For example, by designing a genetic sequence that is expressed in a Cre recombinase-dependent manner but not expressed in an Flp recombinase-dependent manner, it is possible to specifically target dopaminergic neurons in the ventral tegmental area. Similarly, this strategy can be used to target interneurons in the hippocampus that simultaneously express parvalbumin and somatostatin.

Regarding neuronal circuit targeting strategies, stimulating the soma or axonal regions of neurons enables the targeting of neuronal networks connecting two or more brain regions. For neurons with long axons, applying this approach may be challenging. In such cases, it is advisable to allow sufficient expression of the target mechano-sensitive ion channels within the cells or incorporate the neuritin 3' UTR (untranslated region) to ensure uniform expression of the target mechano-sensitive ion channels along the long axons. If the ionic environment differs between two brain regions (e.g., variations in chloride ion exchange, hydrogen ion buffering, or hydrogen ion-induced currents), the effects of the target mechano-sensitive ion channels may differ between the soma and axon. In such scenarios, a chloride ion pump, which is less sensitive to ionic differences, may be employed as a strategy to inhibit neuronal activity selectively.

**[Regulation of Anion Transport Membrane Protein Gating and Neuronal Activity Inhibition via Acoustic Patterns]**

Neuronal activity can be regulated in two directional modes: activation and inhibition. Currently, inhibition of neuronal activity through pharmacological agents, electrical stimulation, or magnetic field-based stimulation is employed as a therapeutic approach. However, these three methods share a fundamental limitation: they lack specificity in targeting the intended neuronal population.

In contrast, the present invention is characterized by its ability to target specific mechanosensitive ion channel proteins within a designated body region to enable drug action without off-target effects. This is achieved by identifying and utilizing the resonance frequency(ies) of the target mechanosensitive ion channels within specific cells of the target body region. Accordingly, in the present invention, an acoustic pattern that utilizes the resonance frequency(ies) of the target mechanosensitive ion channel to regulate its gating can also be applied as a method to inhibit neuronal activity by targeting anion transport membrane proteins as drug targets.

Furthermore, in the present invention, by combining the use of an acoustic pattern that employs the resonance frequency(ies) of the target mechanosensitive ion channel within specific cells of a target body region with a sonogenetic approach-a type of biological technique- it is possible to achieve targeted neuronal inhibition restricted to specific neurons through the expression of anion transport membrane proteins in the designated neuronal cells.

In Example 4, a magnetogenetic approach utilizing magnetic nanoparticles and a rotating magnetic field generator was employed as a neuronal activation technique, where anion transport membrane proteins were used as drug target proteins instead of cation transport membrane proteins. As a result, it was confirmed that by utilizing an electroceutical device providing mechanical stimulation patterns in combination with magnetogenetics, it is possible to modulate the opening and closing of anion transport membrane proteins and thereby inhibit neuronal activity.

Specifically, when the drug target protein is DmFLYC1 ion channel and the tagging protein is MYC-tag, the binding moiety that interacts with the drug target protein-MYC antibody-binds to MYC-tag. When a magnetic field is applied, magnetic nanoparticles injected into the organism rotate, exerting mechanical force on the DmFLYC1 ion channel. In response to this mechanical stimulation, the DmFLYC1 ion channel opens. Upon the opening of DmFLYC1, $Cl^-$ ions influx into the cell, leading to hyperpolarization of the cell membrane potential, which corresponds to the results shown in Figs. 12 and 13.

Similarly, when the acoustic wave pattern of the present invention targets anion transport membrane proteins as a drug target and utilizes the resonant frequency(ies) of the target mechanosensitive ion channel within a specific cell of a target body region, it can regulate cellular activity. If the target cell is a neuron, this process can be employed to inhibit neuronal activity.

Ion transport membrane proteins are known to undergo mechanically gated opening and closing, depending on their intrinsic properties, and anion transport membrane proteins, in particular, play a key role in neuronal inhibition.

Ion transport membrane proteins include both channel proteins and pump proteins, but are not limited thereto. Specific examples include: chloride ion ($Cl^-$) transport membrane proteins, such as FLYC channels, CLC channels, E-CLC channels, CLIC channels, and GABA receptors; non-selective anion transport membrane proteins, such as MscS channels, MscL channels, SWELL channels, ANO channels, and Maxi anion channels; and other ion pumps involved in anion transport.

The transported anions primarily include chloride ions ($Cl^-$), but are not limited to $Cl^-$ alone and may involve various combinations of anions.

The acoustic wave pattern of the present invention, which utilizes the resonant frequency(ies) of the target mechanosensitive ion channel within a specific cell of a target body region to control the opening and closing of the target mechanosensitive ion channel, can also be applied to inhibiting neural activity by targeting anion transport membrane proteins as drug targets. Therefore, it can be used for both basic scientific research and clinical applications, as described below.

(1) Investigation and characterization of specific neuronal properties through the inhibition of neuronal activity.
(2) Study of neural circuit connectivity by suppressing neuronal activity.
(3) Identification and analysis of drug mechanisms of action on neurons via neuronal activity suppression.
(4) Discovery and research of behavioral mechanisms in animals through in vivo inhibition of neuronal activity.
(5) Suppression of excessive neuronal activity in epileptic brains as a therapeutic approach.
(6) Reduction of hyperactivity caused by excessive neuronal activity in ADHD patients.
(7) Suppression of excessive pain perception in chronic pain disorders.

The acoustic wave pattern optimized for the resonant frequency(ies) of the target mechanosensitive ion channel to activate or reversibly inhibit the target mechanosensitive ion channel at a desired site, according to the present invention, can be used for the treatment of epilepsy, ADHD, chronic pain disorders, or the suppression of pain perception.

[Computer Software-Related Inventions Accompanying Biotechnological Inventions]

To enable the brain function control device or the mechanosensitive ion channel gating control device of the present invention to be executed by a computer, the present invention provides a computer-readable recording medium or a medium for transmitting a program to a computer for generating an acoustic wave pattern optimized for the resonant frequency(ies) of the target mechanosensitive ion channel to activate or reversibly inhibit the target mechanosensitive ion channel at a desired site.

The present invention provides computer software that utilizes the interference effects of ultrasound while inducing resonance phenomenon of the target mechano-sensitive ion channel, and generates a spatiotemporally controlled sequence of weak-intensity one-dimensional, two-dimensional, or three-dimensional ultrasound patterns such that the activation of non-target cells remains unaffected. Accordingly, the present invention provides a computer-readable recording medium or a medium for transmitting a program to a computer for generating an acoustic wave pattern optimized for the resonant frequency(ies) of the target mechanosensitive ion channel, in order to activate or reversibly inhibit the target mechanosensitive ion channel at a desired site in the brain function control device or the mechanosensitive ion channel gating control device of the present invention.

As used herein, the term "computer" refers to a device with information processing capabilities. Information processing involves computing or processing information for a specific purpose.

The term "software" refers to a collection of instructions and commands (including voice or video information) that enable a computer or its peripheral devices to execute commands, accept input, process data, store information, generate output, and interact with other systems.

The term "computer program" refers to a program installed in a computer that performs specific functions. It consists of a set of instructions suitable for executing on a computer.

The term "data recording medium" refers to a computer-readable medium that records structured data, which determines the type of processing performed by the computer due to its recorded data structure.

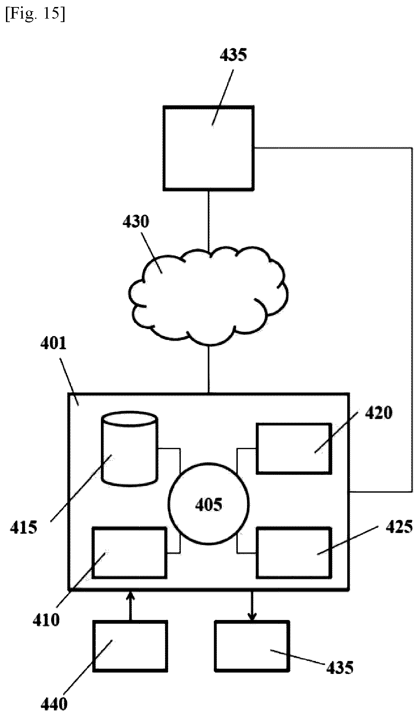

In some embodiments, when an optimized ultrasound condition or pattern for the resonant frequency(ies) of the target mechanosensitive ion channel is input to activate or reversibly inhibit the target mechanosensitive ion channel at a desired site, the generation of the optimized ultrasound for the resonant frequency(ies) of the target mechanosensitive ion channel is processed on a server or computer server (see fig. 15). In certain embodiments, the server 401 includes a central processing unit (CPU, also referred to as a "processor") 405, which may be a single-core processor, multi-core processor, or multiple processors for parallel processing. In some embodiments, the processor used as part of the control assembly is a microprocessor. In some implementations, server 401 further includes: memory 410 (e.g., random access memory (RAM), read-only memory (ROM), or flash memory); electronic storage unit 415 (e.g., a hard disk); communication interface 420 for communication with one or more other systems (e.g., a network adapter); and peripheral devices 425, including cache memory, additional memory, data storage, and/or an electronic display adapter. Memory 410, storage unit 415, interface 420, and peripheral devices 425 communicate with processor 405 through a communication bus (solid line), such as a motherboard. In some embodiments, storage unit 415 serves as a data storage unit for storing data. Server 401 is operatively connected to a computer network ("network") 430 via communication interface 420. In certain implementations, additional hardware-assisted processors are also operatively coupled to the network. In some embodiments, network 430 may include the Internet, an intranet, and/or an extranet, as well as an intranet and/or extranet communicating with the Internet, a telecommunications network, or a data network.

In some embodiments, network 430, assisted by server 401, implements a peer-to-peer (P2P) network, enabling devices coupled to server 401 to operate as either a client or a server. In certain implementations, the server can transmit and receive computer-readable instructions (e.g., device/system operating protocols or parameters) or data (e.g., sensor measurements, raw data obtained from the detection of metabolic byproducts, analysis of raw data obtained from metabolic byproduct detection, and interpretation of such data) via electronic signals transmitted through network 430. Furthermore, in some embodiments, the network is used to transmit or receive data across international borders.

In some embodiments, server 401 communicates with one or more output devices 435, such as a display or a printer, and/or one or more input devices 440, for example, a keyboard, mouse, or joystick. In certain embodiments, the display is a touchscreen display, which functions as both an output and an input device. In some implementations, different and/or additional input devices such as an enunciator, speaker, or microphone may be present. In certain embodiments, the server operates using any of various operating systems, including, for example, Windows®, MacOS®, Unix®, or some version of Linux®.

In some embodiments, storage unit 415 stores files or data related to the operation of the device, system, or method described herein.

In certain embodiments, the server communicates with one or more remote computer systems via network 430. In some implementations, the one or more remote computer systems include, for example, a personal computer, laptop, tablet, phone, smartphone, or personal digital assistant (PDA).

In some embodiments, the control assembly includes a single server 401. In other situations, the system comprises multiple servers that communicate with each other via an intranet, extranet, and/or the internet.

In certain embodiments, server 401 is configured to store device operating parameters, protocols, the methods described herein, and potentially other related information. In some implementations, such information is stored in storage unit 415 or on server 401, and such data is transmitted via the network.

The transmission of programs, data, and other specified information can be conducted through conventional communication networks and communication lines.

Non-limiting examples of computer-readable recording media include hard disks, floppy disks, magnetic recording media, and optical recording media. Non-limiting examples of transmission media include communication (transmission) media, carrier waves, carriers, and communication (transmission) mechanisms.

**[Indications for the Use of Acoustic Wave Patterns as Bioelectronic Medicine Targeting the Resonance Frequency(ies) of Drug Target Proteins]**

The acoustic wave pattern of the present invention, which functions as a form of bioelectronic medicine, is not limited to mechanosensitive ion channels as drug targets. Rather, it can be expanded to include drug target proteins that undergo conformational changes in response to resonance frequency(ies) that induce resonance phenomena-for example, receptors that exhibit conformational changes upon ligand binding.

The conformational change of the drug target protein can lead to activation of intracellular signaling pathways and, in some cases, physiological responses. Such pathways may include gene expression, enzyme activity modulation, ion channel gating, or other cellular responses. The cellular responses can be highly diverse and may involve changes in cellular metabolism, cell division, muscle contraction, neural transmission, or other physiological processes.

A physiological response refers to biological changes that an organism exhibits in reaction to its environment or stimuli, encompassing physical reactions and alterations in biological activity. Physiological responses may be triggered by emotions, stress, nutrition, or physical stimuli. Examples of physiological responses include changes in heart rate, respiratory rate, and blood pressure, among other bodily functions.

Therefore, the acoustic wave pattern of the present invention can be designed to function similarly to ligands such as hormones, neurotransmitters, or other signaling molecules (e.g., cytokines, chemokines); agonists, antagonists, activators, inhibitors, or blockers of drug target proteins; or even therapeutic antibodies.

According to the present invention, an acoustic wave pattern that utilizes the resonant frequency(frequencies) of a drug target protein in specific cells within a targeted body region can precisely regulate the activity of the drug target protein through spatiotemporally diverse mechanical stimulation patterns that induce conformational changes. Non-limiting examples of specific cells include neuronal cells, endocrine cells, epidermal cells, epithelial cells, muscle cells, immune cells, and cancer cells. Therefore, through acoustic wave patterns, which provide mechanical stimulation to drug target proteins, positive modulation of pathological processes can be achieved. For example, the acoustic wave pattern can function as an antagonist, activator, inhibitor, or blocker for drug target proteins embedded in biological lipid membranes.

For example, if the drug target protein is an ion channel or a receptor, an acoustic wave pattern that utilizes the resonant frequency(frequencies) of a specific drug target protein in specific cells within a targeted body region, according to the present invention, can be used as a therapeutic agent for a specific disease. Through the conformational changes experienced by the drug target protein, biological activity (e.g., cell depolarization/hyperpolarization, modulation of protein expression levels) or therapeutic effects can be induced within milliseconds, seconds, or hours. Ion channels that exhibit biological activity or therapeutic effects within seconds may include ligand-gated ion channels, ion channel-linked receptors, or G-protein-coupled receptors (GPCRs).

Ion channel-linked receptors, also known as ionotropic receptors, represent a class of cell membrane receptors that are directly coupled to ion channels. These receptors play a fundamental role in neural signaling and transmembrane electrical signal transmission. Ion channels are broadly distributed across various tissues and are involved in synaptic transmission, sensory perception, and muscle contraction.

The fundamental structure of an ion channel-linked receptor consists of two primary components, an extracellular ligand-binding domain and a transmembrane ion channel domain. These structural elements are typically part of the same protein or exist as closely associated subunits.

The extracellular ligand-binding domain is located outside the cell and contains binding sites for specific chemical messengers, such as neurotransmitters or hormones. When these ligands bind to the receptor, the receptor protein undergoes a conformational change, leading to the activation of the associated ion channel.

The transmembrane ion channel domain spans the cell membrane and forms the ion-conducting pore through which ions can flow. This domain consists of multiple transmembrane segments that create a hydrophilic pathway for ion passage. The opening and closing of the ion channel are controlled by ligand-induced conformational changes in the extracellular domain.

When a ligand binds to the extracellular domain, the receptor undergoes a structural transformation, leading to the opening of the ion channel. This allows specific ions, such as sodium ($Na^+$), potassium ($K^+$), calcium ($Ca^{2+}$), or chloride ($Cl^-$), to flow across the cell membrane. The movement of these ions generates electrical currents that alter membrane potential and trigger downstream signaling events.

The activation of ion channel-linked receptors is typically rapid and transient, enabling fast signal transmission and electrical excitability in the nervous system. The duration of ion channel opening is generally brief, ranging from a few milliseconds to several seconds, depending on the specific receptor and its regulatory mechanisms.

Examples of ion channel-linked receptors include the nicotinic acetylcholine receptor, which is involved in neuromuscular transmission, and the NMDA (N-methyl-D-aspartate) receptor, which plays a crucial role in synaptic plasticity and learning in the brain.

Ion channel-linked receptors enable the direct coupling of ligand binding to ion flow, allowing for the efficient and precise transmission of signals across the cell membrane. The activation of these receptors and the subsequent ion conductance are critical for physiological processes and serve as therapeutic targets for various diseases and disorders.

According to the present invention, an acoustic wave pattern that utilizes the resonant frequency(frequencies) of a specific drug target protein in specific cells within a targeted body region can exert a pharmacological effect if the drug target protein is a ligand-gated ion channel, by modulating its gating mechanism. To achieve this, the acoustic wave pattern can be optimized to maintain the ion selectivity or narrow selectivity filter function of the drug target ion channel protein, considering Pore & Filter Selectivity: $Na^+ > Li^+ \gg K^+, Ca^{2+}, Mg^{2+}$.

According to the present invention, an acoustic wave pattern optimized for the resonant frequency(frequencies) of a drug target protein can be designed to specifically target neurons in order to activate or reversibly inhibit the drug target protein in a desired region.

By generating an acoustic wave pattern that utilizes the resonant frequency(frequencies) of a drug target protein in specific neurons within a targeted brain region, the present invention enables the stimulation and control of specific neuronal populations. This approach not only facilitates a deeper understanding of brain function but also offers a potential therapeutic strategy for treating neuron-based disorders.

When targeting brain and neural regions with distinct functional domains, precise stimulation is crucial. First, an accurate mapping of the functions of neurons and tissues within the body must be established. According to the present invention, when an acoustic wave pattern utilizing the resonant frequency(frequencies) of a drug target protein within specific cells of a targeted body region is applied, it is possible to collect clinical data on the effects of the controlled mechanical stimulus intensity and periodicity, as well as biological signals. Additionally, a closed-loop system (a system where the output signal directly influences the control action) can be implemented to gather biofeedback information.

Since the present invention involves stimulating sensitive nervous system within the human body, it is essential to collect and analyze biological signals in real-time to ensure immediate adjustments. By integrating a closed-loop control system with software, it becomes possible to precisely measure the effects of an acoustic wave pattern, optimized for the resonant frequency(frequencies) of a drug target protein, on a specific body region-even wirelessly. The mechanism of action for the acoustic wave pattern optimized for the resonant frequency(frequencies) of a drug target protein, which is designed to activate or reversibly inhibit the drug target protein in a specific region, may vary among patients depending on the therapeutic effect on their condition. Therefore, to achieve optimal therapeutic outcomes, it is necessary to establish a clear mechanistic understanding and develop a system that prescribes personalized physical stimulation intensity and periodicity for each patient. Additionally, the physical stimulation of the acoustic wave pattern(condition), optimized for the resonant frequency(frequencies) of the drug target protein, may be prescribed in combination with conventional chemical drugs to enhance therapeutic effects in activating or reversibly inhibiting a targeted drug target protein within a specific region.

Additionally, the optimized acoustic wave pattern (condition) using the resonance frequency(ies) of the drug target protein, designed to activate or reversibly inhibit the target drug target protein in a desired region, can selectively target specific types of brain cells located in deep brain tissues. In this case, the drug target protein selectively targeted through the optimized acoustic wave pattern (condition) using the resonance frequency(ies) of the drug target protein may be naturally expressed in or artificially introduced into specific types of brain cells. For example, by modifying Example 3, which employs magnetogenetic technology, the optimized acoustic wave pattern (condition) using the resonance frequency(ies) of the drug target protein in accordance with the present invention can regulate brain cells in the lateral hypothalamus (LH), the central hub for appetite control, thereby modulating an animal's feeding behavior and food intake. Furthermore, to activate or reversibly inhibit the drug target protein in a desired region, the acoustic wave pattern (condition) optimized to the resonance frequency(ies) of the drug target protein in a desired region can regulate the activity of the drug target protein in a wireless and remote manner with a desired temporal resolution and accuracy. Consequently, it enables relatively precise neural activity activation and/or inhibition on a sub-second timescale.

Accordingly, the acoustic wave pattern (condition) optimized to the resonance frequency(ies) of the drug target protein for activating or reversibly inhibiting the target drug target protein in a desired region, as disclosed in the present invention, can be used for neuromodulation by targeting the central nervous system (CNS), which regulates metabolism through the release of endocrine neuro-signals, or the peripheral nervous system (PNS), which transmits neural signals released from the CNS to organs.

Accordingly, the acoustic wave pattern (condition) optimized to the resonance frequency(ies) of the drug target protein for activating or reversibly inhibiting the target drug target protein in a desired region, as disclosed in the present invention, can be used as a physiological function-controlling drug or a behavior-modulating drug by mechanically stimulating targeted neural or muscle cells in a non-invasive manner.

Furthermore, the acoustic wave pattern utilizing the resonance frequency(ies) of the drug target protein in specific cells within a targeted body region, as disclosed in the present invention, can be used to control animal behavior or to treat physiological disorders or diseases through long-term neuron-specific neuromodulation.

The peripheral nervous system (PNS) refers to all components of the nervous system except the central nervous system (CNS). It consists of nerves extending from the CNS to various parts of the body, connecting the nerves of organs, skin, and limbs to the CNS. The PNS is divided into afferent nerves (which transmit sensory information to the brain and spinal cord) and efferent nerves (which convey motor commands from the brain and spinal cord to organs or muscles). Functionally, the PNS is further classified into the somatic nervous system (SNS) and the autonomic nervous system (ANS). The somatic nervous system refers to the involuntary control of smooth muscles and glands and the autonomic nervous system refers to the involuntary control of smooth muscles and glands. The somatic nervous system is responsible for transmitting sensory information to the brain and spinal cord and relaying motor commands from the CNS to muscle fibers. Neural signals transmitted through the SNS can be utilized in controlling artificial limbs and detecting artificial sensory input. The autonomic nervous system regulates visceral reflexes and maintains homeostasis by controlling key biological processes such as blood pressure, body temperature, and metabolism. The ANS is further subdivided into the sympathetic nervous system and the parasympathetic nervous system. The sympathetic nervous system regulates physiological responses that help the body cope with environmental changes and potential threats by increasing heart rate, respiration, blood flow to muscles, sweat secretion, and pupil dilation. The parasympathetic nervous system counterbalances the sympathetic nervous system by slowing heart rate and respiration, reducing blood flow, and constricting pupils once the body is no longer in a stressful situation, thereby maintaining normal physiological functions. Additionally, the peripheral nerves play a critical role in connecting the brain and internal organs. Afferent peripheral nerve stimulation, which transmits visceral information to the brain, can be leveraged to modulate anxiety disorders.

The body contains numerous peripheral nerves, among which the vagus nerve plays the most significant role in regulating metabolism within the autonomic nervous system. The vagus nerve establishes distinct connections with individual organs, and organ-specific control is mediated by spike-patterned action potential activity. The pattern of action potentials determines the release of neurotransmitters, thereby influencing intracellular signaling cascades and modulating the activity of target cells or neighboring cells.

When an acoustic wave pattern optimized for the resonance frequency(ies) of a drug target protein is applied to the drug target protein to activate or reversibly inhibit it in a desired region, the mechanical stimulation induced by the acoustic wave pattern can promote recovery and regeneration after neural injury and restore lost functions. Furthermore, stimulation using an acoustic wave pattern optimized for the resonance frequency(ies) of a drug target protein can facilitate axonal regeneration in damaged peripheral nerves, enhance functional connectivity, and activate spinal cord circuits.

The neural control method of the present invention, which utilizes an acoustic wave pattern(condition) optimized for the resonance frequency(ies) of a drug target protein to activate or reversibly inhibit the target protein in a desired region, can regulate the activation of neurotransmitters by artificially modulating action potentials. This, in turn, can help restore physiological homeostasis in disrupted metabolic functions. Beyond vagus nerve stimulation, sacral nerve stimulation can also be applied as a therapeutic approach for treating fecal incontinence and gastrointestinal disorders.

As used herein, the term "subject" refers to a mammal, including but not limited to humans, primates, mice, rats, cattle, pigs, horses, sheep, dogs, and cats.

## ADVANTAGEOUS EFFECTS

According to the present invention, when an acoustic wave pattern optimized for the resonance frequency(ies) of a target mechano-sensitive ion channel is used as an electroceutical providing a mechanical stimulation pattern to control the opening and closing of the target ion channel within specific cells of a target body region, the modulation of the ion channel can be achieved with high spatiotemporal resolution. This precise control enables the induction of desired pharmacological effects.

## BRIEF DESCRIPTION OF THE DRAWINGS/FIGURES

Fig. 1 is a schematic diagram illustrating a sonogenetics technology that spatiotemporally stimulates cells expressing mechano-sensitive ion channels using an acoustic wave pattern optimized for the resonance frequency(ies) of a target mechano-sensitive ion channel to control its opening and closing within specific cells of a target body region.

Fig. 2 presents (a) c-Fos images as a cellular activity marker under various conditions according to Example 1, and (b) a quantitative graph depicting the level of cellular activation under the respective conditions.

Fig. 3 illustrates ultrasound patterning according to Example 2, including: (a) ultrasound patterning using a circular "ultrasound patterner", (b) ultrasound patterning using an "IOI"-shaped ultrasound patterner, and (c) an example of complex-shaped ultrasound patterning ("Y"-shaped) generated using a deep learning algorithm.

Fig. 4 is a conceptual diagram illustrating the biomolecular mechanisms of ultrasonic neuromodulation.

Fig. 5 is a conceptual diagram illustrating the bidirectionality of neuromodulation and comparing the operational principles of optogenetics and magnetogenetics.

Fig. 6 illustrates the objectives of Example 3 and provides an overview of cell-type-specific neuromodulation using magneto-mechanical genetics (MMG).

Fig. 7 is a conceptual diagram illustrating the research strategy and methodology employed in Example 3.

Fig. 8 illustrates the cell-type-specific neuromodulation using magneto-mechanical genetics (MMG) as applied in Example 3.

(a) MMG apparatus. The MMG apparatus with various sizes (16, 35, and 60 cm) is constructed for both *in vitro* and *in vivo* studies. For *in vivo* experiments of untethered, freely moving animals, the apparatus with 60 cm diameter and 20 cm height is used. As a magnetic field rotates with 0.5 Hz, m-Torquer rotates at the same frequency as the magnetic field, generating forces to activate Piezo1 channels.

(b) Schematic illustration showing a patch-clamp set-up for electrophysiological recording during MMG stimulation.

(c) Representative whole-cell voltage-clamp trace from a Piezo1-expressing (black) and a EGFP-transfected (gray) HEK293 cell evoked by a single pulse (1.0 s, 0.5 Hz, 180°) MMG stimulation. (right inset) Quantification of peak current amplitudes. Data is the mean $\pm$ s.d. of n = 9 cells. *** p < 0.001; two-tailed Student's t test.

(d) Representative current traces recorded from a Piezo1- and EGFP-expressing HEK cell in response to a train of 6 repetitive magnetic pulses. Each black bar represents a single magnetic pulse with a 1-sec duration and 0.5 Hz frequency.

(e) Experimental procedure of Cre-loxP based cell-type specific MMG control of deep brain neural circuit. The Cre-loxP technique is employed to enable cell type-specific expression of Piezo1 mechanosensitive ion channels. m-Torquers are specifically labeled to Piezo1-expressing neurons. Upon a rotating magnetic field, torque forces generated by m-Torquer mechanically open the bound Piezo1 channels and activate specific neurons.

(f, g) Representative fluorescence histology images of the lateral hypothalamus (LH) region showing co-localization of Piezo1 with Vgat in Vgat-Piezo1 mouse (f) or with Vglut2 in Vglut2-Piezo1 mouse (g) Scale bar, 50 $\mu$m.

(h) Statistical analysis of the fraction of Piezo1-expressing cells that are Vgat-positive or Vglut2-positive in respective Vgat-Piezo1 or Vglut2-Piezo1 mice. n = 3 animals.

(i) Representative histology images showing co-localization of m-Torquers and Piezo1 expression in the LH region. Scale bar, 10 $\mu$m.

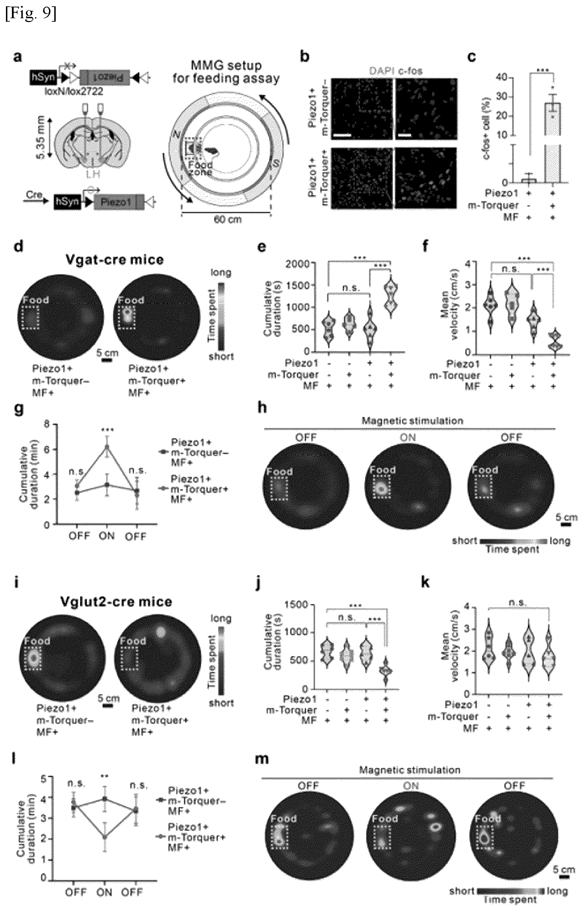

Fig. 9. presents the Vgat- and Vglut2 specific MMG of the lateral hypothalamus (LH) for the bidirectional regulation of real-time animal feeding.

(a) Scheme for viral targeting of Ad-hSyn-FLEX-Piezo1 to the LH of Cre mice and real-time feeding assay.

(b) Fluorescence histology images of Vgat-Piezo1 brain slices for c-Fos expression (red) at the LH in the absence (upper) and in the presence (lower) of m-Torquers upon MMG stimulation. Scale bar, 50 $\mu$m. Right: magnified images of the boxed region. Scale bar, 20 $\mu$m.

(c) Quantification of c-fos-positive cells in the LH of Vgat-Piezo1 mice upon MMG stimulation. Data is the mean $\pm$ s.d. of n = 3 animals. *** p < 0.001; two-tailed Student's t test.

(d) Representative spatial heat map for MMG stimulation of Vgat neurons in the LH of Vgat-Piezo1 mice during real-time feeding assay.

(e, f) Statistical analysis of feeding duration (e) and locomotion (f) of Vgat-Piezo1 mice during MMG stimulation.

(g) Statistical analysis of feeding duration and (h) representative spatial heap map of Vgat-Piezo1 mice during 30-min home cage feeding task with three 10-min epochs (pre-stimulation, during stimulation, and post-stimulation).

(i) Representative spatial heat map for magnetogenetic stimulation of Vglut2 neurons in the LH of Vglut2-Piezo1 mice during real-time feeding assay.

(j, k) Statistical analysis of feeding duration (i) and locomotion (j) of Vglut2-Piezo1 mice during MMG stimulation.

(l) Statistical analysis of feeding duration and (m) representative spatial heap map of Vglut2-Piezo1 mice during 30-min home cage feeding task with three 10-min epochs (pre-stimulation, during stimulation, and post-stimulation). (e, f, g, j, k, l) All data are presented as mean $\pm$ s.d.; ** p<0.01; *** p < 0.001; ns, non-significant; one-way ANOVA followed by Tukey's multiple comparison tests; n = 5-8 animals per group.

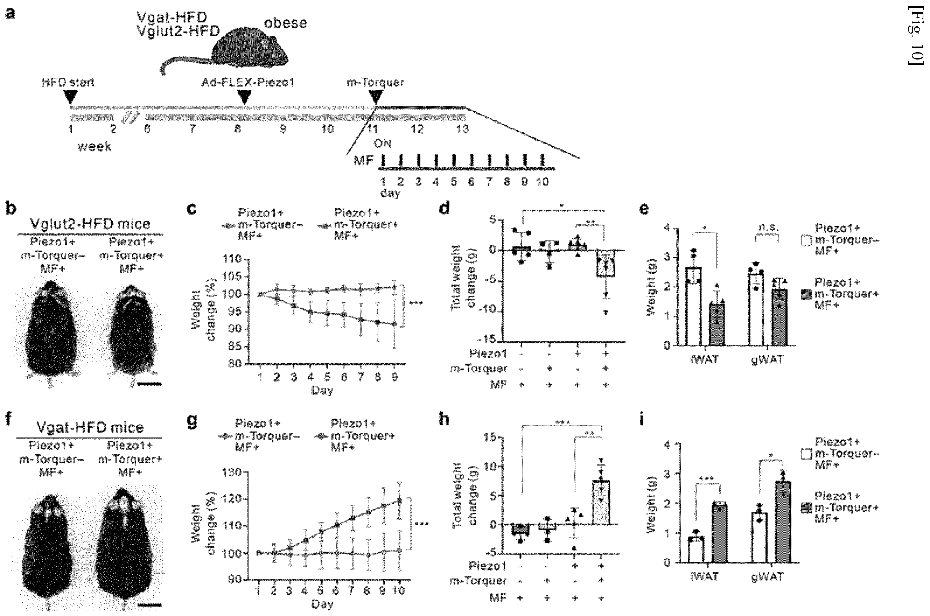

Fig. 10 demonstrates a Long-term cell-type specific neuromodulation for controlling dietary habit and body-weight change in mice.

(a) *In vivo* experimental scheme for long-term neuromodulation of Vglut2- or Vgat-Piezo1 HFD obese mice. Diet-induced obesity was induced by the 8-week high-fat diet regime. After Cre-dependent Piezo1 expression and m-Torquer delivery into the LH, mice were subjected to 2-week MMG stimulation (1-hr/day, 0.5 Hz).

(b) Representative photograph showing Vglut2-Piezo1 HFD mice in the absence of m-Torquers (left) and in the presence of m-Torquers (right) after long-term stimulation.

(c) Body-weight changes of Vglut2-Piezo1 HFD mice over 10 days of MMG stimulation (1-hr/day, 0.5 Hz). Body weights were measured daily. n = 6 (Piezo1+ m-Torquer-) and 6 (Piezo1+m-Torquer+) animals per group.

(d) Statistical analysis of total weight changes after long-term MMG stimulation in Vglut2-Piezo1 HFD mice.

(e) Statistical analysis of mass (gram) of white adipose tissues acquired from Vglut2-Piezo1 HFD mice after 2-week MMG stimulation.

(f) Representative photograph showing Vgat-Piezo1 HFD mice in the absence of m-Torquers (left) and in the presence of m-Torquers (right) after long-term stimulation.

(g) Body-weight changes of Vgat-Piezo1 HFD mice over 10 days of MMG stimulation (1-hr/day, 0.5 Hz). Body weights are measured daily. n = 5 (Piezo1+ m-Torquer-) and 5 (Piezo1+m-Torquer+) animals per group.

(h) Statistical analysis of total weight changes after long-term MMG stimulation in Vgat-Piezo1 HFD mice.

(i) Statistical analysis of mass (gram) of white adipose tissues acquired from Vgat-Piezo1 HFD mice.

(b, f) scale bar = 2 cm, (c, g) All data are presented as mean $\pm$ s.d.; Two-tailed unpaired student's t test, *** p<0.001. (d, e, h, i) All data are presented as mean $\pm$ s.d.; One-way ANOVA with multiple comparison test, * p<0.05, *** p<0.01, n.s., non-significant. n = 5 (Piezo1-m-Torquer-), 4 (Piezo1-m-Torquer+), 6 (Piezo1+m-Torquer-) and 6 (Piezo1+m-Torquer+) animals per group. (e, i) iWAT and gWAT stand for inguinal white adipose tissue and gonadal white adipose tissue, respectively.

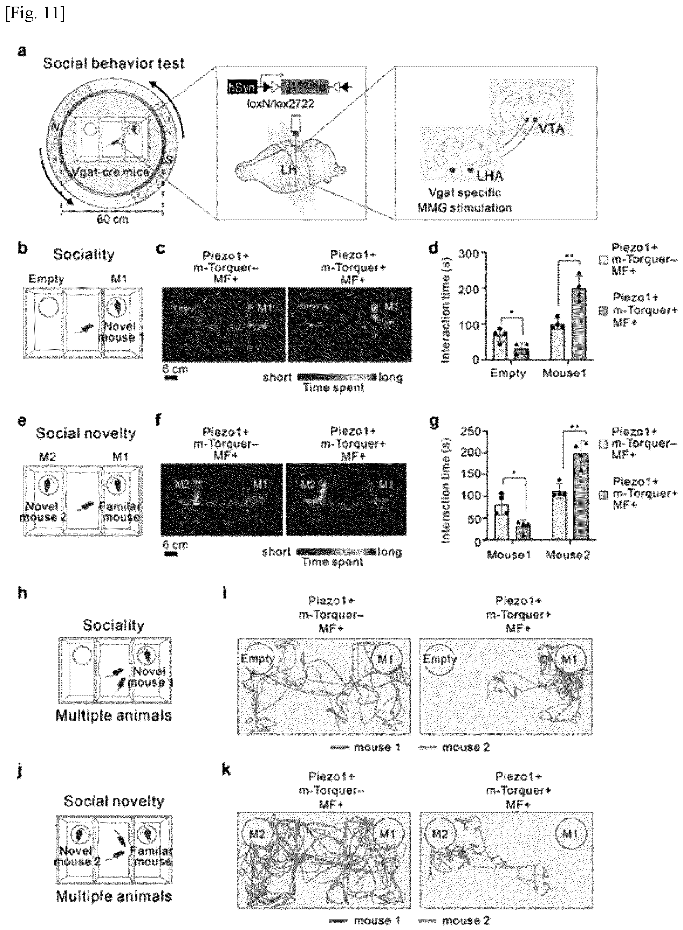

Fig. 11 shows the MMG stimulation of GABAergic neurons in the LH for promoting sociality and social novelty.

(a) (left) MMG setup for the three-chamber sociality assay. (center) Viral delivery for Cre-dependent Piezo1 expression in Vgat neurons in the LH$_A$. (right) Schematics of LH$_A$-VTA projection regulating social behaviors.

(b) Schematic of the three-chamber sociality test.

(c) Representative heat map encoding the spatial location of a Vgat-Piezo1 mouse without (left) and with (right) m-Torquers in a three-chamber sociality test during MMG stimulation.

(d) Quantification of time spent with an empty or an M1 chamber during 5-min stimulation.

(e) Schematics of the three-chamber social novelty test.

(f) Representative heat map encoding the spatial location of a Vgat-Piezo1 mouse without (left) and with (right) m-Torquers in the social novelty test during MMG stimulation.

(g) Quantification of time spent with a familiar mouse (M1) or a second novel mouse (M2) during 5-min stimulation. Activity heat maps and total time spent in the social interaction zone in the three-chamber test are increased in Vgat-specific mice delivered with m-Torquers upon MMG stimulation.

(h) Schematics of three-chamber sociality test with multiple (two) mice using MMG.

(i) Representative spatial map tracking the movement of two Vgat-Piezo1 mice without (left) and with (right) m-Torquers in sociality assays upon MMG stimulation.

(j) Schematics of three-chamber social novelty test with multiple (two) mice using MMG.

(k) Representative spatial map tracking the movement of two Vgat-Piezo1 mice without (left) and with (right) m-Torquers in social novelty assays upon MMG stimulation. (d, g) All data are presented as mean $\pm$ s.d.; * p<0.05, ** p<0.01; two-tailed unpaired Student's t test; n = 4 for Vgat-Piezo1 without m-Torquers; n = 4 for Vgat-Piezo1 with m-Torquers.

Fig. 12 illustrates the method for detecting Cl⁻ influx using the MQAE quenching technique.

Fig. 13 presents the results of measuring the membrane potential of cells expressing DmFLYC1 ion channels after treatment with magnetic nanoparticles and subsequent exposure to a magnetic field over a defined time period, as performed in Example 4.

Fig. 14 is a conceptual diagram illustrating how Piezo1 ion channels respond to various mechanical force activations, leading to chronic inflammatory diseases in multiple systems (Front. Immunol., 26 January 2022, Sec. Inflammation, Volume 13 - 2022).

Fig. 15 is a conceptual schematic diagram of an exemplary computer server used to process the system and method described in the specification.

## Mode for carrying out the invention

The following examples are provided to facilitate understanding of the invention. These examples are presented solely for illustrative purposes and do not limit the scope of the invention.

## Example 1: Verification of Cellular Stimulation Effects Using Ultrasound

As a model system, HEK293 cells were utilized, and the Piezo1 ion channel, a mechanosensitive ion channel, was expressed in these cells to enable responsiveness to mechanical forces. To achieve this, $5\times10^5$ cells were seeded in a 60 mm dish, and 5 $\mu$g of CMV-Piezo1 plasmid was transfected using Lipofectamine 3000, following the standard protocol.

HEK293 cells expressing Piezo1 were then exposed to two different ultrasound frequencies, 2.0 MHz and 6.25 MHz, for 10 minutes, corresponding to the resonance frequency(ies) of Piezo1 in HEK293 cells. The objective was to observe differences in cellular activation based on frequency. The extent of cellular activation was quantified by immunostaining for c-Fos, a reporter of intracellular calcium ion influx, and the number of activated cells was represented as a percentage.

When exposed to low-frequency ultrasound (2.0 MHz), the percentage of activated cells increased by 6.8-fold compared to the non-ultrasound group (5.9% vs. 40.3%, Fig. 2, Groups B and C). When exposed to high-frequency ultrasound (6.25 MHz), the percentage of activated cells increased approximately 12-fold compared to the non-ultrasound group (5.9% vs. 70.6%, Fig. 2, Groups B and D). This activation level was comparable to that observed upon treatment with Yoda1 (10 μM), a chemical agonist of Piezo1 (74% activation, Fig. 2, Group F).

In contrast, HEK293 cells that did not express Piezo1 showed no increase in cellular activation under the same ultrasound conditions (0.0% in both Fig. 2, Groups A and E).

These findings demonstrate that ultrasound can selectively activate cells expressing mechanosensitive ion channels without affecting non-expressing cells. Furthermore, the degree of activation varies with ultrasound conditions, indicating the existence of optimal stimulation parameters. Notably, the current ultrasound conditions achieved an effect comparable to that of a chemical agonist.

**Example 2: Ultrasound Shape Patterning Using Interference Effects**

Ultrasound utilizes wave interference effects to prevent ultrasound waves from reaching certain spatial regions or focus them into specific areas. This principle can be applied to design ultrasound waves into desired shapes and patterns.

To validate this concept, various shapes of "ultrasound patterners (US-patterners)" were fabricated and attached in front of an ultrasound generator to shape and control the ultrasound waveforms. To visualize ultrasound intensity, polydimethylsiloxane (PDMS) microparticles, which respond to ultrasound by aggregating in areas of high ultrasound intensity, were synthesized and used.

For PDMS, the SYLGARD 184 product from Dow Chemical was used. It was mixed at 10% concentration with 1% Pluronic F-127 aqueous solution (a nonionic surfactant) and then hardened in an emulsion using a homogenizer (10,000 rpm). The resulting PDMS particles were then filtered using a strainer to select only particles between 30-100 μm for use.

Using 3D printing, circular and IOI-shaped patterners were fabricated and attached in front of the ultrasound generator. When PDMS microparticles were exposed to ultrasound, within 10 seconds, the particles aggregated into the same shape as the patterners (Fig. 3a). However, the interference effect of the waves was not sufficiently distinct, leading to blurred pattern boundaries and unintended ultrasound focusing in undesired areas. This issue can be resolved using open-source deep learning algorithms and simulations.

For example, when attempting to create a 'Y'-shaped ultrasound pattern, the algorithm can generate the optimal patterner design, which can then be fabricated using 3D modeling and printing. These results demonstrate that wave interference effects can be used to design ultrasound into specific patterns, and that precise 2D ultrasound patterning is possible using computational algorithms. Furthermore, the feasibility of 3D patterning suggests that ultrasound-based cellular stimulation can be spatially controlled with high spatial resolution in three-dimensional space.

**Example 3: Wireless cell-type specific magnetic control of neural activities in live animals**

[Methods]

Following the methodology outlined in Nature Materials (2022) (Nat Mater. 2021 Jul;20(7):1029-1036), the following components were prepared (1) m-Torquer, (2) m-Torquer conjugated with fluorescent particles and Myc antibodies,(3) Magnetic simulation, and (4) CMA (Controlled Magnetic Actuation).

**3-1. Synthesis of m-Torquer**

The m-Torquer was synthesized by conjugating azide-functionalized MNPs onto DBCO-functionalized supports as reported previously with slight modifications[17]. To synthesize the azide-functionalized magnetic nanoparticles (MNPs), MNPs were coated with silica as reported previously with little modification to ensure biocompatibility[46]. A mixture was prepared with 1 nmol MNPs, 78 g Igepal CO-520 (Sigma-Aldrich), 21 ml ammonium hydroxide (Sigma-Aldrich), and 200 μl tetraethylorthosilicate (Sigma-Aldrich) in 2500 ml cyclohexane. After 2 days, 2 μl aminopropyltrimethoxysilane (Sigma-Aldrich) was added and the solution was shaken at 200 r.p.m. for 2 hr at room temperature. Amine-functionalized MNPs were washed via centrifugation and redispersed in DMSO for further surface modification with tetrafluorophenyl (TFP) ester. To prepare azide-functionalized MNPs, 50 μmol azido-dPEG12-TFP ester (Quanta Biodesign) was reacted with 1 nmol amine-functionalized MNPs in dimethyl sulfoxide (DMSO) overnight. The azide-functionalized MNPs were purified via centrifugation and redispersed in DMSO.

To prepare DBCO-functionalized supports, 2.5 mmol DBCO-PEG5-TFP ester (Click Chemistry Tools) was reacted with 1 nmol amine-functionalized polystyrene microspheres (0.2 μm, Polyscience) overnight by copper-free click chemistry[47]. The DBCO-functionalized supports were purified via centrifugation and redispersed in DMSO. Then, to synthesize the m-Torquer, azide-functionalized MNPs, and DBCO-functionalized microspheres were mixed at a molar ratio of 2000:1. After 8 h, the m-Torquer was purified by centrifugation and redispersed in DMSO. For further functionalization, carboxylate groups were introduced by adding DBCO-PEG5-COOH (Click Chemistry Tools) and succinic anhydride (Sigma Aldrich) to the solution each at a final concentration of 1 mg·ml$^{-1}$. Carboxylated m-Torquer was purified via centrifugation and redispersed in 10 mM phosphate buffer (pH 7.2). The synthesized m-Torquer was characterized with an SEM using JSM-7900F with PC-SEM software.

### 3-2. Synthesis of m-Torquer conjugated with Myc antibodies

For Myc antibody surface functionalization, 100 $\mu$g protein A (Sigma Aldrich) was first conjugated onto 1 mg carboxylated m-Torquer via EDC/NHS coupling. After purification by centrifugation, 40 ug Myc antibody (Sigma Aldrich) was added to protein A conjugated m-Torquer. Myc antibody conjugated m-Torquer was purified by centrifugation and redispersed in 10 mM phosphate buffer (pH 7.2) at a final concentration of 50 mg·ml$^{-1}$.

### 3-3. Magnetic setup for *in vivo* MMG stimulation

For *in vivo* MMG stimulation of freely moving mice in a minimally restrained setup, the 60 cm (diameter) in-house-built magnetic arena was used. To build the 60 cm magnetic setup, ten 1T NdFeB magnets (12.5 × 15 × 12.5 cm) were assembled in a stainless stress cage. The 60 cm magnetic setup was mounted on an in-house-built motorized rotation stage and controlled by Arduino. A specialized arena designed for various animal behavioral assays, including feeding, social interactions, and motor function, was assembled and placed into the center of the magnetic arena. The magnetic field strength of the various configurations of magnetic setup was simulated by finite element analysis using COMSOL Multiphysics.

### 3-4. Plasmid and viral vector construction

For *in vivo* and *in vitro* magneto-mechanical-genetics (MMG) experiments, Ad-hSyn-FLEX-Myc897-Piezo1. Mouse Piezo1 with myc tag inserted at residue 897 (Myc897-Piezo1) was cloned in an antisense direction to create Ad-pEnt-hSyn-FLEX-Myc897-Piezo1. Myc897-Piezo1 was flanked by a pair of canonical loxP sites and a pair of adenoviral constructs were then sent to KOMABIOTECH Inc., for adenovirus packaging with serotype 5. Lenti viral particles of serotype 5 were produced by the Viral Core Facility of KOMABIOTECH Inc. Lentiviral particles were produced in-home using HEK293T cells and 3$^{rd}$ generation lentiviral packaging system. For transduction, in LH with Ad-hSyn-FLEX-Myc897-Piezo1 virus, it has been reported that the synapsin (Syn) promotor is neuron-specific with little glial expression.

### 3-5. Expression of Piezo1 in primary cortical neurons

Cortical tissues were dissected from C57BL/6 mice (Nara Biotech) on embryonic day 14 and placed in prechilled Hank's buffered salt solution (HBSS, Gibco). Then, the tissues were trypsinized for 15 min and physically dissociated into single neurons. The dissociated neurons ($10^5$ cells·cm2) were plated on glass coverslips or confocal dishes coated with poly-D-lysine (Sigma-Aldrich) and laminin (Sigma-Aldrich), and incubated in neurobasal media (Gibco) containing 2% B27 supplement (Gibco), 0.5 mM L-glutamine (Gibco), 25 $\mu$M L-glutamate (Sigma-Aldrich) and 50 units per ml penicillin/streptomycin (Gibco) in 5% $CO_2$ at 37 °C. This experiment was carried out in accordance with the recommendations in the Guide for the Care and Use of Laboratory Animals of the Yonsei University Institutional Animal Care and Use Committee. The protocol was approved by the Committee on the Ethics of Animal Experiments of Yonsei University (approval number IACUC-A-201707-294-02). The recombinant adenovirus used for Myc897-Piezo1 expression was applied to cultured neurons on 4 DIV (days in vitro). The protocol for viral infection was approved by the Institutional Biosafety Committee of Yonsei University (approval number IBC-A-201706-146-01). After day five (9 DIV), 2 $\mu$l of m-Torquer (10 mg·ml$^{-1}$) was applied to the neurons.

### 3-6. HEK293 cell recording with the m-Torquer system

#### *Sample preparation:*

For the recording of HEK293 cells, a Piezo1 expressing HEK293 cellular line was generated using a PB transposon system following the manufacturer's protocol with slight modification. Briefly, 0.5 $\mu$g of super PiggyBac transposase (SBI #PB210PA-1) and 2.5 $\mu$g of Myc897-Piezo1 encoding PB transposon vector were transfected to 1x$10^6$ of HEK293 cells in a 6-well plate using Lipofectamine. After three days, puromycin selection was done to remove non-integrated cells. The HEK293 cell culture of the cellular line was maintained in Dulbecco's modified Eagle's medium with 4.5g/L glucose (DMEM; Gibco) supplemented with 10% fetal bovine serum (FBS; Gibco) and 1% penicillin-streptomycin (Gibco). A day before recording, cells were transferred to Poly-D-Lysine coated cover glass and incubated overnight. Subsequently, cells were treated with 8 $\mu$l of m-Torquer (50 mg/ml) diluted to 1 mL with culture medium for m-Torquer labeling. After 1 hr incubation at 37 °C, cells were washed with complete medium to remove unbound m-Torquers. Then, the cover glass with cells was loaded on a nonmagnetic bath chamber and placed upon electrophysiology equipment with the MMG stimulator system.

***Patch-clamp equipment with MMG stimulator system:***

Measurements were performed with a 700A Multiclamp Axon Amplifier and the signals were digitized with a Digidata 1440A using pClamp 11 software (all Molecular Devices). To apply MMG stimulation, we assembled 6 NdFeB magnets (2.5 × 3 x 4 cm) caged by a 3D printed holder, which arrangement was set by the simulation to generate a uniform magnetic field ($|B| \approx 25$ mT) at the center point of 3 cm apart. The assembled magnets were mounted above the electrophysiological recording space, and the motorized rotation of the magnet was controlled by customized Arduino (Namil Optical Instruments) connected with Digidata 1440A and thus synchronized with measurement. The assembled magnets were held by a separate pole apart from the faraday cage to avoid transmission of vibration generated by the rotation of magnets. The head stage was set apart 10 cm from the chamber loader to avoid noise and controlled by Motorized Micromanipulator (MM-500, RWD Life Science). The electrode holder (World Precision Instruments) was engaged with the head stage through a customized nonmagnetic holder.

***Electrophysiological recording of MMG-induced current:***

For the recording of MMG-evoked currents, whole-cell patch-clamp recording was performed with voltage-clamped condition ($V_h = -60$ mV). Cells were perfused in the bath solution with the following composition (in mM): 150 NaCl, 3 KCl, 10 HEPES, 5.5 glucose, 2 $MgCl_2$, 2 $CaCl_2$ with pH adjusted to 7.3 by NaOH (300 mOsmol/kg). Patch pipettes (5-8 M$\Omega$) were fabricated from borosilicate glass and filled with the inner solution with the following composition (in mM): 135 $CeMeSO_4$, 8 NaCl, 10 HEPES, 0.25 EGTA with pH adjusted to 7.3 by KOH (290 mOsmol/kg). All measurements were carried out at room temperature (23 °C). All data were reduced 100 times by data reduction of Clampfit software.

**3-7. In situ calcium imaging in live primary neurons with the m-Torquer system**

A solution containing 2 $\mu$l of X-Rhod-1 (5 mM, dispersed in 20% (w/v) Pluronic F-127 in DMSO, Thermo Fisher Scientific) was diluted in 1 ml cell culture medium and applied to primary neurons. Cells were incubated for 30 min at 37 °C, 5% $CO_2$. A 2 $\mu$l volume of m-Torquer (10 mg·ml$^{-1}$) was diluted to 200 $\mu$l with culture medium and applied to primary neurons. After a 1 h incubation at 37 °C, unbound particles were removed and 1 ml of HBSS containing 2 mM calcium chloride was added. Then, the cell culture dish was placed under the microscope. The CMA was positioned in the middle of the in-house-built plastic sample stage with a live-cell chamber (37 °C, 5% $CO_2$). The fluorescence signal of X-Rhod-1 was recorded with an EMCCD camera while the rotating magnetic field was applied (imaging rate: 20 frames per second).

**3-8. Animals**

All experiments were approved by the Yonsei University Institutional Animal Care and Use Committee (IACUC) and all procedures involving the handling of animals were in accordance with the National Institutes of Health guidelines. Male mice (>6 weeks old) were used for all behavioral and molecular studies. Mice were maintained in a temperature-controlled room (22 ± 1°C) on a 12 hr light-dark cycle with *ad libitum* access to food and water, and specific pathogen-free conditions. All WT C57/BL6 mice were acquired from Orient Bio and Central Lab. Animal Inc. Vgat::IRES-Cre: Slc32a1tm2(cre)Lowl/MwarJ and Vglut2::IRES-Cre: Slc17a6 m2(cre)Lowl/J mice[48,49] were acquired from Jackson Laboratory. All Cre mouse lines are in a wild-type (C57BL/6J) background.

**3-9. Genotyping**

DNA isolated from the tail biopsies was used for mouse genotyping of the Vgat::IRES-Cre or Vglut2::IRES-Cre gene. The DNA extraction process from tissue followed the MyTaq Extract-PCR Kit (Meridian Bioscience). The presence of the Vgat::IRES-Cre allele was verified by PCR amplification using the WT-Vgat and KI-Vgat primer sets. Conditions of PCR were as follows: denaturation steps at 95 °C for 3 min, followed by 35 cycles at 95 °C for 15 s, 60.5 °C for 15 s, and an elongation step at 72 °C for 20 s. The genotyping primers used for the RT-PCR assays are as follows: WT-Vgat primers, Forward 5'-CTTCGTCATCGGCGGCATCTG-3'; Reverse 5'-CAGGGCGATGTGGAATAGAAA-3'. KI-Vgat primers, Forward 5'-CACCCTGTTACGTATAGCCG-3'; Reverse 5'-GAGTCAT CCTTAGCGCCGTA-3'. The presence of the Vglut2::IRES-Cre allele was verified by PCR amplification using the WT-Vglut2 and KI-Vglut2 primer sets. Conditions of PCR were as follows: denaturation step at 95 °C for 3 min, followed by 35 cycles at 95 °C for 15 s, 60 °C for 15 s, and an elongation step at 72 °C for 20 s. The genotyping primers used for the RT-PCR assays are as follows: WT-Vglut2 primers, Forward 5'-AAGAAGGTGCGCAAGACG-3'; Reverse 5'- CTG CCACAG ATTGCA CTTGA-3'; KI-Vglut2 primers, Forward 5'- AAGAAGGTGCGCAAGACG-3', Reverse 5'- ACACCG GCCTTATTCCAAG-3'.

### 3-10. Surgical procedures

***General:***

All surgeries were performed on mice under aseptic conditions and body temperature was maintained with a heating pad. Mice were anesthetized intraperitoneally with a ketamine and xylazine solution (100 mg·kg$^{-1}$ of ketamine and 10 mg·kg$^{-1}$ of xylazine), and placed into a stereotaxic frame (David Kopf Instruments, Tujunga, CA, USA). All measurements were made relative to bregma for virus and m-Torquer surgeries. Corneas were protected from drying using eye gel (Puralube Vet Ointment, Dechra). After cleaning the periosteum, the vertical coordinates of bregma and lambda were measured to align in the same plane (level head). A craniotomy (~1 mm in diameter) was made above the injection site. The viral injection was performed using a beveled 33-gauge microinjection needle with a 10 $\mu$l microsyringe (UMP3; WPI, Sarasota, FL, USA), delivering the virus at a rate of 100 nl·min$^{-1}$ using a microsyringe pump and a controller (Micro4; WPI, Sarasota, FL, USA). After injection, the needle was left in place for 5 min to pass before withdrawing the needle 50-100 $\mu$m and leaving it for an additional 10 min to allow diffusion of the viral solution into the brain tissues before the needle was slowly withdrawn completely. After surgery, mice recovered from anesthesia under a heating pad. Animal experiments were carried out in accordance with the recommendations in the Guide for the Care and Use of Laboratory Animals of the Yonsei University Institutional Animal Care and Use Committee. The protocol was approved by the Committee on the Ethics of Animal Experiments of Yonsei University (approval number IACUC-A-202107-1299-05) and by the Institutional Biosafety Committee of Yonsei University (approval number IBC-A-202108-286-01).

***Viral injection for Cre-dependent Piezo1 expression:***

For Myc897-Piezo1 expression, all experimental mice underwent stereotaxic injections at the age of 8 weeks. For cell-type specific MMG stimulation targeting lateral hypothalamus (LH), Vgat::IRES-Cre or Vglut2::IRES-Cre mice were injected bilaterally with 1.0 $\mu$l adenovirus serotype 5 (AV5) encoding Myc897-Piezo1 under a double-floxed inverted open-reading frame construct (AV-hSyn-FLEX-Myc897-Piezo1) (1 x 10$^{12}$ VP (viral particles)/ml, KOMABIOTECH Inc.) For the control group without Piezo1 expression, a null version of the virus only carrying Myc897-Piezo1 without FLEX (AV-hSyn-Myc897-Piezo1) was injected into the LH using the following coordinate relative to lambda: AP (anteroposterior), -1.4; ML (mediolateral), $\pm$0.9; DV (dorsoventral), -5.35.

***M-Torquer introduction:***

3 weeks after viral injection, Vgat::IRES-Cre or Vglut2::IRES-Cre mice were injected bilaterally with M-torquer (50 mg·ml$^{-1}$) 1.0 $\mu$L at the following coordinate relative to lambda: AP (anteroposterior), -1.4; ML (mediolateral), $\pm$0.9; DV (dorsoventral), -5.35. M-Torquer injection was performed using a beveled 33-gauge microinjection needle with 10 $\mu$l microsyringe (UMP3; WPI, Sarasota, FL, USA), delivering virus at a rate of 100 nl·min$^{-1}$ using a microsyringe pump and a controller (Micro4; WPI, Sarasota, FL, USA). After injection, the needle was left in place for 5 min to pass before withdrawing the needle 50-100 $\mu$m and leaving it for an additional 10 min to allow diffusion of the m-Torquer into the brain tissues before the needle was slowly withdrawn completely. After surgery, mice recovered from anesthesia under a heating pad.

### 3-11. *In vivo* MMG stimulation

Mice were anesthetized as previously described. Using the LH coordinate, 1 $\mu$l of AV-hSyn-FLEX-Myc897-Piezo1 (titer) was injected bilaterally into the LH of 8 week old mice. For Piezo1(-) controls, the same volume of saline was injected instead of adenovirus. Three weeks later, 2.5 $\mu$l of 100 mg ml m-Torquers were injected into the same LH area under isoflurane anesthesia. For m-Torquer (-) controls, the same volume of saline was injected instead of nanoparticles. After 2-3 days of recovery, the mice were placed within the 60 cm MMG apparatus and magnetic arena. Mice received a uniform magnetic field, field gradient $\nabla$B < 10 T/m, and a magnitude of field |B| $\geq$ 20 mT for 30 min while allowed to freely roam around and behave naturally in all behavioral assays used in this study, including open chamber feeding assay, long-term MMG stimulation, and social interaction tests. *In vivo* MMG stimulation experiment was approved by the Committee on the Ethics of Animal Experiments of Yonsei University (approval number IACUC-A-202107-1299-05) and by the Institutional Biosafety Committee of Yonsei University (approval number IBC-A-202108-286-01).

**3-12. Behavioral tests: Open chamber, free-access feeding task**

Animals were placed in a custom circular, single-chamber (300 mm x 250 mm) arena with four food traps to quantify the amount of food consumed and assess the time spent in a designated food zone arena. The arena contained four accessible food traps. For short-term MMG experiments, pellets of standard mouse chow were placed into one of the chambers (designated food zone) while the other three traps remained empty. Mice were allowed to freely explore for 1 min and then received 30 min of continuous magnetic stimulation during which they were allowed to freely move and explore around the arena. To test on-demand, reversible activation of feeding, well-fed mice were monitored for 10 min pre-stimulation, 10 min magnetic stimulation, and 10 min post-stimulation. Their spatial locations, time spent at the food zone, and velocity were recorded via a CCD camera at 15 fps interfaced with EthoVision XT 10 (Noldus, Leesburg, VA) for individual mice in a semi-dark condition. All behavioral tests were performed under low light conditions, and animals were allowed to acclimate to the behavior room for a least 1 hr before the beginning of behavioral testing. Mice were habituated at least 3 days before with magnetic field sounds without stimulation for 30 min.

**3-13. Behavioral tests: Long-term MMG stimulation for diet-induced obese mice**

For the diet-induced obese (DIO) mouse study, Vgat::IRES-Cre or Vglut2::IRES-Cre male mice at the age of 6 weeks were placed on a 60 kcal % high-fat diet (HFD) for 8 weeks. Vgat- or Vglut2- DIO mice on HFD were stereotaxically injected with Ad-hSyn-FLEX-Myc897-Piezo1. After 3 weeks to induce Piezo1 expression, a second injection delivered m-Torquer (1 μl, 50 mg·ml⁻¹) using the same coordinate of the LH. Food intake and weight change of Vgat- or Vglut2- DIO mice were measured while applying magnetic field stimulation for 1 hr every day for 10-14 days. Change in body weight over time was normalized as a percentage of the day 1 initial starting weight for each animal. Fat mass from inguinal and gonadal white adipose tissues were measured from the mice after 10 days of MMG stimulation.

**3-14. Behavioral tests: Three-chamber sociability and social novelty**

Social interaction behaviors modulated by MMG stimulation were examined using the three-chambered social interaction assay as previously described with some modifications for MMG stimulation. The three-chambered apparatus, designed to assess the animal's preference for a social stimulus over a non-social stimulus and measure social novelty, had the dimension of (w) 510 × (D) 300 × (H) 250 mm) divided into 3 equal compartments ((w) 170 × (D) 300 × (H) 250 mm) by plastic dividers with a 2-inch opening in the middle. Vgat::IRES-Cre mice injected with Ad-hSyn-FLEX-Myc897-Piezo1 were assigned into one of the two groups: MMG group with m-Torquer (1 μl, 50 mg·ml⁻¹) injected into the LH_A and the control group without m-Torquer. The experimental mice were habituated to the arena for 5 min in the middle chamber without access to the side chambers. After all chambers were open, the mice were allowed to freely explore the entire apparatus for 5 min before a new mouse and object were placed into the arena. Only the mice which were not biased to either side were tested further for sociality and social novelty. A juvenile male (4-5 weeks of age, Vgat::IRES-Cre) mouse was placed into one of the chambers, and a 5 min recording session, during which mice were MMG stimulated (0.5 Hz), was initiated. A video camera was positioned above the magnetic arena recorded each trial, and mouse location and velocity were tracked. The videos, recorded at 15 fps, were analyzed using Ethovision XT software. For both control and MMG groups, the total amount of time spent investigating a novel mouse during magnetic field (MF) ON epochs was analyzed.

**3-15. Behavioral tests: Multiple animal social interaction test**

Social interaction test involving multiple animals in the same physical area was conducted using the same three-chambered apparatus previously described. For paired-mice social-interaction experiments, Vgat::IRES-Cre male mice born in the litter were bred in a cage and kept in a laminar airflow cabinet maintained at 22 ± 1 °C on a 12 hr light-dark cycle with *ad libitum* access to food and water, and specific pathogen-free conditions. Two Vgat::IRES-Cre mice injected with Ad-hSyn-FLEX-Myc897-Piezo1 were assigned into one of the two groups: MMG group with m-Torquer (1 μl, 50 mg·ml⁻¹) injected into the LH_A, and the control group without m-Torquer. The experimental mice were habituated to the arena for 5 min in the middle chamber without access to the side chambers. After all chambers were open, the mice were allowed to freely explore the entire apparatus for 5 min before a new mouse and object were placed into the arena. Only the mice which were not biased to either side were tested further for sociality and social novelty. A juvenile male (4-5 weeks of age, Vgat::IRES-Cre) mouse was placed into one of the chambers, and a 3 min recording session, during which mice were MMG stimulated (0.5 Hz), was initiated. A video camera was positioned above the magnetic arena recorded each trial, and mice location and velocity were tracked. The videos, recorded at 15 fps, were analyzed using Ethovision XT software. For both control and MMG groups, the total amount of time spent investigating a novel mouse during magnetic field (MF) ON epochs was analyzed.

### 3-16. Analysis of animal behavioral tests

A python program was manually developed to analyze behavioral tests (code is available at https://github.com/DHSHINN/Magnetogenetics). Raw data were collected by a CCD camera interfaced with EthoVision XT 10 (Noldus, Leesburg, VA) and primarily processed using Microsoft Excel to give out coordinates of mouse movement per time frame, and then was subsequently processed with Seaborn 0.11.0, Matplotlib 3.5.1, and Pandas 1.4.1 to generate heatmaps to visualize and track time spent on each region of the stage. Python code was modified for each unique dataset to normalize and process the coordinates to produce a heatmap and calculate the time spent on a defined area for each trial.

### 3-17. Immunohistochemistry

For immunohistochemical analysis, animals were perfused with phosphate-buffered saline (PBS, pH 7.4) followed by 4% paraformaldehyde in PBS for 1.5 hr after being exposed to a rotating magnetic field (0.5 Hz). Then, the brains were removed and post-fixed in the same fixative for 24 h at 4 °C, frozen and sectioned coronally at 40 $\mu$m thickness using a sliding microtome (Leica Microsystems GmbH, Germany) and stored in cryoprotectant at 4 °C. Brain slices were blocked for 1 hr with 3% bovine serum albumin (BSA) in PBS containing 0.1% Triton X-100 and 5% normal goat serum to prevent nonspecific binding. The subsequent immunostaining was performed with primary antibodies in PBS containing 5% normal goat serum overnight at 4 °C (anti-Myc (Cell Signaling Technology, Inc), 1:300; anti-c-Fos (Cell Signaling Technology, Inc), 1:200; anti-VGAT (Invitrogen), 1:200; anti-Vglut2 (Sigma-Aldrich), 1:200; anti-GFAP (DAKO), 1:200; anti-Iba1 (Wako chemicals, Inc), 1:200; and anti-NeuN (Abcam), 1:200). On the following day, tissues were washed three times with PBS, appropriate secondary antibodies (anti-rabbit IgG (Alexa Fluor 488, Abcam, polyclonal goat antibody), 1:200; anti-mouse IgG (Alexa Fluor 488, Abcam, polyclonal goat antibody), 1:200; anti-rabbit IgG (Alexa Fluor 647, Abcam, polyclonal goat antibody), 1:200; anti-rabbit IgG (Alexa Fluor 647, Abcam, polyclonal donkey antibody), 1:200) were applied for 4 hr at room temperature and stained with 4',6-Diamidino-2-phenylindole dihydrochloride (DAPI) (Invitrogen). Subsequently, sections were washed three times with PBS and mounted on a Crystal Mount™ Aqueous Mounting Medium (Sigma-Aldrich). A confocal microscope (Leica, Germany) was used to observe fluorescence using LasX software. All imaging parameters were constant across all samples. Z-stack and tiled images of mounted brain sections were captured with a 10x, 25x, and 63x objective. For c-Fos quantification, Fiji or ImageJ with thresholding and segmentation functions was used to quantify the number of c-Fos-labeled neurons among all DAPI-stained neurons within the LH. For Piezo1 colocalization with Vgat or Vglut2 neurons, the number of Piezo1-, Vgat-, and Vglut2-expressing neurons were counted and quantified using a similar analysis.

### 3-18. *In vitro* 3D human brain phantom experiment

HEK293 cell culture was maintained in Dulbecco's modified Eagle's medium with 4.5g/L glucose (DMEM; Gibco) supplemented with 10% fetal bovine serum (FBS; Gibco) and 1% penicillin-streptomycin (Gibco). For MMA stimulation in a 3D human brain-like phantom, HEK293 cells were co-transfected with pcDNA3.1-pCMV-FLEX-Myc897-Piezo1 and pCMV-Cre (Addgene #123133) expression plasmids using 10 ul lipofectamine 3000 with 5000 ng of total DNA in Opti-MEM medium (Gibco). After 2 days of recovery and Piezo1 expression, the cells were treated with 8 $\mu$l of m-Torquer (50 mg/ml) diluted to 1 ml with culture medium for m-Torquer labeling. After a 3 hr incubation at 37 °C, cells were washed with complete medium to remove unbound m-Torquers. A total of $5x10^5$ cells were transferred to and embedded in the Matrigel matrix (size = 1.8 cm x 1.8 cm x 1.8 cm, volume = 120 ul). MMG stimulation was provided for 30 min with 0.5 Hz frequency using the MMG-60 apparatus. The samples were analyzed using RT-PCR for *c-fos, Piezol, Cre,* and $\beta$-*actin* mRNA expression.

### *RT-PCR:*

Total RNA was extracted from cells using the RNAeasy Mini Kit (QIAGEN, Hilden, Germany). cDNAs were synthesized using 3 $\mu$g of total RNA, oligo dT (10 pmol), and SuperScript reverse transcriptase III (200 units/$\mu$l) in a total reaction volume of 20 $\mu$l. The primer sequences used for *c-fos, Piezol, Cre, and $\beta$-actin.* The primers used for the RT-PCR assays are as follows: *c-fos* primers, Forward 5'-CAAGCGGAGACAGACCAACT-3'; Reverse 5'-AGTCAG ATCAAGGGAAGCCA-3'; *Piezol* primers, Forward 5'-TTCTTCGGGTTGGAGAGGTA-3', Reverse 5'-TGTCACCATGT GGTTAAGGATG-3; *Cre* primers, Forward 5'-GCCTGCATTACCGGTCGA TGCAAC-3', Reverse 5'-CGTATATCCTGGCAGCGAT CGC-3; $\beta$-*actin* primers, Forward 5'-GCACCACACCTTCT ACAATG-3', Reverse 5'-TGCTTGCTGATCCACATCTG-3.

*IVIS optical imaging:*

HEK293 cells were co-transfected with pCMV-Luc and pcDNA3.1-pCMV-Myc897-Piezo1 expression plasmids using 10 $\mu$l Lipofectamine 3000 with 5000 ng of total DNA in Opti-MEM medium (Gibco). After 2 days of recovery and Piezo1 expression, the cells were treated with 8 $\mu$l of m-Torquer (50 mg/ml) diluted to 1 ml with culture medium for m-Torquer labeling. After a 3-hr incubation at 37 °C, cells were washed with complete medium to remove unbound m-Torquers. A total of $5 \times 10^5$ cells were transferred to and embedded in the Matrigel matrix (size = 1.8 cm x 1.8 cm x 1.8 cm, volume = 120 ul). MMG stimulation was provided for 30 min with 0.5 Hz frequency using the MMG-60 apparatus. Cells embedded in the Matrigel were treated with 150 $\mu$g/ml D-luciferin diluted in media for 30 min. Chemi-luminescence signals from luciferase expression were captured using the IVIS Lumina III In Vivo Imaging System (PerkinElmer). Total radiant efficiency of the samples was measured using Living Image software 4.7.4 (PerkinElmer).

*Luciferase reporter assay:*

For luciferase assay, $Ca^{2+}$ dependent luciferase reporter-expressing HEK293 cell line was generated using lentiviral transduction. Briefly, lentivirus for CSN-mCMV-eGFP-2A-FLuc were applied to $5 \times 10^4$ of HEK293 cells cultured in a 6-well plate. Luciferase reporter-expressing HEK293 cells were co-transfected with pcDNA3.1-pCMV-FLEX-Myc897-Piezo1 (2000 ng) and pCMV-Cre (Addgene #123133, 4000 ng) expression plasmids using 10 $\mu$l Lipofectamine 3000 in Opti-MEM medium (Gibco). After 2 days of recovery and Piezo1 expression, cells were treated with 8 $\mu$l of m-Torquer (50 mg/ml) diluted to 1 ml with culture medium for m-Torquer labeling. In vitro MMG stimulation was performed using a 35-cm MMG apparatus with 0.5 Hz for 24 hrs. Then, cells were washed with complete medium to remove unbound m-Torquers and analyzed for luciferase activity using a EnSight Multimode Microplate Reader (PerkinElmer). All reactions were performed in triplicate. Reporter activity was normalized to total protein amount to determine transfection efficiency.

## 3-19. Statistical analyses

Statistical analysis was performed using GraphPad Prism 9.3.1 software (GraphPad) or Microsoft Excel.

**[Results ]**

## 1. Cell-type specific MMG neuromodulation in freely behaving animals

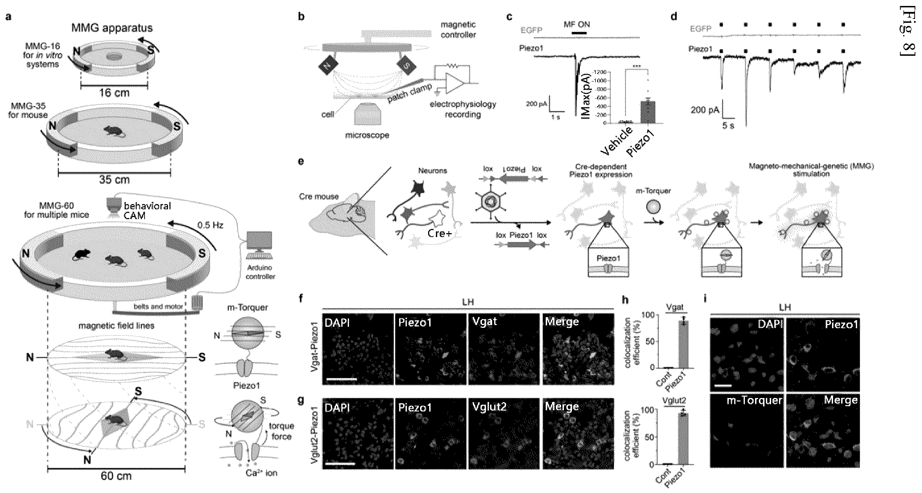

For the MMG stimulation of the target neurons in freely behaving animals the MMG apparatus was constructed as follows: a rotational magnetic force generator (MFG), a CMOS camera for behavior monitoring (behavior CAM), and an Arduino controller **(Fig 8a).** MFGs with different sizes can be constructed depending on applications: 16-cm for *in vitro* (cells), 35-cm for a single mouse, and 60-cm for multiple mice and a large animal **(Fig 8a).** The 60-cm MFG has a magnetic arena of 60 cm in diameter and 20 cm in height with NdFeB magnets for a uniform magnetic field, $|B| \approx 20$ mT and field gradient, $VB < 10$ Tm$^{-1}$, rotating at slow speed (0.5 Hz) (*c.f,* 100 kHz for MTG setup) **(Fig 8a).** The nanomagnetic torque generator (m-Torquer) is a 200 nm magnetic nanoparticle for torque generation under a rotating magnetic field. When the rotating magnetic field is ON, MFG generates ~2 pN of force over the entire magnetic arena to actuate Piezo1 channel for calcium entry and subsequently neural excitation **(Fig 8a).**

To examine whether Piezo1 ion channel gating by MMG stimulation induces the changes in membrane potential and current, Cre-dependent Piezo1-expressing HEK293 (Piezo1+) cells were electrophysiologically recorded under whole-cell, voltage-clamped mode at -60 mV. A novel patch-clamp system capable of synchronized electrophysiological recording under a uniform, rotating magnetic field ($|B| \approx 25$ mT, $VB < 10$ Tm$^{-1}$) was constructed **(Fig 8b).** Application of a rotating magnetic field (0.5 Hz, 1 sec) induced large and rapid depolarizing currents in Piezo1+ cells, which reached a maximum rise rate of $128 \pm 28$ pA/ms (mean $\pm$ s.d., n = 9, **Fig 8c**). Mean whole-cell inward currents were significantly larger in Piezo1+ cells ($|I_{max}| = 516 \pm 83$ pA) than in controls transfected with empty vector, which showed negligible responses ($|I_{max}| = 35 \pm 7$ pA) **(Fig 8c, inset).** Both electrophysiology and imaging of $Ca^{2+}$ influx using X-Rhod-1 showed a response latency of ~100 ms, which is within the range of Piezo1 gating kinetics[29,30] but slower than the kinetics of opsins[2] (**Fig 8c**). The force transfer from m-Torquer to the channel is direct and thus immediate, resulting in relatively faster kinetics than the other magnetogenetics approaches including MTG where the channel gating is mediated by indirect heat diffusion through space. In addition, a train of magnetic pulses (0.5 Hz, 6 pulses, with each pulse of 1-sec duration, and with an interval of 9-sec) elicited a series of current spikes with reliable timing and amplitude in Piezo1+ cells while the controls do not show any current **(Fig 8d).** Repeated stimulations induced currents with variable amplitudes, consistent with a previous report[31] that repetitive mechanical stimulation of Piezo1 in HEK293 cells invokes current peaks with varying amplitudes.

To achieve neuron-specific MMG stimulation *in vivo,* two Cre-dependent mouse lines, Cre-vesicular $\gamma$-amino-

butyric acid transporter (Vgat) and Cre-vesicular glutamate transporter 2 (Vglut2) with selective expression of mechanosensitive Piezo1 channels were established. Vgat and Vglut2 are the promoters specifically expressed in GABAergic and glutamatergic neurons, respectively, which have distinct physiological roles in regulating neural networks. Mechanosensitive Piezo1 ion channel modified with a Myc tag for labeling of m-Torquers was packaged into the adenovirus for Cre-dependent neuron-specific expression (Ad-hSyn-FLEX-Myc897-Piezo1) **(Fig 8e)**. The adenovirus was stereotaxically delivered to the target brain region of Cre mice to selectively express Piezo1 in GABAergic or glutamatergic neurons, respectively.

Neural cell-type specific expression of Piezo1 was confirmed by strong co-localization fluorescence signals of Piezo1 (green) and Vgat or Vglut2 (red) **(Fig 8f, g).** The majority of the Piezo1-expressing neurons were Vgat- (93%) and Vglut2-immunopositive (90%), indicating robust and selective neuron-type specific expression of Piezo1 **(Fig 8h).** In addition, Piezo1 signals were primarily detected in the lateral hypothalamus (LH) region and primarily localized to the cell membrane, indicating region-specific and membrane-specific expression. The m-Torquers labeled these Piezo1-expressing neurons, showing fluorescence signals of m-Torquers (red) were co-localized with Piezo1 (green) **(Fig 8i).**

## 2. MMG stimulation of lateral hypothalamus neurons for bidirectional modulation of feeding

After expressing Piezo1 followed by m-Torquer introduction into the brain, *in vivo* neuromodulation with m-Torquer-based MMG was demonstrated by stimulating two distinct neuronal populations, i.e., GABAergic and glutamatergic neurons in the LH. Located in the deep brain, the LH contains the GABAergic and glutamatergic neurons with complementary functions that together form neural circuits regulating complex behaviors including feeding and reward[34-38]. Adenovirus for Cre-dependent neuron-specific Piezo1 expression (hSyn-FLEX-Myc897-Piezo1) was stereotaxically injected into the LH in both hemispheres (X= $\pm 0.9$ mm; Y= -1.4 mm; Z= -5.35 mm) of either Cre-Vgat or -Vglut2 mice. Three weeks after the Piezo1 expression, m-Torquers were injected at the same location using the defined injection condition (1.0 $\mu$l at 100 nl·min$^{-1}$ rate) **(Fig 9a, left).** These mice were habituated in a MMG setup for 2-3 days and stimulated by the rotating magnetic field with varying periods. Neuronal activation by Yoda1 (an agonist for Piezo1) or MMG stimulation was examined by histological analysis of c-Fos, a marker of neural activity. MMG stimulation triggered neural activity, confirmed by increased c-Fos expression (red) in the LH **(Fig 9b).** A significantly higher proportion of c-Fos-positive cells was observed in both Vgat-Piezo1 (27 %) **(Fig 9b, c)** and Vglut2-Piezo1 (17%) mice than in controls without m-Torquer (0.12% in Vgat-Piezol, 0.14% in Vglut2-Piezo1).

We chose, as a first example of complex animal behaviors, the bidirectional control of mouse feeding behaviors by cell-type specific MMG neuromodulation. We conducted the real-time food intake assay with a designated food zone in a MMG setup where the mouse was placed and monitored for feeding during 30 min MMG stimulation **(Fig 9a, right).** The heatmaps of mice trajectories show that the MMG stimulation elicited acute feeding behavior in Vgat-Piezo1 mice with m-Torquer (Vgat-MMG mice) but not in controls (Vgat-Piezo1 mice without m-Torquer) **(Fig 9d).** Food intake duration of Vgat-MMG mice increased significantly (20.4 min) in response to a magnetic field. It is >2-fold increase compared to control groups without m-Torquers (8.5 min), Piezo1 (10.5 min), and both (8.8 min) **(Fig 9e).** Vgat-MMG mice exhibited diminished locomotion by ~50% compared with control groups, possibly due to increased staying time at the designated food zone **(Fig 9f).** This feeding behavior by magnetic neuromodulation was also reversible. A 30 min home cage feeding task, comprised of three 10 min epochs (pre-stimulation, stimulation, and post-stimulation) was conducted, and the mice displayed significantly increased food intake during the stimulation (6.2 min) and then ceased food intake after the stimulation (2.3 min), returning to the level of pre-stimulation (3.0 min) **(Fig 9g, h).**

After confirmation of the increased feeding behavior of MMG-stimulated Vgat mice, we tested the Vglut2 mice in the same experimental condition. Stimulations of Vgat and Vglut2 are known to be bidirectional in neural circuitry toward animal behaviors, and the excitation of Vglut2 should deactivate the feeding. After m-Torquer delivery into Vglut2-Piezo1 mice to create Vglut2-MMG mice, the mice were allowed to feed naturally and performed a real-time feeding assay during magnetic stimulation for 30 min. In response to a magnetic field, Vglut2-MMG mice showed an acute response: the feeding behavior ceased, and the mouse moved away from the food zone, as shown in the heatmaps **(Fig 9i).** Overall food intake time of Vglut2-MMG mice was decreased significantly in response to a magnetic field (5.4 min), compared to control groups without m-Torquers (10.6 min), Piezol (9.8 min), and both (11.2 min) **(Fig 9j).** The locomotion of mice, shown as mean velocity, did not vary much between the stimulated and the control groups, indicating that the reduced feeding did not affect locomotion **(Fig 9k).** Based on a 30-min feeding task, Vglut2 neuron-specific stimulation was also reversible as Vglut2-MMG mice exhibited reduced feeding behavior during the magnetic field ON (2.0 min) while turning the magnetic field OFF promoted feeding behavior (3.5 min), returning to the level of pre-stimulation (3.7 min) **(Fig 9l, m).** These data support the findings that excitatory and inhibitory neuronal populations in the LH produce opposite effects in the regulation of mice feeding [34,36,39]. In sum, our results confirm our MMG system is a reliable and important addition to the repertoire of neuromodulation for examining the roles of different neuronal cell types or circuits.

## 3. Application of MMG for long-term obesity modulation

While long-term, chronic brain stimulation is critical for modeling behaviors and diseases, current methods show challenges due to the physical tethers and thereby restriction in animal behaviors [40]. We applied long-term neuromodulation using MMG in the complex syndrome of obesity to interrogate the effects of prolonged modulation of mice feeding behaviors. Vgat- or Vglut2-Cre mice were maintained in an 8-week high-fat diet (HFD) to generate a mouse model of diet-induced obesity (DIO) and allowed for Cre-dependent Piezo1 expression to create either Vglut2-Piezo1 or Vgat-Piezo1 lines **(Fig 10a)**. After m-Torquer delivery at week 11, the mice were treated with daily 1 hr, 0.5 Hz MMG stimulation in a magnetic arena with access to food for 2 weeks, and their body weight and food intake were monitored **(Fig 10a)**. In the case of HFD Vglut2-Piezo1 mice, after the session, the results showed substantial weight loss and alleviation of obesity, while controls did not **(Fig 10b, c)**. Gradual weight reductions on a daily basis were observed **(Fig 10c)**, resulting in a significant body-weight reduction by ~4.3 g on average (from 49.2 g on day 1 to 44.9 g on day 10), which is equivalent to 10 % body weight loss, while the control groups without m-Torquers, Piezo1, or both maintained obesity albeit MMG stimulation **(Fig 10d)**. The total amount of food intake was also significantly decreased in MMG stimulated Vglut2 mice (by 25.2 g) compared to the control groups without m-Torquers (50.8 g), Piezo1 (48.2 g), and both (53.5 g). Anatomical examinations showed reduced body fat: the sizes of inguinal and gonadal white adipose tissue (iWAT and gWAT) were reduced compared to the control groups (by 47% and 21% for iWAT and gWAT, respectively) **(Fig 10e)**.

In the case of HFD Vgat-Piezo1 mice, on the contrary, substantial weight gains and aggravation of obesity were observed after two weeks of stimulation **(Fig 10f, g)**. Body weight was increased by 7.5 g on average (from 39.2 g on day 1 to 46.8 g on day 10), being equivalent to 18 % body weight gain compared to the controls **(Fig 10g, h)**. Total food intake was also increased considerably (40 g), compared to control groups without m-Torquers (25.4 g), Piezo1 (25.1 g), and both (27.4 g), and both iWAT and gWAT were noticeably enlarged compared to the control groups (120% and 61% enlargement, respectively) **(Fig 10i)**. Histological analysis showed negligible expressions of GFAP and Iba1 (markers for tissue inflammation), and a normal level of NeuN (a marker for neurons), suggesting long-term MMG stimulation did not induce any cytotoxicity or inflammatory responses. Overall, these results show that long-term, neuron-specific neuromodulation is possible by MMG, controlling the animal behaviors bidirectionally. These results imply the therapeutic potential for alleviating physiological disorders while allowing animals to behave freely during stimulation without implanted devices otherwise needed in opto- and electro-genetics.

## 4. Wireless control of social behavior in multiple animals)

With capabilities for wireless and large-area neuromodulation in freely moving animals, this MMG technology can be used for examining the circuitry that underlies complex social behaviors [6,41-43]. The three-chamber cage assay for assessing sociality and social novelty was conducted with the MMG setup **(Fig 11a)**. The stimulation of GABAergic neurons in the lateral hypothalamus area (LH$_A$) projects inhibitory inputs to VTA GABAergic neurons, which in turns reinforces/promotes social interaction **(Fig 11a)**. Adenovirus of hSyn-FLEX-myc897-Piezo1 was injected into the LH$_A$ region (X= ±0.9 mm; Y= -1.4 mm; Z= -5.35 mm) of Vgat-Cre mice, and subsequently m-Torquer for MMG stimulation. To assess sociality, the first novel mouse (M1; male, 4-5 weeks juvenile) was placed into one of the chambers the in MMG setup where the test mouse (Vgat-MMG) was habituated. Then, during the magnetic stimulation (0.5 Hz rotating magnetic field, 5 min), the time spent by the Vgat-Piezo1 mouse engaging in an empty chamber or M1 was measured **(Fig 11b)**. The heatmap analyses show Vgat-MMG mice spent significantly more time (red, ~200 s) interacting with M1 than controls (Vgat-Piezo1 mice without m-Torquer; ~100 s), while spending less time investigating the empty chamber (~31 s) than control (~69 s), reflecting increased sociality by LH Vgat neuron stimulation **(Fig 11c, d)**. Then, the test of social novelty was performed by placing the second stranger mouse (M2) into another chamber, and the interaction of Vgat-MMG mice with M1 (a familiar mouse) and M2 (a novel mouse, social novelty) was measured **(Fig 11e)**. The heatmap results show Vgat-Piezo1 mice exhibited an increased preference for social novelty, spending significantly more time in the M2 chamber (red, ~199 s) than control mice without m-Torquer (~112 s) **(Fig 11f, g)**.

Using MMG capable of non-tethered animal behavioral testing in a large magnetic arena, social interactions of multiple individual animals could be simultaneously modulated within the same physical space [6,44]. Two Vgat-MMG mice were placed in the abovementioned three-chamber arena, and they were simultaneously stimulated and monitored for their social behaviors in response to a magnetic field for 3 min **(Fig 11h-k)**. Interestingly, in the sociality assay, the mice trajectory shows that both Vgat-MMG mice showed an increased preference for interaction with a novel mouse (M1) in a synchronized fashion, while controls (Vgat-Piezo1 without m-Torquers) exhibited more random activity without preference for a novel mouse **(Fig 11h, i)**. Similarly, in the social novelty test where another novel mouse (M2) was introduced to the chamber, both Vgat-MMG mice tended concurrently again to spend more time with M2 than M1, while controls showed no such social novelty preference **(Fig 11j, k)**. Altogether, these results clearly support that regulating LH$_A$-VTA inhibitory neurons promotes social interactions in mice.

5. **Remote cell-type specific ion channel gating in a pseudo-human brain**

The long-working distance of the MMG system could enable deep brain stimulation of larger animal models such as primates in a non-contact manner. However, experiments with live primates are currently not available due to the cost, regulation, and ethical issues. As a proof-of-concept study for reliable MMG application in larger animals, we created a large-scale brain phantom (size = 16 cm × 14.5 cm × 8.5 cm) mimicking the human brain using non-magnetic, light-impermeable resins. Two vacant wells were engrafted inside the 3D human-brain phantom for cell cultures . HEK293 cells were co-transfected with Cre and FLEX-Piezo1 and FACS enriched for robust Cre-dependent Piezo1 expression. The sorted cells were labeled with m-Torquer conjugated with anti-Myc antibody, embedded in the Matrigel matrix, which has similar mechanical properties as neural tissues, and cultured inside the brain phantom. After the phantom was placed inside the 60 cm MMG setup, we provided 1 hr MMG stimulation and analyzed the cells for $Ca^{2+}$ influx-dependent activities by measuring *c-fos* mRNA expression. The MMG stimulation elicited a 2.6-fold increase in *c-fos* levels compared to the control group without m-Torquer. In addition, the $Ca^{2+}$-dependent luciferase reporter assay resulted in a significantly higher (3.8-fold) expression of luciferase in Piezo1-expressing cells in response to MMG stimulation than the controls in the absence of m-Torquer . With the working distance of a sub-meter scale (up to 60 cm), which is significantly greater than that of MTG, our MMG system allows for wireless neural stimulation at clinically relevant tissue depth in any part of the brain, with potential for the study of larger animals such as primates and translational applications.

In summary, this example demonstrates that the m-Torquer conjugate-based drug of the present invention enables deep brain stimulation without implantation or tethering in freely moving mice. This is achieved through the targeted administration of the m-Torquer conjugate-based drug, which activates lateral hypothalamic (LH) neurons ectopically expressing the mechanosensitive transient receptor Piezo1. The Piezo1-expressing neurons in the LH of mice can be activated by a rotating circular magnet array (CMA) positioned over 70 cm above the animal's head, a distance greater than the bore size of standard human magnetic resonance imaging (MRI) devices, in conjunction with the m-Torquer conjugate-based drug, which functions as a nanoscale magnetic torque actuator within the magnetic field. Through this approach, we identified neural circuits in the LH that play a critical role in feeding-related behaviors. Specifically, the m-Torquer conjugate-based drug enables Piezo1-mediated stimulation of LH neurons, which in turn modulates feeding behavior. Vgat-positive inhibitory neurons in the LH promote feeding, whereas Vglut2-positive neurons induce feeding aversion. Furthermore, the m-Torquer conjugate-based drug demonstrates that the activation of its drug target proteins in the deep brain via magnetic stimulation can regulate other behaviors, such as social interaction. Additionally, in diet-induced obese (DIO) mice, we confirmed that Vglut2-specific magnetogenetic neuromodulation mediated by the m-Torquer conjugate-based drug suppresses food intake and alleviates obesity.

**Example 4: Regulation of Anion Transport Membrane Protein Gating and Inhibition of Neural Activity via Rotational Force of Magnetic Nanoparticles**

The present example describes a method for inducing anion influx into specific target cells using a magnetogenetic approach. The method involves the use of anion transport membrane proteins, magnetic nanoparticles (m-Torquer), a magnetic field generator, and a rotating magnetic field.

Following the methodology outlined in Nature Materials (2022) (Nat Mater. 2021 Jul;20(7):1029-1036), the following components were prepared (1) m-Torquer, (2) m-Torquer conjugated with fluorescent particles and Myc antibodies, (3) Magnetic simulation, and (4) CMA.

The specific methods of the present example are described in Examples 1 to 4 of Korean Patent Application No. 10-2022-0100260, filed on August 10, 2022, and in Korean Patent Application No. 10-2023-0107956, filed on August 17, 2023. The disclosures (including drawings) of Korean Patent Application No. 10-2022-0100260 and Korean Patent Application No. 10-2023-0107956 are incorporated herein by reference.

The m-Torquer magnetic nanoparticles used in the present example have a core-shell structure, with the shell containing octahedron-shaped unit magnetic nanoparticles that are randomly arranged via 1,2,3-Triazole bonds to the core (Fig. 4). SEM imaging confirmed that these nanoparticles have a diameter of approximately 300 to 700 nm. Compared to individual octahedral magnetic nanoparticles, the m-Torquer nanoparticles exhibit a magnetic moment that is over 470 times higher.

The present example provides a method for inducing anion influx into target cells using a magnetogenetic technique, which includes the following steps:

(1) Generation of an anion transport membrane protein capable of binding to m-Torquer magnetic nanoparticle.
(2) Production of an anion transport membrane protein whose gating can be regulated using the magnetogenetic technique.
(3) Expression of the anion transport membrane protein in target cells, followed by treatment with magnetic nanoparticles to enable binding.

(4) Application of a rotating magnetic field to the magnetic nanoparticles, delivering rotational force stimulation to the anion transport membrane protein and thereby activating it.

In Step (1), a nucleotide sequence encoding an antigenic motif (i.e., a polypeptide) was synthesized and introduced into the extracellular loop domain of the anion transport membrane protein to facilitate binding to the antibody moiety of the magnetic nanoparticles (i.e., polypeptide, the arch-shaped binding site of the ion channel as illustrated in FIG. 12 of Korean Patent Application No. 10-2023-0107956).

As a specific example, the present study utilized the mechanosensitive anion channel DmFLYC1, which mediates chloride ion transport (Figs. 9 and 10 of Korean Patent Application No. 10-2023-0107956).

The amino acid sequence of the recombinant anion transport membrane protein, expressed based on the corresponding genetic sequence, is designated as Sequence ID No. 1, whereas the amino acid sequence of the protein tag is designated as Sequence ID No. 2.

Accordingly, the anion transport membrane protein expressed in cells based on Sequence ID No. 1 contains an extracellular domain displaying a protein tag composed of the amino acid sequence denoted as Sequence ID No. 2.

[Table 3]

| Sequence ID No. | Name | Amino Acid Sequence |
|---|---|---|
| Sequence ID No. 1. | Amino Acid Sequence of DmFLYC1 Anion Transport Membrane | MGSYLHEPPGDEPSMRIEQPKTADRAPEQVAIH ICEPSKVVTESFPFSETAEPEAKSKNCPCPEIARI GPCPNKPPKIPINRGLSRISTNKSRPKSRFGEPS |

(continued)

| Sequence ID No. | Name | Amino Acid Sequence |
|---|---|---|
| | Protein | WPVESSLDLTSQSPVSPYREEAFSVENCGTAGS RRGSFARGTTSRAASSSRKDETKEGPDEKEVY QRVTAQLSARNQKRMTVKLMIELSVFLCLLGC LVCSLTVDGFKRYTVIGLDIWKWFLLLLVIFSG MLITHWIVHVAVFFVEWKFLMRKNVLYFTHG LKTSVEVFIWITVVLATWVMLIKPDVNQPHEQ KLISEEDLQTRKILEFVTWTIVTVLIGAFLWLVK TTLLKILASSFHLNRFFDRIQESVFHHSVLQTLA GRPVVELAQGISRTESQDGAGQVSFMEHTKTQ NKKVVDVGKLHQMKQEKVPAWTMQLLVDVV SNSGLSTMSGMLDEDMVEGGVELDDDEITNEE QAIATAVRIFDNIVQDKVDQSYIDRVDLHRFLI WEEVDHLFPLFEVNEKGQISLKAFAKWVVKVY NDQAALKHALNDNKTAVKQLNKLVTAILIVM MIVIWLIVTGIATTKLIVLLSSQLVVAAFIFGNT CKTIFEAIIFVFVMHPFDVGDRCVIDGNKMLVE EMNILTTVFLKWDKEKVYYPNSILCTKAIGNFF RSPDQGDVLEFSVDFTTPVLKIGDLKDRIKMYL EQNLNFWHPQHNMVVKEIENVNKIKMALFVN HTINFQDFAEKNRRSELVLELKKIFEELDIKYN LLPQEISIRNM |
| Sequence ID No. 2. | Myc Tag Sequence | EQKLISEEDL |

In Step (2), the responsiveness of the anion transport membrane protein to the rotational force of the magnetic nanoparticles was analyzed to assess its capability for functional modulation. If necessary, the nucleotide sequence was synthesized and modified to enhance its sensitivity to mechanical stimulation, ensuring adequate functional control (Fig. 15 of Korean Patent Application No. 10-2023-0107956).

In Step (3), the genetic information of the anion transport membrane protein was introduced in a form suitable for the target cells to facilitate its expression (Fig. 12 of Korean Patent Application No. 10-2023-0107956). This step includes the use of a viral vector as a method for gene delivery, wherein a viral vector carrying the gene encoding the anion transport membrane protein was constructed and subsequently introduced into the target cells or the tissue of an animal (Fig. 13 of Korean Patent Application No. 10-2023-0107956).

After gene delivery, a step was performed to induce expression of the anion transport membrane protein in the target cells. When using viral vectors for gene delivery, this process took approximately 2 to 3 weeks. After expression of the anion transport membrane protein in the target cells, a subsequent step was conducted to induce antigen-antibody binding between the anion transport membrane protein and the magnetic nanoparticles by introducing the magnetic nanoparticles into the environment where the target cells were present. The binding of the anion transport membrane protein to the magnetic nanoparticles took approximately 1 hour to 1 day.

In step (4), a rotating magnetic field generator was used to generate a rotating magnetic field in the environment containing the target cells. The rotating magnetic field was a uniform concentric magnetic field (25 mT to 100 mT) rotating in either a clockwise or counterclockwise direction. This rotating magnetic field induced the rotation of the magnetic nanoparticles within the magnetic field region, thereby generating a rotational force. This rotational force was transmitted to the anion transport membrane protein bound to the magnetic nanoparticles, ultimately facilitating the influx of anions into the target cells, thereby suppressing the activity of the target neurons (Fig. 13 of Korean Patent Application No. 10-2023-0107956).

[Discussion]

The left panel of Fig. 13 of Korean Patent Application No. 10-2023-0107956 illustrates a schematic diagram of the formation of the DmFLYC1-Myc ion channel according to Example 1. The ion channel of the present invention can be expressed in cells by introducing a genetically modified sequence, which may be delivered into the cell via an adeno-associated virus (AAV) or lentivirus. In this case, the genetically modified sequence may include a DmFLYC1-Myc expression cassette.

The right panel of Fig. 13 of Korean Patent Application No. 10-2023-0107956 presents an image confirming the formation of the FLYC ion channel and MYC protein tag according to Example 1. As shown in the right panel of Fig. 13, the protein tag in cells transfected with the modified gene sequence is expressed at the same location as the ion channel, verifying its co-localization and successful expression.

Fig. 14 of Korean Patent Application No. 10-2023-0107956 presents an image of cultured HEK293 cells expressing the anion transport membrane protein (DmFLYC1) with an added antigenic nucleotide sequence designed to facilitate binding to the antibody moiety of magnetic nanoparticles. The cells were treated with a fluorescent antigen to visualize antigen-antibody binding. In Fig. 14 of Korean Patent Application No. 10-2023-0107956, the FLYC ion channel (blue, corresponding to Fig. 13) is observed alongside the fluorescent protein RFP (red, Fig. 14 of Korean Patent Application No. 10-2023-0107956). When the MYC protein tag (green, Fig. 14 of Korean Patent Application No. 10-2023-0107956) was co-expressed with FLYC, MYC was detected at the same location as FLYC but showed no colocalization with intracellular RFP. This result confirms that the MYC tag is conjugated to the extracellular domain of FLYC, verifying its proper surface expression.

Fig. 15, which demonstrates the targeting of DmFLYC1 with magnetic nanoparticles (MNPs), shows that the MNPs (green, Fig. 15 of Korean Patent Application No. 10-2023-0107956) are bound to the extracellularly exposed domain of the ion channel (blue, Fig. 15 of Korean Patent Application No. 10-2023-0107956).

Fig. 12 illustrates the method for detecting Cl⁻ influx using the MQAE quenching technique. It demonstrates that when DmFLYC1 is activated via magnetogenetics (magnetogenetic activation), intracellular $[Cl^-]_i$ accumulates. The left panel of Fig. 16 depicts the MQAE quenching technique used to confirm Cl⁻ influx. The top portion of the left panel represents the reaction mechanism of MQAE binding to Cl⁻, while the bottom portion conceptualizes the fluorescence quenching of MQAE when Cl⁻ ions enter HEK293 cells expressing FLYC1 ion channels due to the rotation of magnetic nanoparticles. The right panel of Fig. 16 compares fluorescence intensity between HEK293 cells injected with MQAE, containing (1) ion channels without a protein tag (left graph) and (2) ion channels with a protein tag (right graph). The left graph indicates that in the absence of a protein tag, fluorescence intensity remains unchanged regardless of the presence of magnetic nanoparticles (MNPs) or rotating magnetic fields (RMFs). However, in the right graph, when MNPs (m-Torquer) and RMFs are present, the Fo/F ratio increases, confirming fluorescence quenching. This result indicates that the ion channel is opened, allowing Cl⁻ influx into the cell.

Fig. 13 presents the results of membrane potential measurements in cells expressing DmFLYC1 ion channels after treatment with magnetic nanoparticles (m-Torquer) and exposure to a magnetic field over a specific period. Referring to the upper graph in Fig. 13, a decrease in membrane potential is observed upon the application of a magnetic field (indicated by the red arrow), suggesting that Cl⁻ ions entered the cell upon ion channel opening. This result implies that the opening of the ion channel was induced by the rotation of the magnetic nanoparticles (m-Torquer) in response to the applied magnetic field. Conversely, in the lower graph of Fig. 13, where no magnetic field was applied, the periodic reduction in membrane potential is absent. This confirms that the decrease in membrane potential observed in the upper graph was specifically due to the magnetic field application.

In summary, when the ion channel is DmFLYC1 and the antigenic moiety facilitating binding to the antibody moiety of the magnetic nanoparticles is MYC-tag, the antibody moiety of the magnetic nanoparticle binds to the MYC-tag. Upon the application of a rotating magnetic field, the injected magnetic nanoparticles (m-Torquer) rotate, resulting in the opening of the DmFLYC1 ion channel. This channel opening allows Cl⁻ ions to flow into the cell, leading to membrane hyperpolarization, which is consistent with the results presented in Fig. 17. Thus, this study demonstrates that cell activity can be modulated using the rotational force of m-Torquer conjugates.

**EP 4 588 510 A1**

**Claims**

1. A brain function control device for non-invasively activating or inhibiting a target neural circuit or neural network, wherein the device is configured to generate an acoustic wave pattern that utilizes the resonance frequency(ies) of a target mechano-sensitive ion channel within a specific neuron in a target brain region to modulate the opening and closing of the target mechano-sensitive ion channel.

2. The brain function control device of Claim 1, wherein the device utilizes the interference effect of ultrasound while inducing resonance phenomenon of the target mechano-sensitive ion channel, and generates a spatiotemporally controlled sequence of weak-intensity one-dimensional, two-dimensional, or three-dimensional ultrasound patterns such that the activation of non-target cells remains unaffected..

3. The brain function control device of Claim 1, wherein the activation or inhibition of the target neural circuit or neural network occurs through alteration of neuronal connectivity length, addition or removal of connections, and/or formation of new neurons.

4. The brain function control device of Claim 1, wherein the acoustic wave pattern that controls the opening and closing of the target mechano-sensitive ion channel within the specific neuron in the target brain region is configured to spatiotemporally regulate dopamine secretion in the specific neuron.

5. The brain function control device of Claim 1, wherein the device is mountable on the head of a freely moving animal, and is remotely controllable to generate an acoustic wave pattern that modulates the opening and closing of the target mechano-sensitive ion channel within the specific neuron in the target brain region.

6. The brain function control device of Claim 1, wherein the acoustic wave pattern that spatiotemporally modulates the opening and closing of the target mechano-sensitive ion channel within the specific neuron in the target brain region is configured to induce neuroplasticity and/or synaptic plasticity.

7. The brain function control device of Claim 1, wherein the acoustic wave pattern that spatiotemporally modulates the opening and closing of the target mechano-sensitive ion channel within the specific neuron in the target brain region is configured to enhance or suppress neural circuit adaptation in response to external stimuli, internal physiological signals, or administered drugs.

8. The brain function control device of Claim 1, wherein the acoustic wave pattern that spatiotemporally modulates the opening and closing of the target mechano-sensitive ion channel within the specific neuron in the target brain region is configured to construct, strengthen, expand, or weaken neural circuits or their interconnections.

9. The brain function control device of Claim 1, wherein the device does not utilize high-intensity focused ultrasound (FUS) capable of causing thermal side effects.

10. The brain function control device of Claim 1, wherein a weak-intensity acoustic wave pattern is configured to be generated with high spatiotemporal resolution to induce the resonance phenomenon of the target mechano-sensitive ion channel within the specific neuron in the target brain region, without altering the activity of non-target cells, thereby preventing or mitigating unintended off-target effects, thermal side effects, auditory disturbances, or pain-related side effects.

11. The brain function control device of any one of Claims 1 to 10, wherein the target mechano-sensitive ion channel within the specific neuron in the target brain region to be modulated is either naturally expressed in a specific cell within the target body region, or is artificially expressed and/or genetically engineered using sonogenetic technology to possess a predetermined resonance frequency, in a specific cell within the target body region.

12. The brain function control device of any one of Claims 1 to 10, wherein the target mechano-sensitive ion channel within the specific neuron in the target brain region to be modulated is a cation channel or an anion channel present in a biological lipid membrane.

13. The brain function control device of any one of Claims 1 to 10, wherein the acoustic wave pattern is divided into an operational period and a resting period, each ranging from 10 milliseconds to 10 seconds, to minimize heat generation in biological target.

14. The brain function control device of any one of Claims 1 to 10, wherein the acoustic wave pattern applied to the specific neuron in the target brain region is configured to (i) induce neuronal polarization, or (ii) regulate protein expression levels via ion concentration modulation.

15. The brain function control device of any one of Claims 1 to 10, wherein modulation of the neural circuit by the acoustic wave pattern occurs selectively in the specific neuron within the target brain region, and wherein real-time activation or inhibition of the target neural circuit or neural network is synchronized with the generation of the acoustic wave pattern.

16. A mechano-sensitive ion channel gating control device for a target mechano-sensitive ion channel in a biological lipid membrane, wherein the device is configured to generate an acoustic wave pattern utilizing the resonance frequency(ies) of a specific mechano-sensitive ion channel within a specific cell in a target body region to modulate the opening and closing of the target mechano-sensitive ion channel.

17. The mechano-sensitive ion channel gating control device of Claim 16, wherein the acoustic wave pattern is configured to match and induce the resonance frequency(ies) of the target mechano-sensitive ion channel, the resonance frequency(ies) being in the range of 0.5 MHz to 100 MHz, and preferably in the range of 0.5 MHz to 10 MHz.

18. The mechano-sensitive ion channel gating control device of Claim 16, wherein the target mechano-sensitive ion channel to be modulated is either naturally expressed in the specific cell within the target body region, or is artificially expressed and/or genetically engineered using sonogenetic technology to possess a predetermined resonance frequency, in a specific cell within the target body region.

19. The mechano-sensitive ion channel gating control device of Claim 16, wherein the specific cell is selected from the group consisting of cancer cells, immune cells, neurons, endocrine cells, epithelial cells, epidermal cells, and muscle cells.

20. The mechano-sensitive ion channel gating control device of Claim 16, wherein the device utilizes the interference effect of ultrasound while inducing resonance phenomenon of the target mechano-sensitive ion channel, and generates a spatiotemporally controlled sequence of weak-intensity one-dimensional, two-dimensional, or three-dimensional ultrasound patterns such that the activation of non-target cells remains unaffected.

21. The mechano-sensitive ion channel gating control device of Claim 16, wherein the device is mountable on a freely moving animal's target body region and is remotely controllable to generate an acoustic wave pattern that utilizes the resonance frequency(ies) of the specific mechano-sensitive ion channel within the specific cell in the target body region to modulate the opening and closing of the ion channel.

22. The mechano-sensitive ion channel gating control device of Claim 16, wherein a weak-intensity acoustic wave pattern is configured to be generated with high spatiotemporal resolution to induce the resonance phenomenon of the target mechano-sensitive ion channel within the specific cell in the target body region, without altering the activity of non-target cells, thereby preventing or mitigating unintended off-target effects, thermal side effects, auditory disturbances, or pain-related side effects.

23. A computer-readable recording medium or a medium for transmitting a program to a computer for use in a brain function control device of any one of Claims 1 to 10 or the mechano-sensitive ion channel gating control device of any one of Claims 16 to 22, wherein the medium stores or transmits a program executable by a processor to generate an acoustic wave pattern optimized for the resonance frequency(ies) of a target mechano-sensitive ion channel in order to activate or reversibly inhibit the target mechano-sensitive ion channel within a specific body region.

24. An acoustic wave oscillator, operably controlled by the program of Claim 23, in the brain function control device of any one of Claims 1 to 10 or the mechano-sensitive ion channel gating control device of any one of Claims 16 to 22, wherein the oscillator is configured to generate an acoustic wave pattern optimized for the resonance frequency(ies) of a target mechano-sensitive ion channel to activate or reversibly inhibit the target mechano-sensitive ion channel within a specific body region.

25. The acoustic wave oscillator of Claim 24, wherein the program of Claim 23 is configured to optimize the frequency and intensity of acoustic waves for targeted therapy and to modulate acoustic wave patterns utilizing interference effects, thereby activating or reversibly inhibiting the target mechano-sensitive ion channel expressed in a biological target.

[Fig. 1]

[Fig. 2]

**a** Behavior of PDMS microparticles in US pattern

US-patterner

before    after

US
10 sec

4 mm

2 mm

high
(US)
low

**b** Behavior of PDMS particles in US pattern

US-patterner

before    after

US
10 sec

1 mm  2 mm

2 mm

high
(US)
low

**c** target ultrasound pattern

US simulation algorithm

US-patterner design

3D modeling and printing

US-patterner

EP 4 588 510 A1

[Fig. 3]

[Fig. 4]

Push

10 ms

Acoustic radiation force

Neuromodulation

Mechano-Sonogenetics

1. Ultrasound

2. Mechanical Force

3. Calcium entry  4. Amplification

ER

1. Ultrasound

3-1. Activation of mechanosensitive channels

Voltage-gated $Ca^{2+}$ and $Na^+$ channels

T-type $Ca^{2+}$ channel

TRPM4

Membrane

$Na^+$

4. Signal amplification

$Ca^{2+}$

$Ca^{2+}$   $Na^+$

2. Mechanical deformation of actins

$Ca^{2+}$

5. Bursting action potentials

3-2. Endoplasmic reticulum

[Fig. 5]

Neuromodulation methods

magnetic

electrical

optinal

acoustic

chemical

Neuronal activity

Yang et al., Adv Mater, 2021

## Optogenetics

ChR2

ChR2 viral vector

Packaging cell line

ChR2 virus

Deisseroth, Nat. Neuro., 2015

Induction of neural activity

Inhibition of neural activity

## Magnetogenetics

CMV    Myc897-Piezo1

Ad-Myc+Piezo1

Myc tag

m-Torquer

Piezo1

Myc897-Piezo1 expression

$F_z$

Gene delivery    Nucleus

Ca2+

Lee et al, Nat. Materials., 2021

Induction of neural activity

Inhibition of neural activity

**1. Cre-Loxp System for cell-specific magnetic control**

Viral injection

Cre+ Cre- Cre+ Cre- Cre- Cre+

lox2722 lox2722

EF1a eYFP ChR2

Reversible Cre-mediated recombination

loxP loxP

EF1a ChR2 eYFP

Permanent Cre-mediated lox site excision

loxP lox2722

EF1a ChR2 eYFP

Transgenic parvalbumin::Cre

eYFP

Parvalbumin

Overlay

30μm

Fenno, *Annu. Rev. Neurosci.* (2011)

**2. Long-term , Deep brain neuromodulation**

Piezo1  m-Torquer

**LH: lateral hypothalamus**
Important for diverse behaviors including motivation, rewards...

LH

**3. Magnetic control of feeding in freely moving mice**

LH Vglut2 neurons

Ca²⁺  m-Torquer
Myc
Piezo1
Ca²⁺

↓ Food consumption
↓ Weight changes

LH Vgat neurons

Ca²⁺  m-Torquer
Myc
Piezo1
Ca²⁺

↑ Food consumption
↑ Weight changes

food  S N

MG stimulation

LH Vglut2 (Glutamate) neurons
⚡ *activate*
VTA GABA neurons ↑
VTA Dopamine neurons ↓
Feeding ↓

LH Vgat (GABA) neurons
⊥ *inhibit*
VTA GABA neurons ↓
VTA Dopamine neurons ↑
Feeding ↑

[Fig. 6]

EP 4 588 510 A1

[Fig. 7]

a. Cre-mouse production

Tissue-specific Cre    LoxP(Floxed)Piezo1

Cre-LoxP activated Piezo1

Male 14 Males    Male 13 Males

300bp
Vglut2-cre    Vglut2-cre

VGAT-Cre    VGLUT2-Cre

b. Cell-specific MG (Piezo1, m-Torquer)

Specifficity

Mouse brain

Hypothalamus
target
regions

Piezo1

VGLUT2-excitatory neurons

Piezo1

VGAT-inhibitory neurons

Force (1-10 pN)

Mechano-
gating

c. in vivo feeding control

-Short-term
-Long-term

Pre-manetostimulation

Magnetostimulation

Post-magnetostimulation

d. in vivo social behavior control

f    Piezo1+
m-Torquer−
MF+

Piezo1+
m-Torquer+
MF+

M2    M1    M2    M1

6 cm    short ▬▬▬▬ long
Time spent

Ad-Flex-Piezo1    m-Torquer

3 weeks

Lateral
Hypothalamus

Lateral
Hypothalamus

Vglut2
or Vgat

Vglut2
or Vgat

Feeding    Social    Large animal

[Fig. 8]

[Fig. 9]

[Fig. 10]

[Fig. 11]

EP 4 588 510 A1

[Fig. 12]

[Fig. 13]

67

[Fig. 14]

[Fig. 15]

**INTERNATIONAL SEARCH REPORT**

| International application No. |
| --- |
| **PCT/KR2023/014074** |

## A. CLASSIFICATION OF SUBJECT MATTER

**A61N 7/00**(2006.01)i

According to International Patent Classification (IPC) or to both national classification and IPC

## B. FIELDS SEARCHED

Minimum documentation searched (classification system followed by classification symbols)

A61N 7/00(2006.01); A61N 1/00(2006.01); C12M 1/42(2006.01); C12N 13/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 힘-감응 이온채널(force-sensitive ion channel), 공명주파수(resonance frequency), 음파(sound wave), 뇌(brain), 뉴런(neuron), 치료(treatment)

## C. DOCUMENTS CONSIDERED TO BE RELEVANT

| Category* | Citation of document, with indication, where appropriate, of the relevant passages | Relevant to claim No. |
| --- | --- | --- |
| A | AZADEH, Seyedeh Sara et al. Ultrasound and sonogenetics: a new perspective for controlling cells with sound. Iranian Journal of Pharmaceutical Research. 01 January 2021, vol. 20, no. 3, pp. 151-160.<br>See abstract; page 158; and figure 1. | 1-25 |
| A | US 8340773 B2 (TOWE, Bruce et al.) 25 December 2012 (2012-12-25)<br>See columns 2 and 6; and figures 6A-6B. | 1-25 |
| A | YOO, Sangjin et al. Focused ultrasound excites cortical neurons via mechanosensitive calcium accumulation and ion channel amplification. Nature Communications. 25 January 2022 (online publication date), vol. 13, article no. 493, pp. 1-13.<br>See entire document. | 1-25 |
| A | AZANZA, Maria J. et al. Frequency resonance effect of neurons under low-frequency weak magnetic field. Journal of Magnetism and Magnetic Materials. 30 November 2006 (online publication date), vol. 310, pp. 2865-2867.<br>See entire document. | 1-25 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **11 January 2024** | **11 January 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/014074** |

### C.    DOCUMENTS CONSIDERED TO BE RELEVANT

| Category* | Citation of document, with indication, where appropriate, of the relevant passages | Relevant to claim No. |
| --- | --- | --- |
| A | KR 10-2022-0117853 A (INDUSTRY-ACADEMIC COOPERATION FOUNDATION, YONSEI UNIVERSITY et al.) 24 August 2022 (2022-08-24)<br>See entire document. | 1-25 |

Form PCT/ISA/210 (second sheet) (July 2022)

## INTERNATIONAL SEARCH REPORT

| International application No. |
|---|
| **PCT/KR2023/014074** |

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

     a.  ☑ forming part of the international application as filed.

     b.  ☐ furnished subsequent to the international filing date for the purposes of international search (Rule 13*ter*.1(a)),

         ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

International application No.

**PCT/KR2023/014074**

| Patent document cited in search report | | | Publication date (day/month/year) | Patent family member(s) | | | Publication date (day/month/year) |
|---|---|---|---|---|---|---|---|
| US | 8340773 | B2 | 25 December 2012 | US | 10016612 | B2 | 10 July 2018 |
| | | | | US | 2006-0167500 | A1 | 27 July 2006 |
| | | | | US | 2010-0179628 | A1 | 15 July 2010 |
| | | | | US | 2011-0077722 | A1 | 31 March 2011 |
| | | | | US | 2012-0041499 | A1 | 16 February 2012 |
| | | | | US | 2013-0096656 | A1 | 18 April 2013 |
| | | | | US | 2014-0316499 | A1 | 23 October 2014 |
| | | | | US | 2016-0001086 | A1 | 07 January 2016 |
| | | | | US | 2017-0120060 | A1 | 04 May 2017 |
| | | | | US | 7702395 | B2 | 20 April 2010 |
| | | | | US | 8369956 | B2 | 05 February 2013 |
| | | | | US | 8626303 | B2 | 07 January 2014 |
| | | | | US | 8774928 | B2 | 08 July 2014 |
| | | | | US | 9457196 | B2 | 04 October 2016 |
| | | | | US | 9555258 | B2 | 31 January 2017 |
| | | | | WO | 2004-016315 | A1 | 26 February 2004 |
| KR | 10-2022-0117853 | A | 24 August 2022 | KR | 10-2022-0117855 | A | 24 August 2022 |
| | | | | KR | 10-2022-0117856 | A | 24 August 2022 |
| | | | | US | 2022-0257963 | A1 | 18 August 2022 |
| | | | | US | 2022-0257964 | A1 | 18 August 2022 |
| | | | | US | 2022-0259583 | A1 | 18 August 2022 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2023012209 W
- KR 1020220100260

- KR 1020230107956

**Non-patent literature cited in the description**

- Sec. Inflammation. *Front. Immunol*, 26 January 2022, vol. 13, 2022
- *Nature Materials*, 2022

- *Nat Mater.*, July 2021, vol. 20 (7), 1029-1036